(19) Europäisches Patentamt
European Patent Office
Office européen des brevets

(11) **EP 3 133 087 A1**

(12) **DEMANDE DE BREVET EUROPEEN**

(43) Date de publication:
22.02.2017 Bulletin 2017/08

(51) Int Cl.:
***C07K 16/28*** (2006.01)

(21) Numéro de dépôt: **16184011.1**

(22) Date de dépôt: **24.01.2005**

(84) Etats contractants désignés:
**AT BE BG CH CY CZ DE DK EE ES FI FR GB GR HU IE IS IT LI LT LU MC NL PL PT RO SE SI SK TR**

(30) Priorité: **23.01.2004 FR 0400675**

(62) Numéro(s) de document de la (des) demande(s) initiale(s) en application de l'article 76 CBE:
**10183899.3 / 2 365 002**
**05717480.7 / 1 709 082**

(71) Demandeurs:
• **bioMérieux**
**69280 Marcy l'Etoile (FR)**
• **Institut National de la Santé et de la Recherche Médicale (INSERM)**
**75013 Paris (FR)**

(72) Inventeurs:
• **MARCHE, Patrice**
**38240 Meylan (FR)**
• **ROLLAND, Alexandre**
**38360 Sassenage (FR)**
• **JOUVIN-MARCHE, Evelyne**
**38240 Meylan (FR)**
• **PERRON, Hervé**
**69290 Saint Genis Les Ollieres (FR)**

(74) Mandataire: **Agasse, Stéphane et al**
**Cabinet GERMAIN & MAUREAU**
**B.P. 6153**
**69466 Lyon Cedex 06 (FR)**

Remarques:
Cette demande a été déposée le 12.8.2016 comme demande divisionnaire de la demande mentionnée sous le code INID 62.

(54) **DOSAGE DE CYTOKINES ASSOCIÉES À LA MSRV/HERV-W**

(57) Composition comprenant au moins un anticorps choisi dans le groupe (i) des anticorps anti-Env-SU MSRV/HERV-W ou dans le groupe (ii) des anticorps anti-TLR4 capables de se lier spécifiquement à la fraction soluble de la protéine Env de MSRV/HERV-W ou au récepteur TLR4 de la fraction soluble de la protéine Env de MSRV/HERV-W, et un véhicule pharmaceutiquement acceptable.

EP 3 133 087 A1

**Description**

Depuis plusieurs années, de nombreuses études ont mis en évidence l'expression importante de divers rétrovirus, notamment endogènes (HERVs), dans des pathologies telles que le diabète [1], la sclérose en plaques (SEP) [2] et la schizophrénie (SCZ) [3]. Les HERVs possèdent des homologies avec les rétrovirus animaux connus et proviennent probablement de leur intégration au sein de la lignée germinale humaine. Les séquences de ces HERVs dans le génome humain sont en général incomplètes même si des séquences provirales entières ont déjà été identifiées.

Des particules rétrovirales dans des cultures de cellules leptomeningées de patients atteints de SEP ont déjà été isolées [4]. L'étude de ces particules a montré qu'elles possédaient des séquences génétiques homologues à l'ADN humain définissant une nouvelle famille de rétrovirus endogènes (HERV-W) [2, 5, 6]. La présence de MSRV dans le sérum et/ou le liquide céphalo-rachidien (LCR) de patients a maintenant été confirmée par différentes équipes [7-9] et une corrélation entre la charge virale et l'évolution de la maladie a été mise en évidence [10]. Il a ensuite été mis en évidence que MSRV et sa protéine d'enveloppe possédaient des propriétés pro-inflammatoires médiées par les lymphocytes T, de type superantigène (SAg) [11]. Un modèle animal (souris SCID humanisées) a été mis en place confirmant *in vivo* le potentiel immuno-pathogénique de telles particules et notamment leur capacité à induire la sécrétion de cytokines pro-inflammatoires, médiée par les lymphoctes T [12].

Dans la suite de la description les virus de la famille MSRV/HERV-W seront indifféremment dénommés MSRV ou MSRV/HERV-W.

D'autres pathologies présentent, comme la SEP, un profil d'activation du système immunitaire caractérisé par la présence de grandes quantités d'IL-6. Parmi elles, la schizophrénie (SCZ), - maladie neuropsychiatrique liée à des facteurs génétiques et environnementaux -, présente suivant les cas des quantités sérique d'IL-6 largement supérieures à la normale [13]. Par ailleurs, des séquences rétrovirales similaires à celles de MSRV ont été identifiées chez des patients SCZ [3]. Et, plus récemment, il a été démontré que le LCR de patients SCZ nouvellement diagnostiqués présentent des séquences rétrovirales de la famille MSRV/HERV-W associées à des particules circulantes [14].

Une telle expression est compatible avec un rôle pour MSRV/HERV-W dans différentes pathologies neurologiques par l'intermédiaire des effets pro-inflammatoires de sa protéine d'enveloppe et de la voie d'activation engagée. Cet élément rétroviral (lui-même sous dépendance de co-facteurs d'activation) et ses effets associés sont tout à fait pertinents dans le cas de maladie démyélinisantes inflammatoires [15]. Dans de le cas de la schizophrénie, une telle inflammation révélée au niveau systémique par la surexpression d'IL-6, est aussi pertinente, localement au niveau de la substance grise du cerveau, au regard des effets neurotoxiques et excitotoxiques connus de l'inflammation médiée par les microgliocytes/macrophages du cerveau [16-30].

L'expression différentielle de séquences ARN MSRV/HERV-W a aussi été rapportée dans le tissus corticofrontal de patients schizophrènes, et non chez des témoins incluant notamment des psychoses maniaco-dépressives (bioplar disorders) [31]. En outre, le reflet systémique de ce différentiel rétroviral « MSRV/HERV-W » a été mis en évidence dans le sang de jumeaux homozygotes discordants pour la pathologie schizophrénique, corroborant ainsi l'existence d'une réplique « systémique » pouvant jouer notamment un rôle dans l'hyperexpression d'IL-6 circulante précédemment rapportée [3].

Les effets de la protéine d'enveloppe de MSRV/HERV-W chez des patients schizophrènes ont leur place dans la cascade pathogénique de la schizophrénie, au niveau du rôle de facteurs inflammatoires spécifiques dans la genèse de signaux neurotoxiques et/ou excitotoxiques corticaux ou sub-corticaux.

Il existe à ce jour des publications concordantes de différentes équipes indépendantes qui montrent une association des éléments de la famille MSRV/HERV-W avec des pathologies telles que la SEP et la SCZ, mais d'autres maladies pourraient également s'avérer concernées.

Les présents inventeurs ont maintenant montré de manière inattendue que la protéine Env de MSRV/HERV-W a une autre activité pro-inflammatoire, indépendante de celle médiée par les lymphocytes T, cette nouvelle activité pro-inflammatoire passant par des cellules différentes des cellules T et par un récepteur différent du récepteur des cellules T (TCR) et résultant dans l'activation d'une voie pro-inflammatoire différente de celle qui résulte de l'activation du TCR par un superantigène. Cette nouvelle activité pro-inflammatoire est donc différente de l'activation pro-inflammatoire causée par une fonction superantigénique qui par définition passe par une liaison au TCR des lymphocytes T. Les inventeurs ont trouvé que c'est précisément le domaine de la fraction soluble (Env-SU) de la protéine d'enveloppe de MSRV/HERV-W qui est responsable de ces nouveaux effets pro-inflammatoires médiés par des cellules présentatrices d'antigènes (macrophages, monocytes, cellules dendritiques et microgliocites) et un récepteur non identifié, jusqu'à ce jour, pour son rôle dans le déclenchement des effets pro-inflammatoires médiés par Env-SU MSRV/HERV-W. Ainsi, Env-SU, naturellement présente à la surface des particules rétrovirales, cible les cellules présentatrices d'antigènes (APC), les active et induit la sécrétion de grandes quantités de TNF-$\alpha$, d'IL-1$\beta$ et d'IL-6. Ces effets pro-inflammatoires ont été étudiés chez des patients atteints de SEP puis comparés à ceux obtenus chez des donneurs. Les inventeurs ont ainsi montré que la production d'IL-6 induite par Env-SU était augmentée chez les patients SEP et était corrélée avec leur score clinique (EDSS). La présence élevée d'IL-6 dans le sérum, le LCR et les lésions de patients SEP [32-37] est présumée jouer un rôle important dans le développement et la persistance des lésions observées au niveau du système nerveux central des patients SEP.

Les présents inventeurs ont donc trouvé, de manière surprenante, que le récepteur de Env-SU impliqué dans ces nouveaux effets pro-inflammatoires est la protéine TLR4 humaine (Toll-Like Receptor 4). Le gène codant pour TLR4 est localisé sur le chromosome 9 (9q32-q33). La protéine est constituée de 839 acides aminés et présente un poids moléculaire de 95679 Da. Il était connu que TLR4 coopérait avec une autre molécule appelée MD-2 et, qu'ensemble avec CD14, ce complexe est impliqué dans la reconnaissance des lipopolysaccharides (LPS) bactériens conduisant à l'activation du facteur NF-kappa-B, à la sécrétion de cytokines et à la réponse inflammatoire, mais son rôle de récepteur de la fraction soluble de la protéine d'enveloppe (Env-SU) de MSRV/HERV-W n'était pas connu avant la présente invention. La protéine TLR4 n'étant pas exprimée sur les lymphocytes T, ces derniers ne sont pas les cibles primaires des effets mis en évidence ici avec le récepteur TLR4. Les inventeurs ont également montré que les particules rétrovirales MSRV associées à l'ARN circulant détecté dans les fluides biologiques de patients sont, en dehors de toute réplication rétrovirale, inductrices de cette nouvelle voie d'activation pro-inflammatoire précoce passant par le récepteur TLR4 présent sur des cellules présentatrices d'antigènes, telles que macrophages, monocytes, cellules dendritiques et microgliocytes. Ils ont pour cela inactivés les virions MSRV purifiés à partir de surnageants de culture productrices [4] et testé leur acivité liée à la présence de la protéine Env de MSRV/HERV-W. Les résultats présentés dans la partie expérimentale confirment que la voie précoce d'activation de l'immunité innée par le récepteur TLR4 est ciblée par la protéine d'enveloppe sous la forme soluble Env-SU et sous la forme membranaire à la surface des virions MSRV.

Les résultats obtenus dans le cadre de la présente invention permettent donc d'établir des stratégies d'immunothérapie dans des pathologies, en particulier neurologiques, telles que la SEP et la SCZ et ont notamment permis d'identifier les vecteurs capables de véhiculer un ou des agents thérapeutiques à travers la barrière hémato-encéphalique. L'un des aspects primordiaux des résultats de l'invention, dans le cadre de la thérapie, est qu'ils ont permis de cibler une composante inflammtoire liée à l'activation des microgliocytes/macrophages cérébraux, avec une spécificité unique dans ce domaine grâce à l'identification du système ligand/récepteur « Env-SU MSRV/HERV-W et TLR4 » impliqué dans la génèse des signaux inflammatoires précoces qui, par exemple, initient une cascade démyélinisante quand ils proviennent des microgliocytes/macrophages localisés dans la substance blanche (SEP) ou une cascade excitotoxique/neurotoxique quand ils sont produits par ces mêmes cellules dans la substance grise (SCZ).

Ainsi, la présente invention a pour objet une méthode pour traiter un individu présentant une pathologie associée à la présence de MSRV/HERV-W comprenant l'administration à l'individu d'une composition thérapeutique ou médicament comprenant au moins un anticorps choisi dans le groupe (i) des anticorps anti-Env-SU MSRV/HERV-W capables de se lier spécifiquement (se liant spécifiquement) à la fraction soluble de la protéine Env de MSRV/HERV-W ou dans le groupe (ii) des anticorps capables de se lier spécifiquement (se liant spécifiquement) au récepteur TLR4 de la fraction soluble de la protéine Env-SU MSRV/HERV-W pour inhiber la cascade pro-inflammatoire induite par l'activation de MSRV/HERV-W, et un véhicule plus, si nécessaire un vecteur pharmaceutiquement acceptable. Lesdits anticorps inhibent la cascade pro-inflammatoire induite par l'activation de Env-SU MSRV/HERV-W. Ladite méthode est en particulier utilisée pour le traitement de la SEP et la SCZ, mais peut être appliquée au traitement d'autres maladies si elles sont associées à une expression de la protéine pro-inflammatoire de MSRV/HERV-W dans un contexte où cette dernière initie une cascade pathologique.

Lesdits anticorps anti-Env-SU sont en particulier capables de se lier à une région qui correspond aux acides aminés 122-131 (inclus) et/ou à une région qui correspond aux acides aminés 312-316 (inclus) et/ou à une région qui correspond aux acides aminés 181-186 (inclus) de la séquence identifiée en SEQ ID NO : 1.

Selon la méthode de l'invention, on peut administrer au patient une composition ou médicament comprenant au moins un anticorps anti-Env-SU MSRV/HERV-W ou au moins un anticorps anti-TLR4. Dans un mode de réalisation de la méthode de l'invention on administre au patient une composition ou un médicament comprenant au moins un anticorps anti-Env-SU MSRV/HERV-W et au moins un anticorps anti-TLR4.

De préférence, dans la méthode de l'invention, l'anticorps anti-Env-SU est choisi parmi les anticorps suivants : anticorps monoclonaux anti-Env-SU MSRV/HERV-W (anticorps 3B2H4, 13H5A5 et 3H10F10 (bioMérieux)) et l'anticorps anti-TLR4 est l'anticorps anti-TLR4 humaine HTA125 (commercialisé par la société eBioscience). Le mode d'obtention des anticorps monoclonaux bioMérieux est décrit dans la description qui suit. Les anticorps précités ont pour caractéristique originale, et jusqu'alors inconnue, d'être neutralisants vis à vis de l'activité pro-inflammatoire nouvellement mise en évidence sur les cellules présentatrices d'antigènes via le récepteur TLR4.

Les anticorps anti-TLR4 ou anti-Env-SU sont administrés à l'individu à l'aide d'un véhicule pharmaceutiquement acceptable, associé si nécessaire à un vecteur pharmaceutiquement acceptable pour les véhiculer à travers la barrière hémato-encéphalique (BHE). Si, comme c'est le cas pour la SEP, à un certain stade de l'évolution de la pathologie il y a ouverture de la barrière hémato-encéphalique il n'est pas nécessaire d'utiliser de tels vecteurs, mais quand il n'y a pas ouverture de la barrière hémato-encéphalique, ce qui est également le cas pour SCZ, de tels vecteurs sont nécessaires. Ces vecteurs sont bien connus [38-45]. La démarche thérapeutique cible une composante inflammatoire liée à l'activation des microgliocytes/macrophages cérébraux, avec une spécificité unique dans ce domaine. Cette spécificité est liée à l'identification du système ligand-récepteur « Env-SU MSRV et TLR4 » impliqué dans la genèse des signaux inflammatoires précoces qui, par exemple, initient une cascade démyélinisante quand ils proviennent des microgliocytes/macrophages localisés dans la substance blanche (SEP), ou une cascade excitotoxique/neurotoxique quand ils sont produits par ces mêmes cellules dans la substance grise (SCZ).

L'utilité des anticorps anti-Env-SU MSRV ou anti-TLR4 est de bloquer « à la source » la cascade pro-inflammatoire induite par l'expression de MSRV/HERV-W (elle même, induite par des co-facteurs infectieux de type herpesvirus, par des signaux hormonaux ou par des cytokines particulières, variables selon les pathologies) dans les différentes maladies associées à une expression pathologique de MSRV/HERV-W.

Ainsi, la présente invention a pour objet une composition, étant entendu que cette composition est à visée thérapeutique, qui comprend au moins un anticorps choisi dans le groupe (i) des anticorps anti-Env-SU MSRV/HERV-W ou dans le groupe (ii) des anticorps anti-TLR4, capables de se lier spécifiquement (qui se lient spécifiquement) à la fraction soluble de la protéine Env de MSRV/HERV-W ou au récepteur TLR4 de la fraction soluble de la protéine Env de MSRV/HERV-W, et un véhicule pharmaceutiquement acceptable. Si nécessaire, ladite composition comprend également un vecteur pharmaceutiquement acceptable ; lesdits anticorps inhibant la cascade pro-inflammatoire induite par l'activation de Env-SU MSRV/HERV-W. De préférence, la composition comprend au moins un anticorps anti-Env-SU MSRV/HERV-W et au moins un anticorps anti-TLR4. Les anticorps préférés dans cette composition sont les anticorps anti-Env-SU MSRV/HERV-W (3B2H4, 13H5A5 et 3H10F10) et l'anticorps anti-TLR4 HTA125. Les anticorps précités sont des anticorps monoclonaux « neutralisants » vis à vis de l'activité pro-inflammatoire nouvellement mise en évidence sur les cellules présentatrices d'antigènes via le récepteur TLR4.

Lesdits anticorps anti-Env-SU sont en particulier capables de se lier à une région qui correspond aux acides aminés 122-131 (inclus) et/ou à une région qui correspond aux acides aminés 312-316 (inclus) et/ou à une région qui correspond aux acides aminés 181-186 (inclus) de la séquence identifiée en SEQ ID NO : 1.

L'invention a aussi pour objet l'utilisation d'au moins un anticorps choisi dans le groupe (i) des anticorps anti-Env-SU MSRV/HERV-W ou dans le groupe (ii) des anticorps anti-TLR4 capables de se lier spécifiquement à la fraction soluble de la protéine Env de MSRV/HERV-W ou au récepteur TLR4 de la fraction soluble de la protéine Env de MSRV/HERV-W pour la préparation d'un médicament; lesdits anticorps inhibant la cascade pro-inflammatoire induite par l'activation de Env-SU MSRV/HERV-W. En particulier, il est utilisé au moins un anticorps anti-Env-SU MSRV/HERV-W et au moins d'un anticorps anti-TLR4. L'anticorps anti-Env-SU HERV-W est choisi parmi les anticorps 3B2H4, 13H5A5 et 3H10F10 et l'anticorps anti-TLR4 est l'anticorps HTA125. Cette utilisation est faite pour le traitement d'une pathologie associée à MSRV/HERV-W, telle que la sclérose en plaques et la schizophrénie.

Lesdits anticorps anti-Env-SU sont en particulier capables de se lier à une région qui correspond aux acides aminés 122-131 (inclus) et/ou à une région qui correspond aux acides aminés 312-316 (inclus) et/ou à une région qui correspond aux acides aminés 181-186 (inclus) de la séquence identifiée en SEQ ID NO : 1.

L'invention a encore pour objet des anticorps choisis parmi les anticorps anti-Env-SU MSRV/HERV-W et anti-TLR4 capables de se lier spécifiquement (se liant spécifiquement) à la fraction soluble de la protéine Env de MSRV/HERV-W ou capables de se lier spécifiquement (se liant spécifiquement) au récepteur TLR4 de la fraction soluble de la protéine Env de MSRV/HERV-W pour inhiber la cascade pro-inflammatoire induite par l'activation de MSRV/HERV-W, en particulier les anticorps 3B2H4, 13H5A5 et 3H10F10. Mais, il est à la portée de l'homme du métier de produire et de sélectionner d'autres anticorps, la condition pour la sélection étant que les anticorps retenus soient capables d'inhiber l'effet pro-inflammatoire de Env-SU dans le test *in vitro* décrit dans la partie expérimentale qui va suivre.

Le terme « anticorps » utilisé dans la présente invention inclut les anticorps monoclonaux, les anticorps chimères, les anticorps humanisés, les anticorps recombinants et les fragments desdits anticorps qui sont caractérisés par une affinité élevée pour la fraction soluble de la protéine d'enveloppe de MSRV/HERV-W et qui ne présentent aucune toxicité ou une très faible toxicité. En particulier, il est préférable d'utiliser un anticorps dont la région variable et/ou la région constante est faiblement immunogène pour l'individu auquel il est administré. Les anticorps de la présente invention sont caractérisés par leur capacité à traiter des patients présentant des pathologies associées à MSRV/HERV-W tout en ne présentant aucune toxicité ou une très faible toxicité. La faible immunogénicité et/ou l'affinité élevée de ces anticorps peut contribuer aux résultats thérapeutiques atteints.

Par fragment d'anticorps on entend les fragments F(ab)2, Fab, Fab', sFv (Blazar et al., 1997, Journal of Immunology 159 : 5821-5833 et Bird et al., 1988, Science 242 : 423-426) d'un anticorps natif et par anticorps chimère on entend, entre autres, un dérivé chimérique d'un anticorps natif (voir par exemple Arakawa et al., 1996, J. Biochem 120 : 657-662 et Chaudray et al., 1989, Nature 339 : 394-397).

La production d'anticorps monoclonaux fait partie des connaissances générales de l'homme du métier. On peut citer à titre de référence Köhler G. et Milstein C. (1975) : Continuous culture of fused cells secreting antibody of predefined specificity, Nature 256 :495-497 et Galfre G. et al. (1977) Nature, 266 : 522-550. L'immunogène peut être couplé à de l'hémocyanine de Lymphet Keyhole (peptide KLH) comme support pour l'immunisation ou à de l'albumine sérique (peptide SA). Les animaux sont soumis à une injection d'immunogène en utilisant de l'adjuvant de Freund. Les sérums et les surnageants de culture d'hybridome issus des animaux immunisés sont analysés pour leur spécificité et leur sélectivité en utilisant des techniques classiques, telles que par exemple des tests ELISA ou de Western Blot. Les hybridomes produisant les anticorps les plus spécifiques et les plus sensibles sont sélectionnés. Des anticorps monoclonaux peuvent également être produits *in* vitro par culture cellulaire des hybridomes produits ou par récupération de liquide d'ascite, après injection intrapéritonéale des hybridomes chez la souris. Quel que soit le mode de production, en surnageant ou en ascite, les anticorps sont ensuite purifiés. Les méthodes de purification utilisées sont essentiellement la filtration sur gel échangeur d'ions et la chromatographie d'exclusion ou la chromatographie d'affinité (protéine A ou G). Les anticorps sont criblés dans des tests fonctionnels pour sélectionner les anticorps les plus performants. La production *in vitro* d'anticorps, de fragments d'anticorps et d'anticorps chimères produits par génie génétique est bien connue de l'homme du métier. A titre d'exemple des anticorps peuvent être produits par clonage du cDNA obtenu à partir de l'ARN codant pour le fragment variable (scFv) de l'anticorps. Les formes « humanisées » d'anticorps non humains, par exemple murins, sont des anticorps chimères qui comprennent une séquence minimale dérivée d'une immunoglobuline non humaine. Pour la plupart, les anticorps humanisés sont des immunoglobulines humaines (anticorps récepteur) dans lesquelles des résidus d'une région hypervariable du récepteur sont remplacés par des résidus d'une région hypervariable d'une espèce donneur (anticorps donneur) non humaine, telle que souris, rat, lapin ou primate non humain, ayant la spécificité, l'affinité et la capacité souhaitées. Dans certains cas, les résidus (FR) de la région Fv de l'immunoglobuline humaine sont remplacés par des résidus correspondants non humains. De plus, les anticorps humanisés peuvent comprendre des résidus qui ne sont pas trouvés dans l'anticorps receveur ou dans l'anticorps donneur. Ces modifications peuvent être effectuées pour améliorer les performances de l'anticorps. En général, l'anticorps humanisé comprendra au moins un et de préférence deux domaines variables, dans lesquels tout ou à peu près tout des boucles hypervariables correspondent à une immunoglobuline non humaine et tout ou à peu près tout des régions FR seront celles d'une immunoglobuline humaine. Les anticorps humanisés facultativement pourront aussi comprendre au moins une partie d'une région constante (Fc) d'une immunoglobuline, telle qu'une immunoglobuline humaine. En général, la région variable est dérivée d'un anticorps mammifère non humain et la région constante est dérivée d'une immunoglobuline humaine. De préférence, la région variable choisie présente une faible immunogénicité et est associée à une région constante qui présente également une faible immunogénicité.

Ces anticorps sont de préférence les anticorps « neutralisants » suivants :

Anticorps monoclonaux anti-Env-SU MSRV/HERV-W : anticorps 3B2H4, 13H5A5 et 3H10F10 (bioMérieux),
Anticorps anti-TLR4 : anticorps monoclonal anti-TLR4 humain HTA125 (commercialisé par la société eBioscience).

Les anticorps anti-Env-SU MSRV/HERV-W sont produits selon les protocoles décrits ci-dessous.

- Production de l'anticorps 3B2H4 :

Les souris sont immunisées selon le protocole suivant : au jour JO, injection intrapéritonéale de 20 $\mu$g d'im-munogène, consistant en de la protéine recombinante purifiée Env/MSRV telles que décrite précédemment [11] en présence d'adjuvant de Freund complet. Aux jours J14 et J28 nouvelle injection intrapéritonéale de la même quantité d'immunogène en présence d'adjuvant de Freund incomplet. Quatre, trois et deux jours avant la fusion faire une injection intrapéritonéale de 100 $\mu$g d'immunogène dilué en eau physiologique.

400 surnageants ont été criblés par technique d'ELISA indirect. Les plaques ont été « coatées » avec 100 $\mu$l d'antigène à 1$\mu$g/ml en tampon bicarbonate 0.05M, pH 9.6. Les plaques « coatées » ont été incubées une nuit à la température de 18-22°C. Les plaques ont été saturées avec 200 $\mu$l de PBS-lait 1% et soumises à incubation 1 heure à 37°+/-2°C. 100$\mu$l de surnageants ou de liquide d'ascites dilués en tampon PBS-Tween 20 0.05% ont été ajoutés et les plaques ont été incubées 1 heure à 37°+/- 2°C. 100$\mu$l d'anticorps polyclonal de chèvre anti-Ig(H+L) de souris conjugué à la phosphatase alcaline (PAL) (Jackson Immunoresearch réf:115-055-062), dilué en tampon PBS-BSA 1% au 1/2000 ont été ajoutés et les plaques ont ensuite été incubées 1 heure à 37°+/-2°C. 100$\mu$l de PNPP (Biomérieux réf 60002990) à la concentration de 2 mg/ml dans de la DEA-HCL (Biomérieux réf 60002989), pH=9,8 ont été ajoutés. Les plaques ont ensuite été soumises à incubation pendant 30 minutes à la température de 37°+/- 2°C. La réaction a été bloquée par addition de 100 $\mu$l de NaOH, 1N. Trois lavages sont effectués entre chaque étape avec 300 $\mu$l de PBS-Tween 20, 0,05%. Un lavage supplémentaire en eau distillée est effectué avant d'ajouter le PNPP.

22 surnageants se sont révélés positifs en ELISA indirect avec une DO > à 0,2 correspondant à quatre fois le bruit de fond. Après les tests de spécificité un seul anticorps est produit.

- Production des anticorps 13H5A5 et 3H10F10 :

Les souris sont immunisées selon le protocole suivant : au jour JO, injection intrapéritonéale de 40 $\mu$g d'im-munogène, consistant en de la protéine recombinante purifiée Env/MSRV telle que décrite [11], en présence d'adjuvant de Freund complet. Aux jours J14, J28 et J78 nouvelle injection intrapéritonéale de la même quantité d'immunogène en présence d'adjuvant de Freund incomplet. Quatre, trois et deux jours avant la fusion faire une injection intrapéritonéale de 50 μg d'immunogène dilué en eau physiologique.

1350 surnageants ont été criblés par technique d'ELISA indirect, comme décrit ci-dessus.

39 surnageants se sont révélés positifs en ELISA indirect avec une DO > à 0,4 correspondant à quatre fois le bruit de fond. Après les tests de spécificité deux anticorps sont produits.

Les anticorps anti-Env-SU et anti-TLR4 précités sont utilisés pour la préparation d'un médicament ou composition thérapeutique pour le traitement de pathologies associées à MSRV/HERV-W, comme décrit ci-dessus. Dans la composition à visée thérapeutique de la présente invention, l'anticorps ou ingrédient actif est associé à un véhicule pharmaceutiquement acceptable et éventuellement à un vecteur pharmaceutiquement acceptable. Les véhicules pharmaceutiquement acceptables sont déterminées et choisis en fonction du mode d'administration retenu et de la pratique standard dans le domaine pharmaceutique. Parce que les protéines sont soumises à digestion quand elles sont administrées par voie orale, une administration parentérale, telle qu'intraveineuse, sous cutanée ou intramusculaire devrait ordinairement être utilisée pour optimiser l'absorption. Les véhicules pharmaceutiquement acceptables sont décrits par exemple dans Remington's Pharmaceutical Sciences 16th ed., Mack Publishing Co. Par exemple, une composition parentérale appropriée pour une administration par injection est préparée en dissolvant 1,5% en poids de l'ingrédient actif dans une solution de chlorure de sodium 0,9%. Il peut être nécessaire d'associer l'anticorps à un vecteur sélectionné qui permette le passage de l'anticorps au travers de la BHE. L'anticorps non-transportable peut ainsi être couplé à un vecteur transportable, tel que l'albumine cationisée, la transférine, l'insuline et le facteur de croissance insuline-like ou à un fragment desdites protéines. Il a notamment déjà été montré que des anticorps monoclonaux non-transportables (IgG3), liés à un vecteur de transport, tel que la transferine ou le facteur de croissance insuline-like, non seulement étaient capables de traverser la BHE, mais également que les propriétés fonctionnelles de ces anticorps étaient conservées. D'autres études ont déjà montré que des produits neuro-pharmaceutiques pouvaient être délivrés au niveau du cerveau par l'intermédiaire de liposomes. Cette approche est également importante car elle offre un mécanisme par lequel toute molécule qui peut être encapsulée dans un liposome peut être dirigée jusqu'au cerveau.

Les anticorps peuvent être administrés soit comme des agents thérapeutiques individuels, soit en combinaison avec d'autres agents thérapeutiques pour accroître et améliorer le traitement. Le dosage dépendra bien sûr de facteurs connus, tels que les caractéristiques pharmacodynamiques de l'agent particulier et de sa voie d'administration, mais également de facteurs tels que l'âge, le poids, la fréquence du traitement et l'effet souhaité et attendu. Habituellement une dose journalière pour un ingrédient actif est comprise entre 0,01 à 100 milligrammes par kilogramme pour un être humain. Ordinairement, 1 à 40 milligrammes par kilogramme par jour, administrés en une ou plusieurs doses journalières, est une quantité efficace pour obtenir l'effet recherché.

L'invention concerne encore l'utilisation de Env-SU MSRV/HERV-W pour déterminer l'état de réactivité des cellules mononuclées sanguines de patients atteints de sclérose en plaques ou de schizophrénie par dosage de cytokines, choisies parmi l'IL-6, l'IL12-p40 et le TNF-α.

Figures.

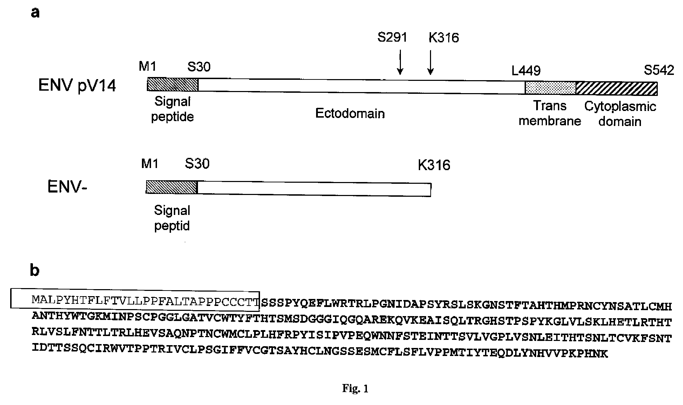

La figure 1 représente les structures de l'enveloppe Env-pV14, du peptide signal et de la fraction soluble de l'enveloppe Env-SU et la séquence en acides aminés du peptide signal et de la fraction soluble de l'enveloppe Env-SU. La figure 1 (a) correspond à la structure de Env-Pv14 (la protéine d'enveloppe complète de MSRV) et à la structure du peptide signal et de la fraction soluble de l'enveloppe Env-SU. La fraction soluble de l'enveloppe (Env-SU) correspond à une fraction de 287 acides aminés représentant l'unité soluble extracellulaire clivée à la position K316 de la protéine complète Env pV14. La figure 1 (b) représente la séquence en acides aminés du peptide signal et de EnvSU. Dans la figure 1 (b) la séquence en acides aminés du peptide signal est encadrée et la fraction soluble de l'enveloppe (Env-SU) est indiquée en caractères gras. La séquence de Env-SU est référencée dans l'identificateur de séquences en SEQ ID NO :1. La séquence complète de l'enveloppe Env pV14 est disponible dans GenBank sous le numéro d'accession AF331500. Les différentes parties de la protéine Env pV14 sont généralement définies comme décrit maintenant, en référence à la figure 1(a) :

le peptide signal commence à l'acide aminé 1 et se termine à l'acide aminé 29 (inclus),

Env-SU commence à l'acide aminé 30 et se termine à l'acide aminé 316 (inclus) et

Le domaine transmembranaire commence à l'acide aminé 317 et se termine à l'acide aminé 542 (inclus).

La masse moléculaire moyenne calculée de Env-SU est égale à 32061.59. Son pI estimé est égal à = 9.61. Sa composition en acides aminés est la suivante :

Acides aminés non-polaires:

| | Nombre | Pourcentage |
|---|---|---|
| A | 9 | 3,14 |

(suite)

|   | Nombre | Pourcentage |
|---|--------|-------------|
| V | 16 | 5,57 |
| L | 25 | 8,71 |
| I | 13 | 4.53 |
| P | 21 | 7,32 |
| M | 7 | 2,44 |
| F | 11 | 3,83 |
| W | 6 | 2,09 |

Acides aminés polaires:

|   | Nombre | Pourcentage |
|---|--------|-------------|
| G | 16 | 5,57 |
| S | 31 | 10,80 |
| T | 34 | 11,85 |
| C | 12 | 4,18 |
| Y | 10 | 3,48 |
| N | 18 | 6,27 |
| Q | 9 | 3,14 |

Acides aminés acides:

|   | Nombre | Pourcentage |
|---|--------|-------------|
| D | 4 | 1,39 |
| E | 10 | 3,48 |

Acides aminés basiques :

|   | Nombre | Pourcentage |
|---|--------|-------------|
| K | 9 | 3,14 |
| R | 12 | 4,18 |
| H | 14 | 4,88 |

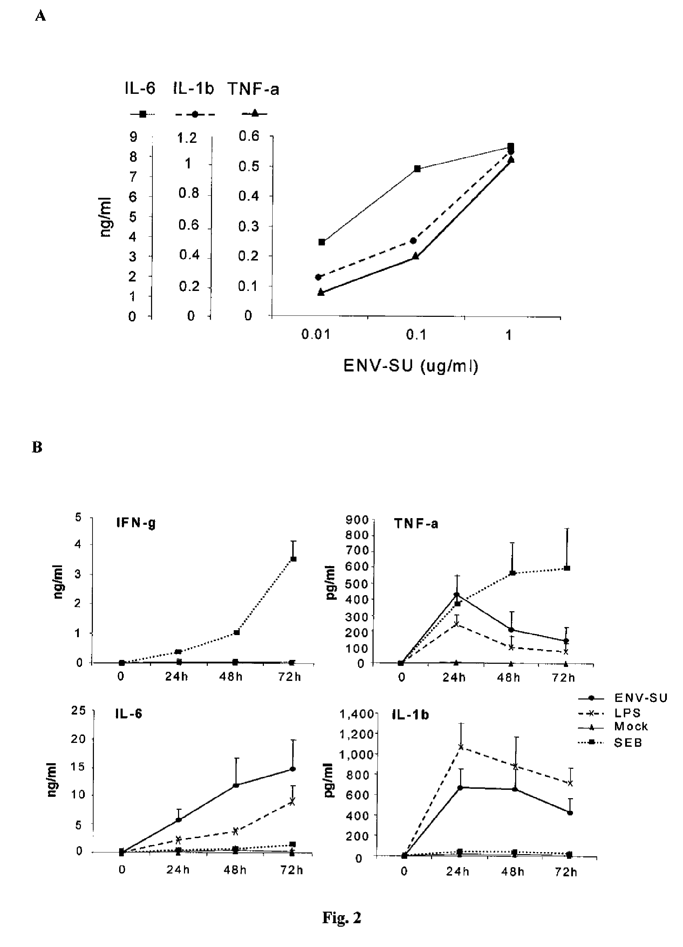

Figure 2: Env-SU induit la production de cytokines pro-inflammatoires dans des cultures de PBMCs (cellules mononuclées) humaines. La figure 2A représente la sécrétion du TNF-$\alpha$, d'IL-1$\beta$ et d'IL-6, analysée par test ELISA (enzyme-linked immunosorbent assay) des surnageants de culture de PBMCs de donneurs sains, stimulés pendant 24 heures avec des doses croissante d'Env-SU. Les résultats correspondent à trois expériences indépendantes. Les doses d'Env-SU sont représentées en abscisse (en $\mu$g/ml). L'axe des ordonnés correspond aux quantités de cytokines (en ng/ml). Dans les courbes, le symbole ■ correspond à la sécrétion d'IL-6, le symbole ● correspond à la sécrétion d'IL-1$\beta$ et le symbole ▲ correspond à la sécrétion du TNF-$\alpha$. Dans la figure 1B, les PBMCs ont été stimulées avec 1 $\mu$g/ml de contrôle autologue, d'Env-SU, de LPS ou de SEB et incubés pendant 24, 48 et 72 heures avant analyse de la sécrétion des cytokines par ELISA. Les axes des abscisses correspondent au temps en heures et les axes des ordonnées correspondent à la production de cytokines IFN$\gamma$, TNF$\alpha$, IL-6 et IL-1$\beta$ en ng/ml pour IFN$\gamma$ et IL-6 (figures 1B (a) et 1B (c)) et en pg/ml pour TNF$\alpha$ et IL-1$\beta$ (figures 1B (b) et 1 B (d)). Dans cette figure -●- correspond à Env-SU, --x-- correspond au LPS, -▲- correspond au contrôle autologue et ······■■······ correspond à SEB.

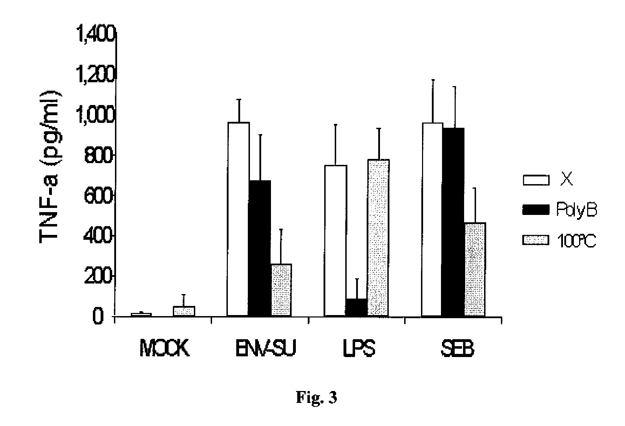

Figure 3 : Les activités stimulant les cytokines de Env-SU ne sont pas dues à une contamination par endotoxines. Les PBMCs ont été stimulées pendant 24 heures avec le contrôle autologue (MOCK), Env-SU, LPS ou SEB. Quand ceci est indiqué, les cellules ont été traitées avec 10 $\mu$g/ml de Polymixine B (PdyB) avant la stimulation (représenté en noir dans la figure). En parallèle, les cellules ont également été incubées avec des protéines et des toxines bouillies (100°C) pendant 30 minutes (représenté en gris dans la figure). Les surnageants de culture ont été récoltés et testés pour la libération du TNF-$\alpha$ par ELISA. Les résultats présentés dans cette figure correspondent à la moyenne de trois expériences. L'axe des ordonnées correspond aux quantités de TNF-$\alpha$ libéré, en pg/ml.

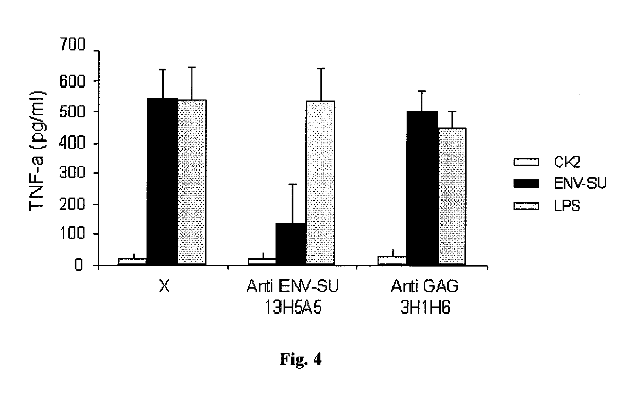

Figure 4 : L'anticorps monoclonal anti-Env-SU (13H5A5) bloque l'activité stimulant les cytokines de Env-SU. Les PBMCs ont été stimulées pendant 24 heures avec 1 $\mu$g/ml du contrôle autologue CK2, Env-SU et LPS et pré-incubées ou non avec 30 $\mu$g/ml d'anticorps monoclonal anti-Env-SU et d'anticorps monoclonal anti-Gag (3H1H6). Les surnageants de culture ont été récoltés et testés pour la sécrétion du TNF-$\alpha$. Les résultats présentés dans cette figure correspondent à la moyenne de trois expériences. L'axe des ordonnées correspond aux quantités de TNF-$\alpha$ libéré, en pg/ml.

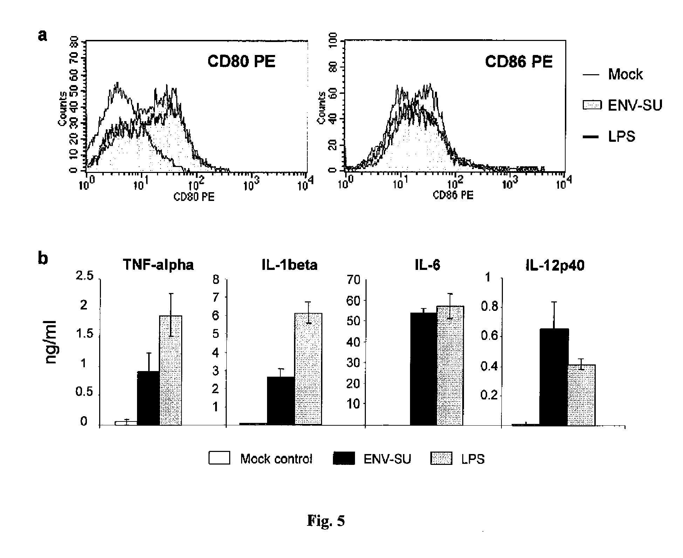

Figure 5 : Env-SU active directement les monocytes humains purifiés. Des monocytes humains ont été purifiés à partir de PBMCs humaines (pureté supérieure à 95%) et ensuite stimulés avec le contrôle autologue (Mock), Env-SU ou LPS, à une concentration de 1 $\mu$g/ml, pendant 24 heures. La figure 5a représente l'expression des marqueurs d'activation CD80 (figure de gauche) et CD86 (figure de droite) analysée par cytométrie de flux. En abscisses est représenté le nombre de cellules comptées et en ordonnés l'intensité de fluorescence par cellule (« counts »). La résultante représente le nombre de cellules comptées pour chaque intensité de fluorescence. L'aire définie par les courbes représente le nombre de cellules totales pour chaque condition testée. La répartition des cellules en fonction de l'intensité de fluorescence est montrée par l'allure de la courbe. L'aire blanche représente les résultats obtenus avec le contrôle témoin (Mock), l'aire grisée, contours traits fins, représente les résultats obtenus avec Env-SU et l'aire grisée, contours très épais, représente les résultats obtenus avec le LPS. La figure 5b représente les sécrétions de TNF-$\alpha$, IL-1$\beta$, IL-6 et IL-12p40, analysées par ELISA. En blanc sont représentés les résultats obtenus après stimulation par le contrôle autologue. Les résultats obtenus après stimulation par Env-SU et LPS sont respectivement représentés en noir et gris. L'axe des ordonnées correspond aux quantités de cytokines sécrétées, en ng/ml. Les résultats représentent la moyenne de trois expériences.

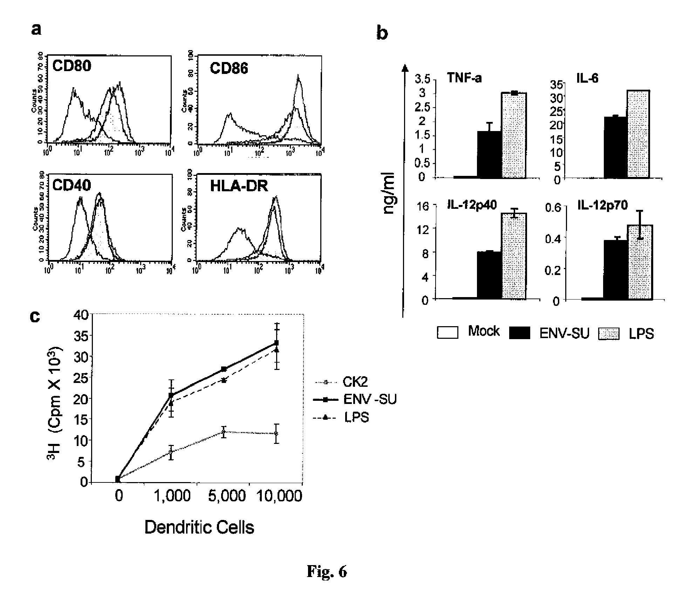

Figure 6 : Env-SU active les cellules dendritiques dérivées de monocytes (MDDC). Les MDDC ont été générées à partir de monocytes purifiés et ensuite stimulées avec le contrôle autologue, Env-SU, ou LPS, à une concentration de 1 $\mu$g/ml, pendant 24 heures. La figure 6a représente l'expression des marqueurs d'activation CD80, CD86, CD40 et HLA-DR, analysée par cytométrie de flux. En abscisses est représenté le nombre de cellules comptées et en ordonnés l'intensité de fluorescence par cellule (« counts »). L'image supérieure gauche représente l'analyse du CD80, l'image supérieure droite celle du CD86, l'image inférieure gauche représente celle du CD40 et l'image inférieure droite celle du HLA-DR. La résultante représente le nombre de cellules comptées pour chaque intensité de fluorescence. L'aire définie par les courbes représente le nombre de cellules totales pour chaque condition testée. La répartition des cellules en fonction de l'intensité de fluorescence est montrée par l'allure de la courbe. L'aire blanche de gauche représente les résultats obtenus avec le contrôle témoin (Mock), l'aire blanche de droite, contours traits plus épais, représente les résultats obtenus avec Env-SU et l'aire grisée représente les résultats obtenus avec le LPS. La figure 6b représente la sécrétion de TNF-$\alpha$, IL-6, IL-12p40 et IL-12p70, analysée par ELISA, dans les surnageants de culture. L'axe des ordonnées correspond aux quantités de cytokines sécrétée, en ng/ml. Dans les histogrammes représentés à la figure 6b, Mock correspond aux résultats obtenus après stimulation avec le contrôle autologue, Env-SU (en noir) correspond aux résultats obtenus après stimulation avec Env-SU et LPS (en gris) correspond aux résultats obtenus après stimulation avec LPS. La figure 6c représente la prolifération allogénique des cellules T par les cellules dendritiques préalablement stimulées par : Env-SU -■-, LPS --▲--, contrôle CK2 -●-. L'axe des abscisses représente le nombre de cellules dendritiques (respectivement 0, 1000, 5000 et 10 000). L'axe des ordonnées représente le nombre de coups par minute émis par les cellules ayant incorporées $^3$H-Thymidine.

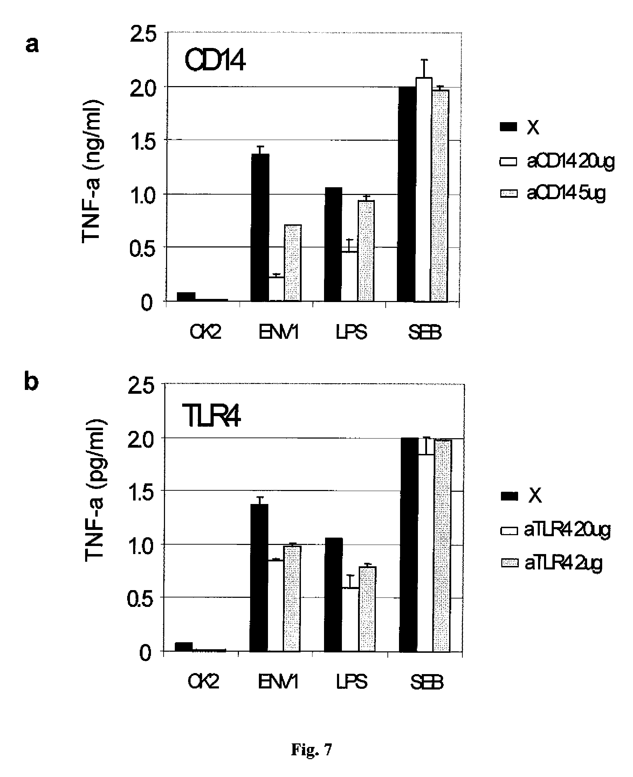

Figure 7 : CD14 et TLR4 sont impliqués dans les propriétés pro-inflammatoires de Env-SU. Les PBMCs ont été pré-incubées pendant une heure avec ou sans anticorps neutralisants anti-CD14 (rhCD14, réf. : AB383, R&D Systems - UK) (figure 7a) et anti-TLR4 (figure 7b), à une concentration de 20 $\mu$g/ml et 5 $\mu$g/ml. Les cellules ont ensuite été stimulées pendant 24 heures avec le contrôle CK2, Env-SU(ENV1), LPS et SEB, à une concentration de 1 $\mu$g/ml. La libération de TNF-$\alpha$ a été analysée, dans les surnageants de culture par ELISA. Les résultats sont montrés dans les histogrammes des figures 7a et 7b. L'axe des ordonnées correspond à la quantité de TNF-$\alpha$ libéré, en ng/ml. Les histogrammes en noir correspondent aux résultats obtenus sans ajout d'anticorps, les histogrammes en blanc correspondent aux résultats obtenus en présence d'anticorps anti-CD14 et anti-TLR4, à 20 $\mu$g/ml et les histogrammes en gris correspondent aux résultats obtenus en présence d'anticorps anti-CD14 et anti-TLR4, à 5 $\mu$g/ml. Les résultats correspondent à la moyenne de trois expériences.

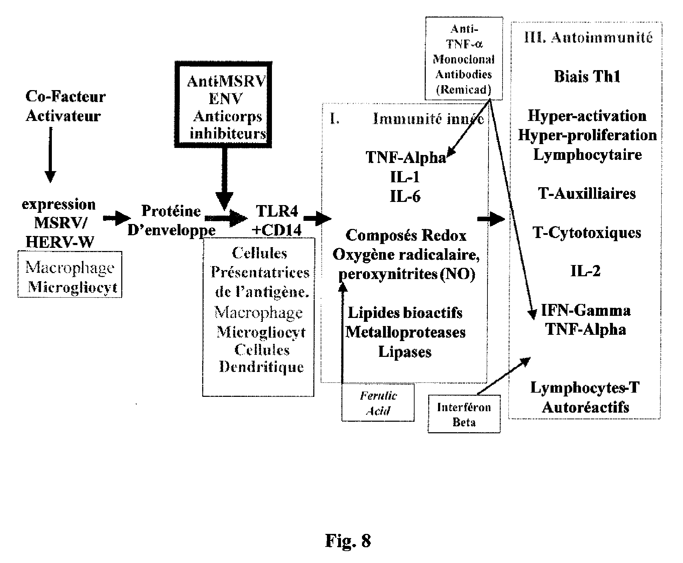

Figure 8 : Cascade d'amplification Immunologique Consécutive à l'activation de la voie TLR4. Exemple des cibles thérapeutiques dans cette cascade.

La figure 8 représente schématiquement la cascade d'activation résultant de l'expression pathologique d'une protéine d'enveloppe MSRV/HERV-W. Celle-ci stimule initialement le récepteur TLR4, en associant éventuellement le co-récepteur CD14. Avant cette interaction il n'y a qu'un agoniste, la protéine MSRV-ENV. Après cette activation, les cellules de l'immunité innée sont activées et des dizaines d'effecteurs moléculaires (cytokines, enzymes, lipides, composés radicalaires ou redox,...) et des cellules activées sont en présence. Dans le cas de la Sclérose en Plaques, une deuxième composante est activée après destruction de la substance blanche du cerveau et présentation d'antigènes de myéline aux lymphocytes-T, à savoir la composante autoimmune liée aux cellules T-autoréactives de l'immunité adaptative. A ce stade, des centaines, voire des milliers de molécules et de cellules différentes sont impliquées dans la médiation des effets immunopathologiques. Nous avons typiquement une cascade d'amplification immunopathologique dont le potentiel est sans commune mesure avec le stimulus initial (ENV MSRV/HERV-W).

Les traitements disponibles ou proposés à ce jour ciblent « en aval », des agonistes pro-inflammatoires ou pathogéniques parmi les nombreux autres agonistes présents au stade de la cascade d'amplification où ils sont apparus (exemples donnés avec les anticorps anti-TNF-alpha, l'interféron beta et une molécule anti-radicalaire comme l'acide ferulique). Dans ce contexte, ils ne peuvent inhiber l'effet des très nombreux autres effecteurs (molécules et cellules) qui ne sont pas sensibles à leur effet pharmacologique. Ceci explique l'effet partiel et relatif de beaucoup de traitements actuels dans une maladie comme la SEP.

De plus, ceux-ci n'empêchent pas que d'autres cellules exprimant (sous l'effet de co-facteurs environnementaux itératifs, comme par exemple, les Herpesviridae) une copie pathogène MSRV/HERV-W produisent de l'enveloppe pro-inflammatoire sur le même site lésionnel, ou dans un autre site cérébral, à la même période ou à une période différente (principe des atteintes multifocales et des rechutes/rémissions qui définissent la SEP dans l'espace cérébral et dans le temps d'évolution de la maladie).

Ainsi, un traitement inhibant l'effet initial à un stade de la cascade où tous les effecteurs aval inductibles par la protéine-cible ne sont pas produit, présente une pertinence et un efficacité potentielle bien supérieure aux approches communément conçues et mises en oeuvre dans ces pathologies associées aux effets pro-inflammatoires de cette protéine d'enveloppe MSRV/HERV-W. En effet, même si la cascade est activée, elle sera « tarie » en amont par cette stratégie thérapeutique, alors que la stimulation en amont continuera dans les autres approches thérapeutiques « en aval ». Enfin, en approche préventive des nouvelles rechutes pendant les périodes de rémission de la maladie, ces anticorps peuvent neutraliser les protéines « ENV MSRV/HERV-W » avant qu'elles n'enclenchent la cascade décrite ici, alors que les autres thérapies ciblant les molécules ou les cellules en « aval » ne peuvent intervenir qu'après mise en action de cette cascade inflammatoire !

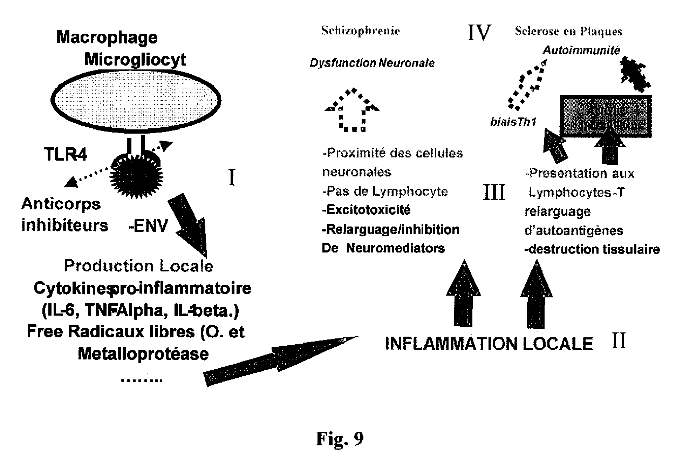

Figure 9 : Quatre étapes clés pour aboutir à deux maladies à partir de l'effet pro-inflammatoire de la protéine ENV MSRV/HERV-W:

(i) deux étapes « amont » communes: I-Activation des récepteurs TLR4 et II-inflammation locale
(ii) deux étapes « aval » différentes: III-démyélinisation ou excitotoxicité neuronale et IV-Sclérose en plaques ou Schizophrénie

La protéine d'enveloppe (Env) est produite par une copie rétrovirale de la famille MSRV/HERV-W, dans un contexte d'activation pathologique comme, par exemple, après transactivation par un co-facteur infectieux de la famille des herpesviridae [46-49] dans un territoire tissulaire déterminé par le tropisme de ce co-facteur et par la présence de cellules dans le tissu cible hébergeant au moins une copie provirale MSRV/HERV-W activable par ce co-facteur et codant pour une protéine d'enveloppe.

Cette protéine ENV ainsi produite se lie au récepteur TLR4 et, selon le contexte, aux co-récepteurs de TLR4 comme le CD14, des cellules de type macrophage ou microgliocyte présente dans le tissu cérébral à proximité de la cellule productrice d'ENV MSRV/HERV-W et/ou de virions MSRV. Si cette dernière est un macrophage ou un microgliocyte, l'effet autocrine sur les recepteurs TLR4 de cette même cellule est possible.

Après cette étape, d'interaction avec le récepteur TLR4, la cascade d'amplification immunopathologique crée, avec la production de dizaines de molécules pro-inflammatoire et médiatrices de destruction tissulaire, une inflammation locale dans le tissu concerné, autour du site de réactivation MSRV/HERV-W.

Après ce stade, la situation diverge selon que les co-facteurs de réactivation MSRV/HERV-W et la localisation des cellules hébergeant ces provirus « répondeurs », ont convergé vers une expression dans la substance blanche ou dans la substance grise.

Dans le premier cas, qui détermine la voie pathologique aboutissant à une pathologie comme la Schizophrénie, la réactivation ou la surexpression induite d'un élément MSRV/HERV-W à proximité des structures neuronales du cortex frontal induit une inflammation locale qui, dans le contexte tissulaire du la substance grise, ne va pas permettre une activité pro-lésionnelle majeure et un recrutement immunitaire spécifique. Cette inflammation locale ne permettra pas une infiltration de lymphocytes T à ce niveau. Par contre, les médiateurs pro-inflammatoires produits à proximité des cellules neuronales responsables des activités « intellectuelles » et cognitives, provoquent une excitotoxicité neuronale focale qui détermine un dysfonctionnement des réseaux neuronaux associés dans l'espace cérébral affecté et dans le temps que dure cette production pro-inflammatoire [17, 20, 22-26, 29, 50, 51]. Selon les territoires touchés, les troubles « psychiques » résultant des activations neuronales excitotoxiques se traduisent par des manifestations hallucinatoires et délirantes qui caractérisent les accès clinicopathologiques de la Schizophrénie. A terme, il est connu que cette excitotoxicité neuronale peut déboucher sur une mort cellulaire (neurotoxicité), ce qui est objectivé par l'élargissement ventriculaire mesuré en IRM dans le cerveau des patients atteints de Schizophrénie[52].

Dans le second cas, qui détermine la voie pathologique aboutissant à la Sclérose en Plaques, la myéline de la substance blanche est extrêmement sensible aux agents radicalaires et pro-inflammatoires qui génèrent une démyélinisation primaire avec présentation d'auto-antigènes aux lymphocytes recrutés par l'inflammation préalable. Dans ces conditions, des biais de réactivité des lymphocytes sont conditionnés par les cytokines sécrétées préalablement par les cellules de type microgliocyte/macrophage (biais Th1) qui peuvent suffire à générer une réponse autoimmune face à des antigènes du « soi » présentés dans ces conditions. Mais, de surcroît, il a été montré que la protéine d'enveloppe MSRV entière ou les virions MSRV pouvaient exercer une activité différente au niveau des lymphocytes T, à savoir une activité de superantigénique caractérisée par une interaction avec le récepteur « TCR ». Cette dernière propriété vient, dans ce contexte « aval » où des lymphocytes T recrutés par l'inflammation primaire infiltrent le tissu, ajouter in fine une activation polyclonale des lymphocytes T qui favorise encore plus la réponse lymphocytaire T autoimmune spécifique aux antigènes de myéline exposés dans le tissu préalablement lésé par l'inflammation primaire. Dans ce contexte, une deuxième dimension de la réaction immunopathologique intervient alors avec les effets de l'autoimmunité et de l'inflammation médiée par les lymphocytes T activés.

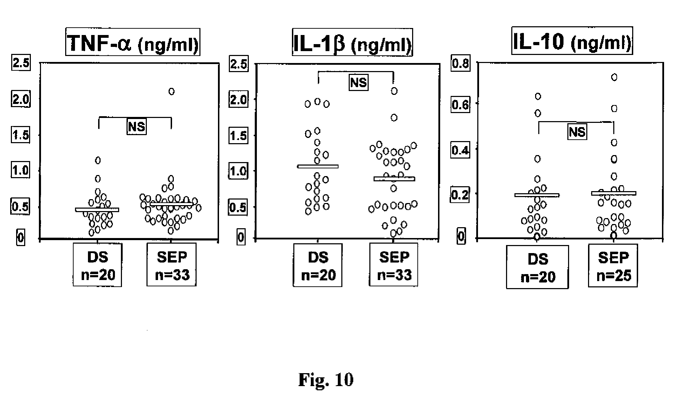

Figure 10 : Production de cytokines induites par ENV-SU sur des PBMC de patients atteints de sclérose en Plaques (SEP) et de donneurs sains (DS): TNF-alpha, IL-1Beta et IL-10

La figure 10 représente la production de cytokines induite par la protéine ENV-SU MSRV dans les cellules mononuclées sanguines (PBMC) prélevées ex vivo d'une part chez des patients atteints de sclérose en plaques (SEP), et d'autre part chez des donneurs sains (DS). L'indication « n= » à côté de DS ou de SEP donne le nombre de personnes testées pour chaque population vis-à-vis de la cytokine représentée.

L'axe des abscisses représente le dosage des cytokines dans le surnageant des cultures de PBMC stimulés en ng/ml. Chaque graphique compare les résultats pour chaque individu testé, représenté par un point (rond) dans chaque population (DS et SEP). Les Trois graphiques représentent, de gauche à droite, la production de « tumour necrosis factor » (TNF)-alpha, d'interleukine (IL)-1beta et d'interleukine(IL)-10. Comme cela a été calculé, les résultats comparés des populations DS et SEP ne sont pas significativement différents pour ces trois cytokines (non significatifs, NS). L'analyse statistique a été effectuée avec le test t de Student.

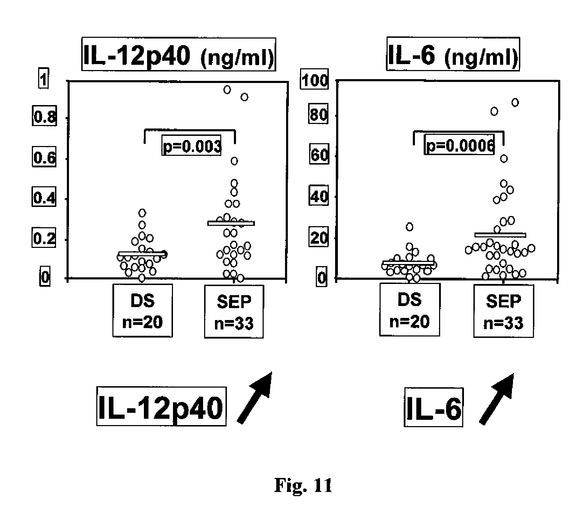

Figure 11 : Production de cytokines induites par ENV-SU sur des PBMC de patients atteints de sclérose en Plaques (SEP) et de donneurs sains (DS): IL-12p40 et IL-6.

La figure 11 représente la production de cytokines induite par la protéine ENV-SU MSRV dans les cellules mononuclées sanguines (PBMC) prélevées ex vivo d'une part chez des patients atteints de sclérose en plaques (SEP), et d'autre part chez des donneurs sains (DS). L'indication « n= » à côté de DS ou de SEP donne le nombre de personnes testées pour chaque population vis-à-vis de la cytokine représentée.

L'axe des abscisse représente le dosage des cytokines dans le surnageant des cultures de PBMC stimulés en ng/ml. Chaque graphique compare les résultats pour chaque individu testé, représenté par un point (rond) dans chaque population (DS et SEP). Les deux graphiques représentent, de gauche à droite, la production d'interleukine (IL)-12p40 et d'interleukine (IL)-6. Comme cela a été calculé, les résultats comparés des populations DS et SEP sont très significativement plus élevés dans la population SEP pour ces deux cytokines (p= 0,003 pour l'IL-12p40 et p=0,0006 pour l'IL-6). L'analyse statistique a été effectuée avec le test t de Student.

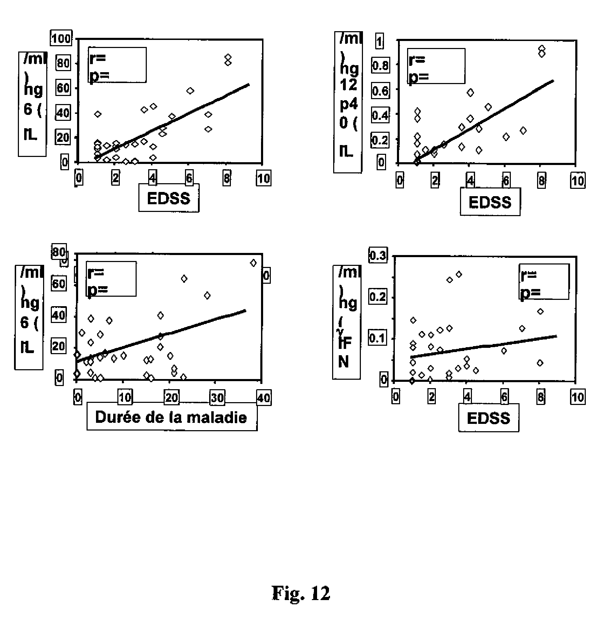

Figure 12 : Corrélations entre les productions de cytokines et les paramètres cliniques des patients.

La figure 12 représente les résultats graphiques de l'analyse de corrélation entre des paramètres cliniques de la population SEP étudiée (en abscisse) et les taux de certaines cytokines (en ordonnée) produites en réponse de la stimulation de leur PBMC par la protéine ENV MSRV-SU. Pour chaque graphique, la valeur de « r » représente le calcul statistique de distribution des points par rapport à la droite de corrélation. La valeur de « p » représente la probabilité statistique que cette corrélation soit obtenue par hasard ; ainsi, toute valeur de p supérieure à 0,05 est « non significative » et toute valeur inférieure à 0,05 est significative d'une corrélation existante entre les facteurs analysés.

Les deux graphiques du haut montrent les paramètres qui ont été trouvés avec une corrélation significative entre le score clinique des patients SEP, EDSS [53] mesurée sur une échelle de gravité de 1 à 10, et l'IL-6 (à gauche) ou l'IL12p40 (à droite).

Les deux graphiques du bas montrent deux exemples des paramètres qui n'ont pas été trouvés significativement corrélés : la durée de la maladie et l'IL-6 (à gauche) ou l'interféron gamma et le score clinique EDSS (à droite).

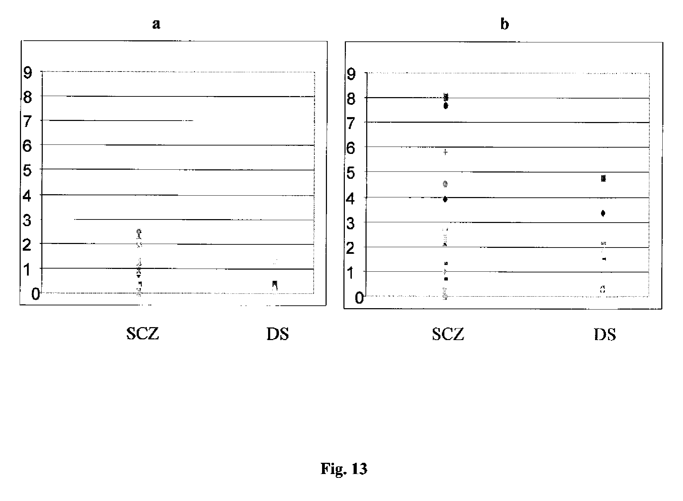

Figure 13 : Production spontanée (a) ou induite par ENV-SU (b) de cytokines dans des PBMC de patients atteints de Schizophrénie (SCZ) et de donneurs sains (DS): IL-10.

La figure 13 représente la production de cytokines induite par la protéine ENV-SU MSRV dans les cellules mononuclées sanguines (PBMC) prélevées ex vivo d'une part chez des patients atteints de schizophrénie (SCZ), et d'autre part chez des donneurs sains (DS).

L'axe des abscisses représente le dosage des cytokines dans le surnageant des cultures de PBMC stimulés en ng/ml. Chaque graphique compare les résultats pour chaque individu testé, représenté par un point dans chaque population (DS et SEP). Les deux graphiques représentent, de gauche à droite, la production d'interleukine (IL)-10 spontanée en culture et d'interleukine(IL)-10 induite après la stimulation par ENV-SU.

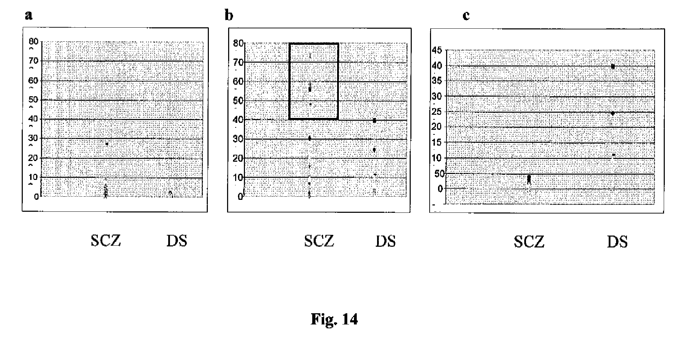

Figure 14 : Production spontanée (a) ou induite par ENV-SU (b) de cytokines dans des PBMC de patients atteints de Schizophrénie (SCZ) et de donneurs sains (DS), avec calcul de l'accroissement relatif (c) : IL-12p40.

La figure 14 représente la production de cytokines induite par la protéine ENV-SU MSRV dans les cellules mononuclées sanguines (PBMC) prélevées ex vivo d'une part chez des patients atteints de schizophrénie (SCZ), et d'autre part chez des donneurs sains (DS).

L'axe des abscisses représente le dosage des cytokines dans le surnageant des cultures de PBMC stimulés en ng/ml. Chaque graphique compare les résultats pour chaque individu testé, représenté par un point dans chaque population (DS et SEP). Les trois graphiques représentent, de gauche à droite : a) la production d'interleukine (IL)-12p40 spontanée en culture, b) d'interleukine(IL)-10 induite après la stimulation par ENV-SU et c) l'accroissement relatif de production d'IL12p40 calculé selon la formule :

```
(Taux après stimulation ENV-SU - Taux spontané) / Taux
spontané.
```

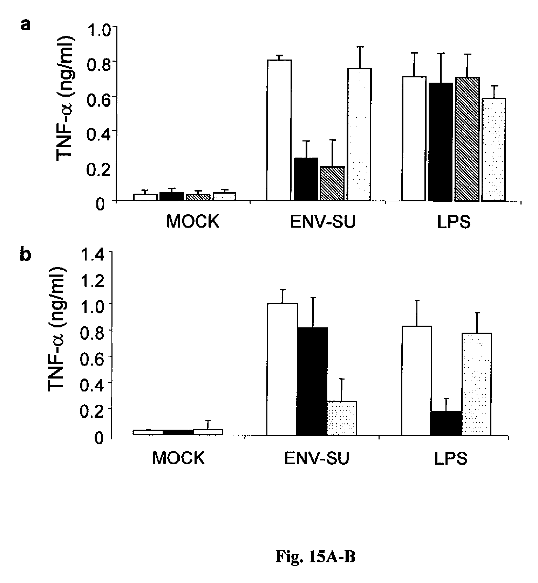

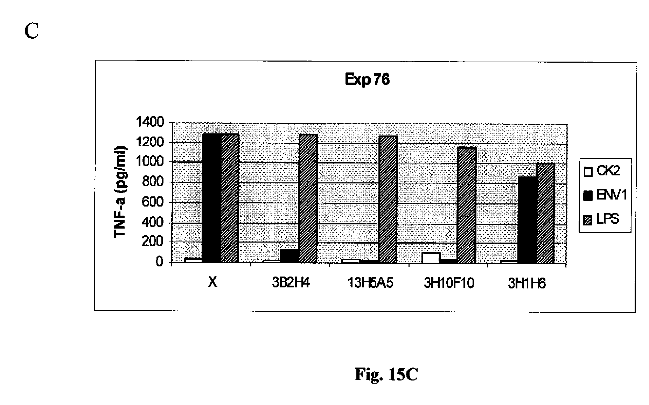

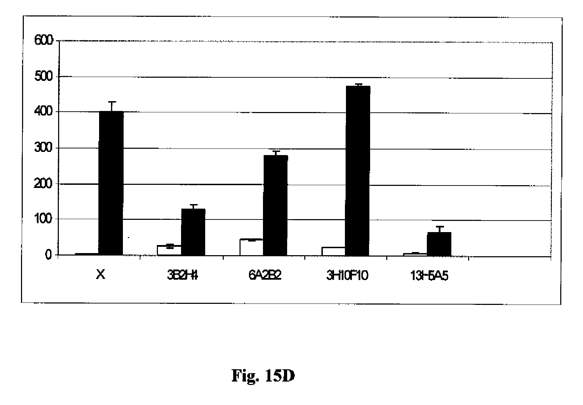

Figure 15 : Identification et sélection des anticorps monoclonaux anti-ENV MSRV/HERV-W inhibiteurs de l'activation pro-inflammatoire des monocytes macrophages induite par la protéine ENV-SU, dans des cultures de PBMC humains provenant de donneurs sains. a) analyse avec deux anticorps ant-ENV et un anticorps témoin b) vérification des conditions de spécificité de l'analyse c) exemple d'expérience indépendante d) autre exemple d'expérience indépendante :

La figure 15a représente, en ordonnée, la sécrétion de TNF-alpha (ng/ml) induite par (en abscisse) la protéine contrôle « Mock » (1microgramme/ml), ENV-SU (1microgramme/ml) et le LPS (1microgramme/ml), dans une culture de PBMC d'un donneur sain. De gauche à droite pour chaque condition de stimulation : la barre blanche représente le résultat en l'absence d'anticorps, la barre noire représente le résultat en présence d'anticorps anti-ENV MSRV 3B2H4 (30 microgramme/ml), la barre hachurée représente le résultat en présence d'anticorps anti-ENV MSRV13H5A5 (30 microgramme/ml) et la barre grisée représente le résultat en présence d'anticorps anti-GAG MSRV 3H1H6 (30 microgramme/ml).

La figure 15b représente, en ordonnée, la sécrétion de TNF-alpha(ng/ml) induite par (en abscisse) la protéine contrôle « Mock » (1microgramme/ml), ENV-SU (1microgramme/ml) et le LPS (1microgramme/ml), dans une culture de PBMC du même donneur sain qu'en 15a. De gauche à droite pour chaque condition de stimulation : la barre blanche représente le résultat en l'absence d'anticorps, la barre noire représente le résultat en présence de polymixine B (25 microgramme/ml) et la barre grisée représente le résultat obtenu avec MOCK, ENV-SU ou LPS chauffé à 100°C pendant 30 minutes, préalablement à leur addition dans la culture de PBMC.

La figure 15c représente, en ordonnée, la sécrétion de TNF-alpha (pg/ml) induite par la protéine contrôle « Mock » illustrée par une barre blanche (1microgramme/ml), ENV-SU illustrée par une barre noire (1microgramme/ml) et le LPS illustré par une barre hachurée (1microgramme/ml), dans une culture de PBMC d'un donneur sain. De gauche à droite pour chaque condition de stimulation, les résultats sont donnés pour les anticorps indiqués en abscisse : anticorps « X » anti-toxoplasme de même isotype que 3B2H4 (30 microgramme/ml), anticorps anti-ENV MSRV 3B2H4 (30 microgramme/ml, anticorps anti-ENV MSRV 13H5A5 (30 microgramme/ml), anticorps anti-ENV MSRV 3H10F10 (30 microgramme/ml) anticorps anti-GAG MSRV 3H1H6 (30 microgramme/ml).

La figure 15d représente, en ordonnée, la sécrétion de TNF-alpha (pg/ml) induite par la protéine contrôle « Mock CK2 » illustrée par une barre blanche (1microgramme/ml) et ENV-SU illustrée par une barre noire (1microgramme/ml), dans une culture de PBMC d'un donneur sain. De gauche à droite pour chaque condition de stimulation, les résultats sont donnés pour les anticorps indiqués en abscisse : anticorps « X » anti-toxoplasme de même isotype que 3B2H4 (30 microgramme/ml), anticorps anti-ENV MSRV 3B2H4 (30 microgramme/ml, anticorps anti-ENV MSRV 6A2B2 (30 microgramme/ml), anticorps anti-ENV MSRV 3H10F10 (30 microgramme/ml) et anticorps anti-ENV MSRV 13H5A5 (30 microgramme/ml).

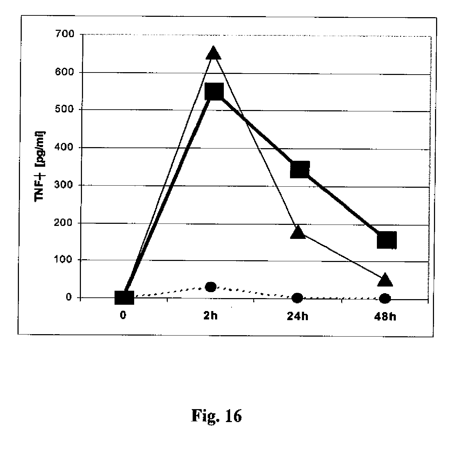

Figure 16 : Cinétique de production du TNF-$\alpha$ sur PBMCs

Des PBMCs de donneurs sains ont été stimulés avec 5$\mu$l de tampon (courbe en pointillés et ronds), 1$\mu$g/ml de ENV-SU (courbe en trait épais avec carrés) ou 1$\mu$g/ml de LPS (courbe en trait fin avec triangles) et incubés pendant 2h, 24h ou 48h (axe des abscisses), avant analyse de la production de TNF-$\alpha$ par ELISA (axe des ordonnées en pg/ml).

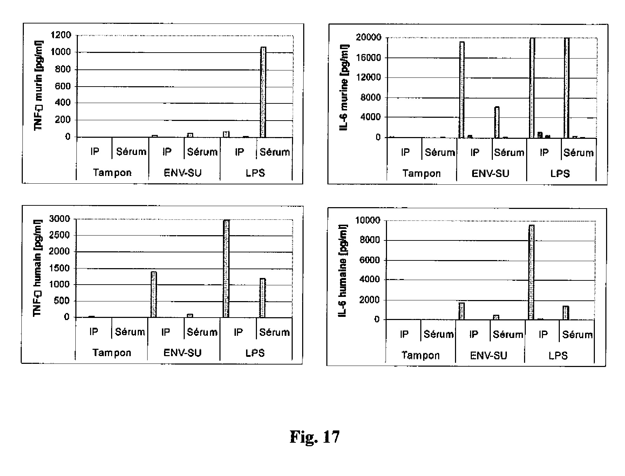

Figure 17 : Effets pro-inflammatoires de ENV-SU chez la SCID-humanisée.

Les souris SCID d'environ 25g ont reçu, comme indiqué en abscisse, des injections de Tampon, de 50$\mu$g d'ENV-SU par animal ou de 50$\mu$g de LPS par animal. Comme indiqué aussi en abscisse pour chaque type d'inoculum, le sérum ou le liquide issu du lavage péritonéal (IP) des souris sacrifiées à 2h, 24h et 48h ont été analysés par ELISA.

Les graphiques de gauche représentent le dosage du TNF-alpha (pg/ml). Celui du haut représente le dosage de la cytokine murine et celui du bas, de la cytokine humaine.

Les graphiques de droite représentent le dosage de l'IL-6 (pg/ml). Celui du haut représente le dosage de la cytokine murine et celui du bas, de la cytokine humaine.

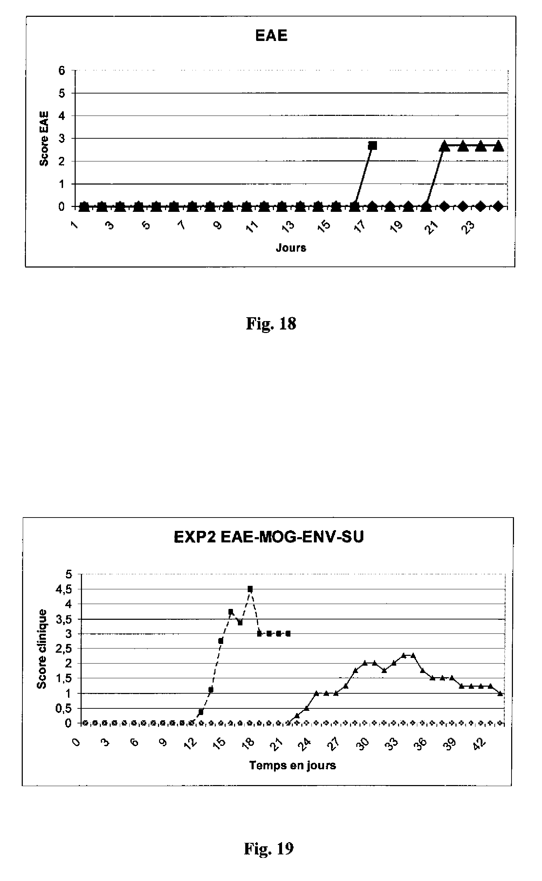

Figure 18 : Induction d'EAE par la protéine ENV MSRV

La figure 18 représente les résultats d'une expérience préliminaire d'induction d'EAE par la protéine ENV MSRV dans des souris C57B16.

L'axe des abscisses représente les jours après l'injection. L'axe des ordonnées représente le score clinique moyen des animaux étudiés.

La courbe avec les carrés représente les animaux témoins positifs injectés avec l'autoantigène MOG (myelin oligodendrocyte glycoprotein) et l'adjuvant complet de Freund (contenant l'extrait de Mycobacterieum tuberculosis). L'étude de cette série a été finalisée ici à 18 Jours.

La courbe avec les triangles représente les animaux injectés avec l'autoantigène MOG (myelin oligodendrocyte glycoprotein), l'adjuvant incomplet de Freund (ne contenant pas l'extrait de M. tuberculosis) et la protéine ENV-SU MSRV. L'étude de cette série a été poursuivie ici jusqu'à 25 Jours.

La courbe avec les losanges représente les animaux témoins négatifs injectés avec l'autoantigène MOG (myelin oligodendrocyte glycoprotein) et l'adjuvant incomplet de Freund (ne contenant pas l'extrait de M. tuberculosis). L'étude de cette série a été poursuivie ici jusqu'à 25 Jours.

Figure 19 : reproduction de l'induction d'EAE par la protéine ENV MSRV

La figure 19 représente les résultats d'une expérience confirmant l'induction d'EAE par la protéine ENV MSRV dans des souris C57B16.

L'axe des abscisses représente les jours après l'injection. L'axe des ordonnées représente le score clinique moyen des animaux étudiés.

La courbe avec les carrés représente les animaux témoins positifs injectés avec l'autoantigène MOG (myelin oligodendrocyte glycoprotein) et l'adjuvant complet de Freund (contenant l'extrait de Mycobacterieum tuberculosis). L'étude de cette série a été finalisée ici à 21 Jours.

La courbe avec les triangles représente les animaux injectés avec l'autoantigène MOG (myelin oligodendrocyte glycoprotein), l'adjuvant incomplet de Freund (ne contenant pas l'extrait de M. tuberculosis) et la protéine ENV-SU MSRV. L'étude de cette série a été poursuivie ici jusqu'à 42 Jours.

La courbe avec les losanges représente les animaux témoins négatifs injectés avec l'autoantigène MOG (myelin oligodendrocyte glycoprotein) et l'adjuvant incomplet de Freund (ne contenant pas l'extrait de M. tuberculosis). L'étude de cette série a été poursuivie ici jusqu'à 42 Jours.

La courbe avec les croix représente les animaux témoins négatifs injectés avec l'autoantigène MOG (myelin oligodendrocyte glycoprotein), l'adjuvant incomplet de Freund (ne contenant pas l'extrait de M. tuberculosis) et du LPS. L'étude de cette série a été poursuivie ici jusqu'à 42 Jours.

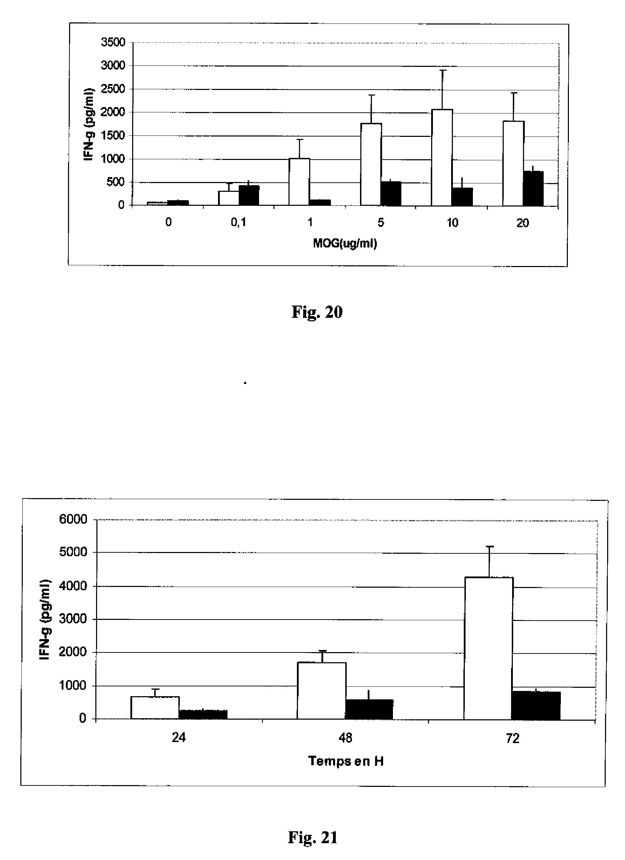

Figure 20 : Dosage à 24h de la réponse autoimmune à des doses croissantes d'autoantigène MOG dans les souris du modèle « EAE/MOG/ENV-SU » et dans le contrôle immunisé sans ENV .

L'axe des abscisses représente les concentrations d'autoantigène MOG (microgramme/ml) mis en présence des PBMC de souris prélevées au cours du protocole illustré dans la figure 19. L'axe des ordonnées représente le dosage d'Interféron gamma sécrété in vitro par les lymphocytes T autoimmuns présents dans les PBMC mis en présence de doses croissante d'antigène MOG.

Les barres blanches représentent les PBMC de souris ayant reçu en injection in vivo (au jour « 0 » de la série illustrée en figure 19) l'autoantigène MOG (myelin oligodendrocyte glycoprotein), l'adjuvant incomplet de Freund (ne contenant pas l'extrait de M. tuberculosis) et la protéine ENV-SU MSRV.

Les barres noires représentent les PBMC de souris témoins ayant reçu en injection in vivo (au jour « 0 » de la série illustrée en figure 19) l'autoantigène MOG (myelin oligodendrocyte glycoprotein) et l'adjuvant incomplet de Freund (ne contenant pas l'extrait de M. tuberculosis).

Figure 21: Cinétique de la réponse lymphocytaire T autoimmune anti-MOG révélée par la sécrétion d'interféron gamma dans les souris du modèle « EAE/MOG/ENV-SU » et dans le contrôle immunisé sans ENV .

L'axe des abscisses représente les délais post-inoculation de la préparation « MOG et adjuvants » (en heures) auxquels ont été prélevés les PBMC sur les souris de la série illustrée dans la figure 19. L'axe des ordonnées représente le dosage d'Interféron gamma sécrété in vitro par les lymphocytes T autoimmuns présents dans les PBMC.

Les barres blanches représentent les PBMC de souris ayant reçu en injection in vivo (au jour « 0 » de la série illustrée en figure 19) l'autoantigène MOG (myelin oligodendrocyte glycoprotein), l'adjuvant incomplet de Freund (ne contenant pas l'extrait de M. tuberculosis) et la protéine ENV-SU MSRV .

Les barres noires représentent les PBMC de souris témoins ayant reçu en injection in vivo (au jour « 0 » de la série illustrée en figure 19) l'autoantigène MOG (myelin oligodendrocyte glycoprotein) et l'adjuvant incomplet de Freund (ne contenant pas l'extrait de M. tuberculosis).

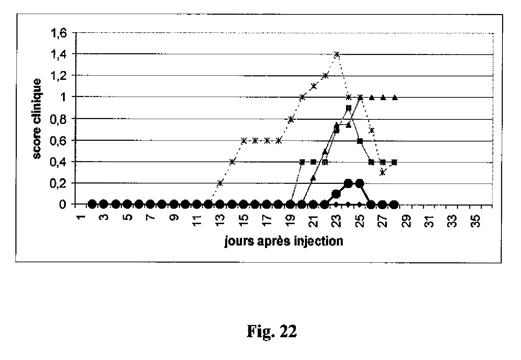

Figure 22 Activité thérapeutique des anticorps anti-ENV MSRV sélectionnés pour leur effet inhibiteur de l'activation pro-inflammatoire de ENV-SU, mise en évidence dans un modèle de SEP mis au point et validé dans la présente invention

La figure 22

Représente les résultats d'une expérience confirmant l'induction d'EAE par la protéine ENV MSRV dans des souris C57B16 et l'effet inhibiteur des anticorps monoclonaux anti-ENV MSRV/HERV-W préalablement sélectionnés dans le test d'inhibition de l'activité pro-inflammatoire médiée par TLR4, induite par le fragment soluble ENV-SU de la protéine ENV.

L'axe des abscisses représente les jours après l'injection. L'axe des ordonnées représente le score clinique moyen des animaux étudiés.

La courbe avec les carrés représente les animaux témoins positifs injectés avec l'autoantigène MOG (myelin oligodendrocyte glycoprotein) et l'adjuvant complet de Freund (contenant l'extrait de Mycobacterium tuberculosis). L'étude de cette série a été finalisée ici à 28 Jours.

La courbe avec les triangles représente les animaux injectés avec l'autoantigène MOG (myelin oligodendrocyte glycoprotein), l'adjuvant incomplet de Freund (ne contenant pas l'extrait de M. tuberculosis) et la protéine ENV-SU MSRV. L'étude de cette série a été poursuivie ici jusqu'à 28 jours.

La courbe avec les losanges représente les animaux témoins négatifs injectés avec l'autoantigène MOG (myelin oligodendrocyte glycoprotein) et l'adjuvant incomplet de Freund (ne contenant pas l'extrait de M. tuberculosis). L'étude de cette série a été poursuivie ici jusqu'à 28 Jours.

La courbe en pointillé avec les croix représente les animaux injectés avec l'autoantigène MOG (myelin oligodendrocyte glycoprotein), l'adjuvant incomplet de Freund (ne contenant pas l'extrait de M. tuberculosis) et la protéine ENV-SU MSRV. Ces animaux ont de surcroît reçu une dose de 50 microgramme par Kilo, soit un microgramme pour une souris de 20 grammes, d'anticorps témoin anti-GAG MSRV 3H1H6. L'étude de cette série a été poursuivie ici jusqu'à 28 jours.

La courbe avec les ronds représente les animaux injectés avec l'autoantigène MOG (myelin oligodendrocyte glycoprotein), l'adjuvant incomplet de Freund (ne contenant pas l'extrait de M. tuberculosis) et la protéine ENV-SU MSRV. Ces animaux ont de surcroît reçu une dose de 50 microgramme par Kilo, soit un microgramme pour une souris de 20 grammes, d'anticorps témoin anti-ENV MSRV 3B2H4. L'étude de cette série a été poursuivie ici jusqu'à 28 jours.

Figure 23 : Séquences aminoacides comparées entre la protéine ENV MSRV (ligne du bas) et la protéine ENV codée par la copie HERV-W 7q (ligne du haut) les séquences encadrées sont identiques (régions conservées).

Figure 24: Analyse en Western-Blot.

Croisement antigénique entre la protéine ENV MSRV et la protéine ENV codée par l'orf env de la copie HERV-W localisée ubiquitairement sur le chromosome 7q

V14= protéine recombinante ENV codée par le clone MSRV pV14

H74= protéine recombinante ENV codée par le clone HERV-W7q pH74

3C1D5 : anticorps monoclonal obtenu a près immunisation avec des protéines recombinantes issues de clones MSRV.

La flèche montre le niveau des bandes détectées. 60 Kda indique le niveau du poids moléculaire correspondant.

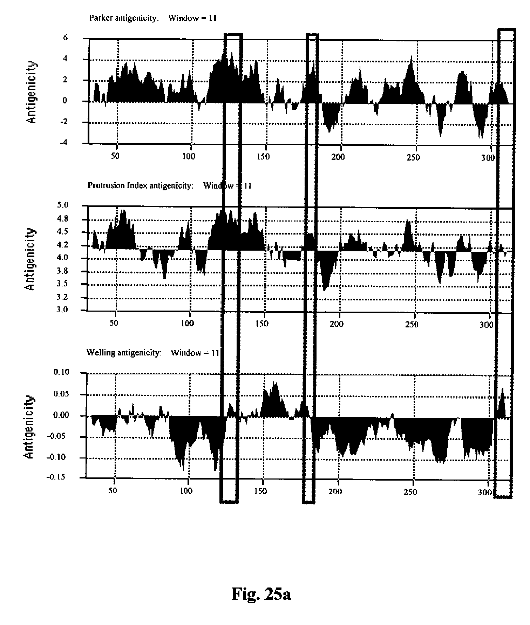

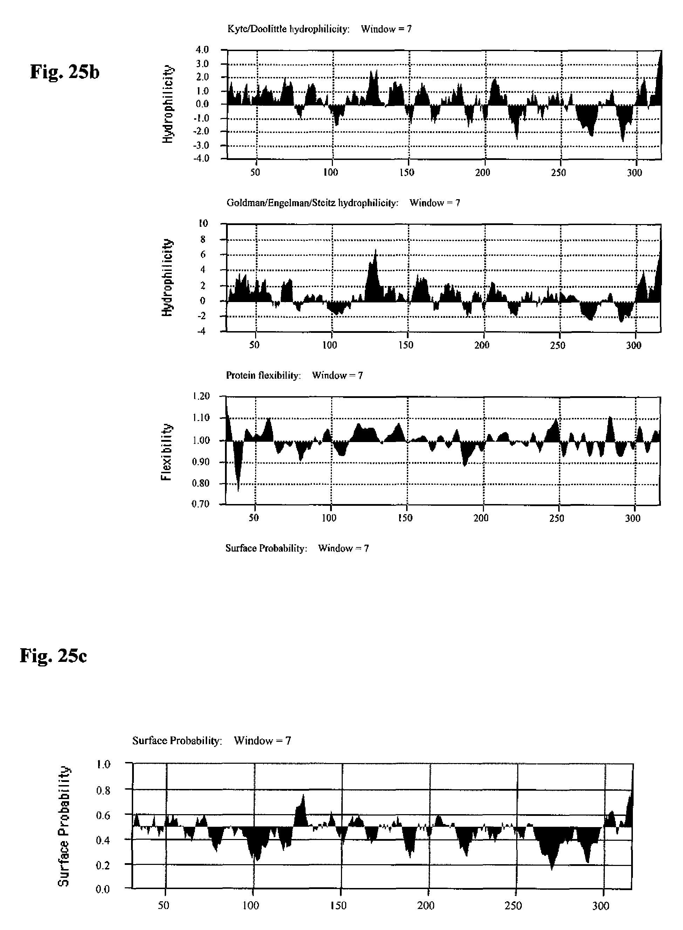

Figure 25 : Analyse des propriétés antigéniques de la protéine ENV-SU

Analyse en Western-Blot.

Les figures 25a, b et c représentent l'analyse de la séquence aminoacide de la protéine MSRV ENV-SU par le logiciel d'analyse « Mac Vector », avec la fonction « Protein analysis toolbox ». Les régions encadrées par les 3 rectangles verticaux représentent les trois régions antigéniques les plus probables selon une analyse des séquences primaires et secondaires.

Figure 25a, trois graphiques qui illustrent l'antigénicité des régions « ENV-SU ». Les aires grisées au dessus du « 0 » de l'axe des ordonnées ont une antigénicité positive, celle en dessous n'en ont pas (antigénicité négative).

Figure 25b, les deux graphiques du haut illustrent l'hydrophilicité des régions « ENV-SU ». Les aires grisées au dessus du « 0 » de l'axe des ordonnées ont une hydrophilicité positive, celle en dessous ont une hydrophilicité négative.

Le graphique du bas illustre la flexibilité des régions « ENV-SU ». Les aires grisées au dessus du « 0 » de l'axe des ordonnées ont une flexibilité positive, celle en dessous ont une flexibilité négative.

Figure 25c, le graphique illustre la probabilité de surface des régions « ENV-SU ». Les aires grisées au dessus du « 0 » de l'axe des ordonnées ont une flexibilité positive, celle en dessous ont une probabilité de surface négative.

**PARTIE EXPERIMENTALE**

**Etudes *in vitro.***

**Matériels et méthodes**

Protéines et toxines.

La protéine de surface de l'enveloppe de MSRV (Env-SU) correspond à une séquence protéique de 287 acides aminés de la protéine totale d'enveloppe (Env Pv14, GenBank AF331500). Les structures et les séquences en acides aminés d'Env Pv14 et d'Env-SU sont respectivement représentées dans les figures 1 (a) et 1 (b). La protéine Env-SU de MSRV recombinante est exprimée dans *E.coli* et purifiée sur colonne FPLC. La qualité et la pureté de la protéine sont confirmées par spectrométrie de masse et Western Blot. La caséine kinase est utilisée comme contrôle négatif autologue. Cette protéine de contrôle a été produite et purifiée dans les mêmes conditions que Env-SU.

Les deux protéines sont testées pour la présence d'endotoxines par un test sur Lysat d'Amebocyte de Lymulus (LAL) réalisé par la société CleanCells (Bouffere, France). Toutes les fractions sont en dessous du seuil de détection de 5 UI/ml. L'entérotoxine B de staphyloccoque (SEB) obtenue chez Toxin Technology (Sarasota, FL, USA) était pure à 95%. Le liposaccharide (LPS) de *E. coli* souche 026:B6 est obtenu chez Sigma Aldrich.

Milieu de culture.

Le milieu de culture est le milieu RPMI 1640 (Gibco) supplémenté en :

L-glutamine 1% (Sigma-Aldrich),

pénicilline / streptomycine 1% (Sigma-Aldrich),
pyruvate de sodium 1% (Sigma-Aldrich),
acides aminés non essentiels 1% (Sigma-Aldrich), et
SVF (sérum de veau foetal) inactivé par la chaleur 10% (BioWest).

Pour les essais de prolifération des cellules T, un sérum AB humain (Sigma-Aldrich) a été utilisé au lieu du SVF.

Isolement des cellules et préparation.

Des cellules mononuclées humaines du sang périphérique (PBMCs) sont isolées à partir de donneurs sains par centrifugation par gradient de densité sur Ficoll Paque. Les monocytes des PBMCs sont purifiés par enlèvement des cellules T, des cellules B, des cellules dendritiques, des cellules NK et des basophiles en utilisant le kit d'isolement des monocytes, commercialisé par la société Miltenyi Biotec. En résumé, les PBMCs sont d'abord incubées avec un cocktail d'anticorps monoclonaux et d'anti-immunoglobuline humaine conjugués à un haptène et marqués magnétiquement (anti-CD3, anti-CD7, anti-CD19, anti-CD45RA, anti-CD56 et anti-IgE), puis avec des microbilles (MACs Micro-Beads) couplées à des anticorps monoclonaux anti-haptène. Les cellules marquées magnétiquement sont finalement enlevées en les retenant sur une colonne dans un champ magnétique. La pureté de la population des monocytes récupérée est toujours supérieure à 95%, comme déterminé par l'expression de CD14 par analyse par cytométrie de flux. Pour la génération de monocytes dérivés des cellules dendritiques (MDDCs), les monocytes purifiés sont cultivés pendant 5 jours sur des plaques de 6 puits contenant de l'IL-4 à 25 ng/ml et du GM-CSF à 50 ng/ml dans 2 ml du milieu de culture. Au jour J3 de la culture, la quantité complète de cytokines est ajoutée aux cellules. Comme montré par analyse de la morphologie et par cytométrie de flux, la préparation de cellules résultante contient plus de 90% de cellules dendritiques CD1a positives.

Stimulation cellulaire.

Les cellules (PBMCs, monocytes ou MDDCs) sont placées dans des plaques de 24 puits, à une concentration de 1 x $10^6$ cellules par puits, dans 1 ml de milieu de culture avant stimulation par Env-SU, LPS, SEB, ou le contrôle autologue. Elles sont ensuite incubées à 37°C dans une atmosphère humidifiée à 5% de $CO_2$. Quand indiqué, les cellules ont été pré-incubées soit avec 10 $\mu$g/ml de Polymyxine B (Sigma-Aldrich), 20 $\mu$g/ml et 5 $\mu$g/ml d'anticorps monoclonal anti-CD14, 20 $\mu$g/ml et 5 $\mu$g/ml d'anticorps anti-TLR-4 (HTA125, eBioscience) ou avec une IgG de contrôle d'isotype 2a (IgG2a) (eBM2a, eBioscience) avant la stimulation cellulaire. Dans certaines expériences, Env-SU, le contrôle autologue, LPS et SEB sont bouillis pendant 30 minutes avant le traitement cellulaire.

Pour déterminer la spécificité des résultats, 1 μg de Env-SU, LPS, SEB et du contrôle autologue est pré-incubé à 4°C pendant 1 heure avec 30 μg/ml d'anticorps monoclonaux dirigés soit contre Env-SU (13H5A5 ; IgG1 ; biomérieux) soit contre GAG (3H1H6 ; IgG1 ; biomérieux).

Ensuite, les cellules sont incubées 24 heures à 37°C, puis les surnageants de culture sont récoltés pour l'analyse de la sécrétion de TNF-α, IL-1β et IL-6 par ELISA.

Essais de prolifération des cellules T.

Les monocytes et les MDDCs stimulés sont utilisés comme stimulateurs des cellules T. Les cellules T allogéniques sont utilisées à 1 x 10^5 cellules par puits comme cellules « répondeuses » dans des microplaques de 96 puits à fond rond. Les cellules stimulatrices sont ajoutées aux cellules T dans des doses croissantes et les cultures sont effectuées en triple dans un volume final de 200 μl de milieu de culture. Après 5 jours d'incubation à 37°C, la prolifération des cellules T est évaluée par mesure de la radioactivité incorporée. Pour ce faire, dans les dernières 18 heures d'incubation, 1μCi de $^3$H Thymidine est ajouté à chaque puits. Les cellules sont ensuite récupérées sur des tapis de filtre de verre pour la mesure de la radioactivité incorporée.

Marquages par immunofluorescence et cytométrie de flux.

Les cellules sont récoltées, lavées dans du PBS et ensuite colorées pour des marqueurs de surface différents. Les anticorps monoclonaux suivants (Becton-Dickinson, San Jose, CA) ont été utilisés : anti-CD1a allophycocyanine (HI149-APC), anti-CD14 isothiocyanate de fluorescéine (MOP9-FITC), CD40 phycoérythrine (5C3-PE), CD80 phycoérythrine (L307.4-PE), CD86 phycoérythrine (IT2.2-PE) et HLA-DR chlorophylle de péridine (L243-PerCP).

La coloration par immunofluorescence directe des cellules est réalisée dans du PBS refroidi sur glace supplémenté par du SVF 2%, avec les différents anticorps aux concentrations recommandées par les fabricants. Après 30 minutes à 4°C, les cellules sont lavées et ensuite analysées en utilisant un FACS Calibur (nom commercial) et le logiciel CellQuest (nom commercial) (Becton Dickinson).

Essais de production des cytokines.

Les surnageants de cultures sont collectés et conservés à -20°C avant analyse de la sécrétion des cytokines. Les quantités de cytokines sont mesurées en utilisant les kits ELISA OptEIA (nom commercial) (Pharmigen) pour IL-1beta, IL-6, IL-10, IL-12p40, IL-12p40 and TNF-alpha, en respectant les instructions du fabricant.

**Résultats.**

Env-SU induit la production de cytokines pro-inflammatoires à partir de PBMCs humaines.

La capacité de la protéine recombinante Env-SU à stimuler la sécrétion de cytokines dans les cultures de PBMCs a été testée. Les PBMCs de donneurs sains ont été incubées pendant 24 heures avec des doses croissantes de la protéine recombinante Env-SU et la sécrétion des cytokines TNF-α, IL-1β et IL-6 a été évaluée par ELISA. Les quantités de cytokines sécrétées ont été comparées avec celles obtenues avec le contrôle autologue, SEB (un superantigène bactérien bien caractérisé) et LPS, bien connu pour avoir des propriétés pro-inflammatoires sur les PBMCs humaines. Toutes les protéines et les toxines ont été utilisées à une concentration de 1 μg/ml (concentration optimale pour l'induction de cytokines pro-inflammatoires déterminée par des expériences dose/réponse). Les résultats montrent que Env-SU induit la sécrétion des trois cytokines d'une manière dépendante de la dose, même à des doses aussi faibles que 10 ng/ml. Comme montré dans la figure 2, la cinétique de sécrétion de cytokines obtenue avec Env-SU est plus proche de celle du LPS que du SEB.En effet, La stimulation par Env-SU dans les conditions du test in vitro mis au point et décrit ici, se différencie tout à fait de la stimulation superantigénique représentée ici par l'antigène SEB : (i) pas de sécrétion précoce d'interféron gamma (qui signe l'activation des lymphocytes T, spécifiquement reconnus au niveau du « T-cell recptor » (TCR) par les superantigènes, (ii) sécrétion importante et précoce d'IL-6 (sécrétée par les monocytes-Macrophages et pas par les lymphocytes T) et de TNF-alpha, qui ne sont pas produits dans les conditions de notre test in vitro par un superantigène (comme montré ici avec l'exemple du SEB). De plus, les exemples montrant l'activité sur les monocytes purifiés et les cellules dendritique de la protéine Env MSRV/HERV-W, par sa région Env-SU, confirment que ces effets ne passent pas par le TCR, qui n'est pas présent sur ces cellules, et donc que l'effet pro-inflammatoire particulier décrit ici est bien différent de l'activation pro-inflammatoire causée par une fonction superantigène qui, par définition, passe par la liaison au TCR et les lymphocytes T. La voie d'activation pro-inflammatoire de la protéine Env MSRV/HERV-W revendiquée ici passe par le « Toll-like recepteur 4 » (TLR4), éventuellement avec le concours de son co-récepteur CD14, qui est activée en amont de l'activation des lymphocytes T, comme illustré dans la figure 8. Cette voie pro-inflammatoire (TLR4) mobilise la composante « innée » du sytème immunitaire qui est mobilisée bien en amont de l'immunité liée aux lymphocytes « T » (immunité adaptative).Cette voie amont peut, après activation des cellules dendritiques, influencer en aval l'état d'activation de l'immunité adaptative (profils Thl ou Th2), par le biais des cytokines sécrétées en réponse à l'activation d'un récepteur de l'immunité innée comme TLR4. Ce dernier effet montre, outre le fait que la voie de l'immunité innée est activée amont de l'immunité adaptative médiée par les lymphocytes T, que même l'effet résultant en aval sur les lymphocytes T ne passe pas par le récepteur TCR. Ceci illustre bien la différence entre l'effet de la protéine Env MSRV/HERV-W (Env-SU) observé à ce niveau (TLR4) et l'effet superantigène (illustré par le superantigène SEB dans cet exemple) qui passe par le récepteur du lymphocyte T (TCR). Or, la voie TLR4 exclut une activation primaire des lymphocytes T à ce stade et passe donc par d'autres cellules (monocytes-macrophages, cellules dendritiques, lymphocytes B). La voie d'activation observée ici est donc en amont de l'effet superantigène, ce que corrobore la cinétique qui correspond au référent « LPS », plutôt que « SEB » dans le test cellulaire utilisé ici.

En effet, Env-SU et LPS sont capables d'induire la sécrétion de grandes quantités de TNF-$\alpha$, d'IL-6 et d'IL-1$\beta$ dès 24 heures, alors que SEB induit seulement la sécrétion de TNF-$\alpha$ même après 72 heures d'incubation. Il est intéressant de noter que Env-SU et LPS atteignent leur pic de sécrétion de TNF-$\alpha$ tandis que SEB induit une sécrétion constante de TNF-$\alpha$. Par rapport à IL-1$\beta$, Env-SU et LPS induisent un profil de sécrétion similaire à celui du TNF-$\alpha$, caractérisé par un pic de sécrétion autour de 24 heures d'incubation suivi d'une diminution constante. SEB qui est connu pour activer une grande population de lymphocytes T portant la même spécificité TCR V$\beta$ n'induit aucun IL-1$\beta$. IL-6 est sécrété de manière constante par les PBMCs stimulées par Env-SU et LPS, mais non par SEB. IL-6 et IL-1$\beta$ sont deux cytokines préférentiellement relarguées par les monocytes/macrophages activés. Ces données montrent que d'une manière similaire à celle de LPS, Env-SU cible les cellules du système immunitaire inné, telles que les monocytes et les macrophages pour la libération de cytokines pro-inflammatoires, et que les lymphocytes T ne sont pas ciblés à ce niveau d'activation.

Pour éliminer la possibilité d'une contamination par endotoxines de la protéine recombinante Env-SU, les PBMCs humaines ont été soit traitées avec un inhibiteur des LPS, la Polymixine B (PB) avant stimulation, soit incubées avec des protéines et des toxines bouillies. En parallèle, a également été ajouté un contrôle autologue produit et purifié dans les mêmes conditions, avec les mêmes réactifs et matériel : caséine kinase humaine CK2.

Après 24 heures d'incubation, les surnageants de culture ont été récoltés et analysés pour la sécrétion de TNF-$\alpha$. Comme montré à la figure 4, le TNF-$\alpha$ induit par Env-SU et SEB est seulement partiellement inhibé par PB, alors que les effets de LPS sont totalement abolis. La protéine autologue de contrôle n'induit aucune sécrétion de cytokines. La libération du TNF-$\alpha$ est également inhibée de manière significative quand les protéines Env-SU sont bouillies pendant 30 minutes, alors que l'activité LPS n'est pas affectée. Ceci est conforme aux résultats négatifs obtenus lors de l'analyse de contrôle de qualité réalisée à la fois sur des échantillons purifiés de Env-SU et du contrôle autologue en utilisant le test LAL, approuvé par la Food and Drug Administration.

Ces résultats démontrent que les effets pro-inflammatoires observés précocement ne sont pas dûs à une contamination par endotoxines et que composant responsable de ces effets est bien une protéine.

Pour confirmer la spécificité des propriétés pro-inflammatoires de Env-SU, les effets d'anticorps monoclonaux ont été étudiés. Les PBMCs ont été incubées pendant 24 heures avec le contrôle autologue, Env-Su ou LPS pré-incubés à 4°C pendant 1 heure avec soit un anticorps monoclonal dirigé contre Env-SU, soit avec un anticorps monoclonal dirigé contre Gag. La protéine Gag utilisée pour développer cet anticorps monoclonal ne présente pas d'activité pro-inflammatoire et constitue un contrôle adéquat. Comme montré à la figure 4, l'anticorps monoclonal anti-Env-SU bloque spécifiquement la sécrétion de TNF-$\alpha$ médiée par Env-SU, mais pas celle de LPS. La sécrétion de cytokines n'est pas affectée par l'anticorps monoclonal anti-Gag. Ces résultats démontrent la spécificité de Env-SU sur l'induction de cytokines et l'activation cellulaire.

Env-SU a la capacité d'induire des cytokines pro-inflammatoires dans des cultures de PBMCs. Il a ensuite été vérifié que Env-SU était capable d'activer directement des monocytes purifiés. Les monocytes purifiés ont été stimulés avec le contrôle autologue, Env-SU ou LPS pendant 24 heures et différents marqueurs d'activation, tels que CD80 et CD86, ont été évalués par cytométrie de flux. Comparé au contrôle autologue, Env-SU induit une régulation en amont des deux marqueurs et les niveaux d'expression obtenus sont similaires à ceux de LPS (figure 5a). De grandes quantités de TNF-$\alpha$, d'IL-1$\beta$, d'IL-6 et d'IL-12p40 sont produites en réponse à Env-SU (figure 5b). Ces résultats montrent que Env-SU induit une activation rapide et directe des monocytes associée à une production de cytokines pro-inflammatoires.

Les cellules dendritiques sont des cellules présentatrices d'antigènes liant les immunités innée et adaptative avec la capacité unique de contrôler l'activation des cellules T naïves. La capacité de Env-SU à activer directement des cellules dendritiques dérivées de monocytes (MDDCs) a été étudiée. Les cellules dendritiques ont été générées *in vitro* à partir de monocytes hautement purifiés et stimulées pendant 24 heures avec le contrôle autologue, Env-SU ou LPS. Env-SU est capable d'augmenter drastiquement l'activation des marqueurs CD80, CD86, CD40 et HLA-DR (figure 6a). Les cytokines pro-inflammatoires IL-6, TNF-$\alpha$, IL-12p40 et IL12p70 sont sécrétées à des niveaux plus élevés. Il est montré que les MDDCs stimulées par Env-SU sont capables d'induire une prolifération allogénique des cellules T à un degré plus important que le contrôle autologue, même quand le nombre de cellules stimulatrices est faible (figure 6c). Donc, de manière similaire au LPS (contrôle positif), Env-SU est capable d'induire la maturation de cellules dendritiques sécrétant de l'IL-12 et est donc capable d'induire des réponses immunes spécifiques primaires.

Pour déterminer si Env-SU utilise la même voie d'activation que LPS, les niveaux de TNF-$\alpha$, sécrété par des PBMCs humaines après stimulation, avec ou sans pré-incubation avec des anticorps neutralisants anti-CD14 ou anti-TLR4 ont été mesurés. Les résultats présentés à la figure 7a montrent que le blocage de CD14 a pour résultat une inhibition significative dépendante de la dose d'Env-SU et de la sécrétion du TNF-$\alpha$ médiée par LPS (83% et 56% d'inhibition respective avec 20 $\mu$g d'anticorps anti-CD14). SEB, qui est connu pour activer les cellules T et les cellules présentatrices d'antigènes via le récepteur des cellules T et le HLA-DR, n'est pas inhibé. Le blocage de TLR4 a pour résultat une inhibition de 37% sur les effets de Env-SU et une inhibition de 43% sur les effets de LPS, avec 20 $\mu$g d'anticorps anti-TLR4 (figure 7b). Aucun effet d'inhibition n'est observé pour les anticorps de contrôle dans les deux expériences. Les récepteurs CD14 et TLR4 sont donc impliqués dans les propriétés pro-inflammatoires médiées par Env-SU.

En conclusion, la fraction soluble de la protéine d'enveloppe de MSRV/HERV-W stimule une réponse immunitaire innée via les récepteurs de reconnaissance CD14 et TLR4 et il est montré dans la présente invention que la cascade immunopathologique conduisant à des lésions inflammatoires peut être bloquée à un stade très précoce par administration d'une composition thérapeutique ou médicament comprenant au moins un anticorps choisi parmi les anticorps anti-Env-SU et/ou anti-TLR4.

Ainsi, dans la présente invention après avoir testé différents anticorps monoclonaux produits par bioMérieux contre les protéines d'enveloppe de MSRV/HERV-W, le test cellulaire mis au point et développé dans la présente invention a permis de d'identifier ceux qui ont la propriété d'inhiber l'effet pro-inflammatoire activant la voie TLR4 et de sélectionner parmi les anticorps inhibiteurs, ceux qui ont un potentiel inhibiteur le plus proche de 100%. Parmi ces anticorps, les anticorps 3B2H4 et 13H5A5 sont les anticorps préférés.

Il a ainsi été possible d'identifier les propriétés d'inhibition des voies précoces de l'inflammation qui sont impliquées dans des pathologies comme la SEP et la SCZ à un niveau très en amont d'une cascade pathogénique qui diverge plus en aval dans les processus de ces deux maladies, ainsi que cela est illustré dans les figures 8 et 9.

L'utilité de ces anticorps, répondant aux définitions données précédemment, pour la préparation d'une composition pharmaceutique ou d'un médicament ressort donc à l'évidence puisqu'ils permettent de bloquer très en amont une cascade pathogénique dans des pathologies, telles que la SEP ou la SCZ. Leur intérêt est aussi démontré par leur effet inhibiteur qui se situe avant l'interaction avec le récepteur TLR-4, puisque l'inhibition est équivalente à celle obtenue avec l'anticorps anti-TLR4 dans le même test cellulaire dédié à l'étude de son activation précoce. Cet effet en amont de l'activation des lymphocytes T permet ainsi de bloquer un agoniste pathologique qui, à ce stade, est commune à des pathologies autoimmunes comme la SEP et non-autoimmunes comme la SCZ (cf. : Figure 9). Ainsi les anticorps de l'invention permettent de bloquer en amont des cascades pathogéniques qui diffèrent en aval dans des pathologies telles que la SEP et la SCZ. La figure 8 montre la cible visée par les anticorps de l'invention dans la cascade pathogénique de la SEP qui anticipe toutes les cibles actuellement visées par les agents thérapeutiques existants. En effet, au stade d'intervention des anticorps de l'invention, il n'y a qu'un agoniste (Env MSRV/HERV-W) et un récepteur (TLR4), alors qu'après activation du récepteur, ce sont des centaines d'agonistes sous forme molécules bioactives (cytokines, enzymes, radicaux libres etc..) qui entrent en jeu dans le processus inflammatoire, puis, dans le cas de la SEP, des milliers d'agonistes moléculaires et cellulaires qui sont mis en jeu après la phase d'activation des clones de lymphocytes T autoimmuns. Dans le cas de la Schizophrénie (SCZ), ce ne sont pas les lymphocytes T qui sont activés dans une voie autoimmune, mais les médiateurs pro-inflammatoires produits après l'activation de la voie TLR4 dans une cellule de la substance grise cérébrale qui provoquent une excitotoxicité au niveau des neurones adjacents. Ces phénomènes d'excitotoxicité induite par les molécules pro-inflammatoires sont bien décrits et provoquent un relargage anormal de neuromédiateurs qui, dans le cortex frontal d'un individu, provoque des phénomènes hallucinatoires. Il est encore plus intéressant de réaliser que ces phénomènes excitotoxiques aboutissent souvent à une neurotoxicité qui se traduit par une mort neuronale. Or, cette mort neuronale est connue dans la SCZ et est objectivée par les élargissements ventriculaires typiquement visualisés par l'imagerie IRM chez les patients à un stade avancé de la pathologie. En effet, la perte progressive des neurones du cerveau de ces patients est compensées par une augmentation du volume des ventricules cérébraux qui devient décelable en IRM après une certaine durée d'évolution de la maladie. Les figures 8 et 9 illustrent donc bien le fait que les anticorps anti-Env identifiés et sélectionnés par le test cellulaire mis au point dans le cadre de la présente invention bloquent réellement le stimulus « primaire » le plus en amont de cette cascade, après l'activation d'une ou plusieurs copies pathogènes de la famille MSRV/HERV-W.

Afin de confirmer encore plus précisément l'association entre l'expression d'une protéine d'enveloppe (Env) de la famille rétrovirale MSRV/HERV-W et les pathologies SEP et SCZ, des études ont été réalisées en utilisant le test d'activation de l'immunité innée décrit dans la présente invention, pour rechercher un biais d'activation immunologique chez les patients SEP ou SCZ, par rapport à des témoins sains.

**Etudes ex vivo**

**Etude *ex vivo* chez des patients atteints de sclérose en plaques (SEP).**

La sécrétion d'IL-6 induite par Env-SU est augmentée dans les cellules mononuclées sanguines prélevées ex *vivo* chez les patients SEP et corrèle leur score clinique (EDSS).

Lors de cette étude, la réactivité à Env-SU de PBMCs de patients SEP et de donneurs sains a été comparée. Trente deux patients ont été inclus, vingt étant en phase aiguë et 12 en phase stable selon une analyse faite par IRM. Leur niveau de handicap a également été déterminé selon les critères de l'EDSS (Extended Disability Score). En parallèle, 19 donneurs sains ont été testés. Brièvement, $1\times10^6$ de PBMC ont été incubés avec Env-SU ou le contrôle Mock pendant 24 heures puis les surnageants de culture ont été analysés pour la sécrétion de cytokines telles que IFN-$\gamma$, TNF-$\alpha$, IL-1$\beta$, IL-6, et IL-10. Les résultats obtenus (Env-SU - Mock) ont tout d'abord été comparés entre les groupes. Aucune différence significative n'a été observée pour IFN-$\gamma$, TNF-$\alpha$, IL-1$\beta$ et IL-10 (Figure 10). Par contre, des différences importantes ont été obtenues avec l'IL-6 et l'IL12p40 qui sont augmentée chez les patients (Figure 11). De plus, il a été observé une corrélation positive entre le niveau de sécrétion d'IL-6 ou d'IL-12p40 obtenus et le score clinique des patients (Figure 12). Aucune autre corrélation n'a été obtenue avec d'autres cytokines ou données cliniques (age, sexe, traitement). Dans la figure 12, l'absence de corrélation de l'IL-6 induite avec la durée de la maladie, ainsi que l'absence de corrélation entre l'interféron gamma et l'EDSS sont donnée en exemple.

En ce qui concerne l'interféron gamma qui est ici sécrété exclusivement par les lymphocytes T, il est très intéressant de noter que, contrairement aux cytokines associées à l'immunité innée sécrétées ici par les monocytes/macrophages, il ne corrèle pas avec le score clinique (EDSS). Ceci montre bien que, comme cela a été mis en évidence *in vitro* avec les tests cellulaires, l'effet révélé ex *vivo* corrèle bien un effet qui n'est pas médié à ce stade par les lymphocytes T, et donc, ne s'apparente pas à un effet superantigène.

Ces résultats suggèrent que les patients SEP présentant les signes cliniques les plus avancés (EDSS élevé) sont "hyper-sensibles" aux facteurs rétroviraux tels que Env-SU, mais peut également suggérer un rôle pour Env-SU dans la pathogénèse de la SEP via les cytokines pro-inflammatoires et l'IL-6.

Ceci confirme les données obtenues avec la charge virale MSRV dans le LCR des patients SEP, dans l'étude de Sotgiu et coll. [10], qui montrait une augmentation progressive de la charge virale MSRV avec l'aggravation de la maladie. Selon les résultats de l'invention, la réponse induite par la protéine d'enveloppe MSRV augmente de manière corrélée avec l'aggravation de la maladie mesurée par l'EDSS. Ces deux études indépendantes réalisées ex *vivo* sur des patients SEP, avec des approches différentes (dosage des acides nucléiques MSRV par RT-PCR d'une part et dosage d'une réponse immunologique à la protéine d'enveloppe MSRV d'autre part) confirment l'association entre le processus de la maladie elle-même et le rétrovirus MSRV.

**Etude *ex vivo* chez des patients atteints de schizophrénie (SCZ).**

La sécrétion d'IL-12p40 induite par Env-SU est augmentée dans les cellules mononuclées sanguines prélevées ex *vivo* chez les patients SCZ et permet de surcroît d'identifier aux taux les plus élevés, une sous-population de patients résistants aux thérapeutiques antipsychotiques et/ou ayant des formes particulièrement évolutives de SCZ.

Lors de cette étude la réactivité à Env-SU de PBMCs de patients SCZ et de donneurs sains a été comparée. Vingt cinq patients ont été inclus. En parallèle, 15 donneurs sains ont été testés selon un protocole identique à celui de l'étude précédente avec les patients SEP.

Les surnageants de cultures ont été analysés pour la sécrétion de cytokines telles que TNF-$\alpha$, IL-12p40, IL-1$\beta$, IL-6, et IL-10. A ce stade de l'étude, une différence notable a été observée entre une partie des patients atteints de SCZ et l'ensemble des témoins pour différentes cytokines testées. Les résultats sont présentés dans les tableaux 1 et 2 qui suivent. Le tableau 1 représente les différents donneurs sains dont le code est indiqué dans les lignes de la première colonne avec, dans chaque ligne, les quantités en ng/ml dosées pour les différentes cytokines indiquées en haut de colonne avec les deux conditions indiquées dans la ligne suivante pour leurs colonnes respectives (stimulation Mock et ENV-SU). Le tableau 2 représente les différents patients SEP dont le code est indiqué dans les lignes de la première colonne avec , dans chaque ligne, les quantités en ng/ml dosées pour les différentes cytokines indiquées en haut de colonne avec les deux conditions indiquées dans la ligne suivante pour leurs colonnes respectives (stimulation Mock et ENV-SU). Dans les tableaux 1 et 2, les deux lignes du bas (mean et St Dev) indiquent respectivement pour chaque colonne, la moyenne et l'écart type des données mesurées.

Tableau 1 :

| | TNF-a | | IL-6 | | IL-10 | | IL-12p40 | | IL-1b | |
|---|---|---|---|---|---|---|---|---|---|---|
| | Mock | ENV1 | Mock | ENV1 | Mock | ENV1 | Mock | ENV1 | Mock | ENV1 |
| ND 151003 | 24 | 500 | 216 | 3493 | 12 | 59 | 0 | 245 | 38 | 1149 |
| ND 291003 | 14 | 1491 | 166 | 14230 | 14 | 337 | 0 | 398 | 32 | 1677 |
| ND 011003 | 114 | 969 | 3727 | 12141 | 38 | 478 | 1 | 99 | 214 | 1712 |
| ND 300402 | 18 | 58 | 172 | 601 | 20 | 21 | 9 | 31 | 69 | 193 |
| ND 020702 | 0 | 139 | 111 | 1028 | 3,5 | 44 | 5 | 16 | 99 | 392 |
| ND 180602 | 48 | 264 | 92 | 1816 | 16 | 32 | 20 | 57 | 110 | 424 |
| ND 070801 | 0 | 124 | 84 | 1028 | 6 | 31 | 20 | 42 | 25 | 235 |
| ND 180901 | 1 | 228 | 39 | 2328 | 7 | 60 | 0 | 113 | 21 | 651 |
| ND 060701 | 30 | 260 | 345 | 3263 | 17 | 155 | 6 | 38 | 33 | 277 |
| ND 110901 | 0 | 328 | 70 | 7352 | 14 | 214 | 11 | 169 | 9 | 587 |
| ND 280801 | 7 | 58 | 77 | 882 | 20 | 30 | 4 | 57 | 52 | 168 |
| ND 190603 | ND | ND | 21 | 123 | 0 | 6 | 1 | 12 | 16 | 58 |
| ND 180602 (40 | 15 | 541 | 30 | 7569 | 6 | 171 | 11 | 107 | 8 | 723 |
| ND 2 (1) | ND | ND | 2870 | 8863 | 125 | 187 | 124 | 291 | ND | ND |
| ND 140504 | ND | ND | 185 | 803 | ND | ND | 8 | 8 | ND | ND |
| Mean | 22,58 | 413,33 | 547,00 | 4368,00 | 21,32 | 130,36 | 14,67 | 112,20 | 55,85 | 634,31 |
| St Dev | 20,94 | 307,94 | 733,73 | 3775,33 | 17,19 | 108,55 | 16,00 | 87,33 | 41,33 | 421,61 |

Tableau 2

| code patients | TNF-a | | IL-6 | | IL-10 | | IL-12p40 | | IL-1b | |
|---|---|---|---|---|---|---|---|---|---|---|
| Schizo | Mock | ENV1 | Mock | ENV1 | Mock | ENV1 | Mock | ENV1 | Mock | ENV1 |
| 1 | 46 | 475 | 298 | 3491 | 82,9 | 25,6 | 9,5 | 302 | 8 | 835 |
| 2 | 40 | 1570 | 787 | 21956 | 73 | 389 | 29 | 559 | 84,6 | 5632 |
| 3 | 587 | 1639 | 9588 | 30299 | 124,6 | 800 | 171 | 436 | 988 | 6237 |
| 6 | 33 | 863 | 408 | 8494 | 88,9 | 139 | 35 | 152 | 46 | 1459 |
| 9 | 40 | 236 | 338 | 2819 | 34,5 | 22 | 63 | 24 | 111 | 932 |
| 10 | 10 | 310 | 527 | 7552 | 92,9 | 216 | 2 | 75 | 5 | 458 |
| 12 | 11 | 950 | 769 | 22510 | 14,9 | 769 | 87 | 158 | 70 | 1614 |
| 13 | 1031 | 2840 | 27022 | 38290 | 94 | 580 | 271 | 481 | 2396 | 10309 |
| P2 | NT | NT | 738 | 2857 | 34,01 | 68,75 | 3 | 12 | NT | NT |
| P3 | NT | NT | 3362 | 12521 | 193 | 269 | 32 | 93 | NT | NT |
| P4 | NT | NT | 3481 | 14305 | 189 | 273 | 72 | 84 | NT | NT |
| P5 | NT | NT | 903 | 4141 | 33 | 133 | 13 | 210 | NT | NT |
| P8 | NT | NT | 579 | 7925 | 14 | 109 | 7 | 28 | NT | NT |
| P16 | NT | NT | 787 | 1009 | 5 | 16 | 0 | 9 | NT | NT |
| P17 | NT | NT | 113 | 432 | 0 | 0 | 0 | 0 | NT | NT |
| P18 | NT | NT | 3166 | 10755 | 125 | 236 | 2 | 44 | NT | NT |

(suite)

| code patients | TNF-a | | IL-6 | | IL-10 | | IL-12p40 | | IL-1b | |
|---|---|---|---|---|---|---|---|---|---|---|
| Schizo | Mock | ENV1 | Mock | ENV1 | Mock | ENV1 | Mock | ENV1 | Mock | ENV1 |
| P20 | NT | NT | 57 | 90 | 0 | 0 | 10 | 0 | NT | NT |
| P22 | NT | NT | 2461 | 8793 | 40 | 134 | 26 | 107 | NT | NT |
| P23 | NT | NT | 880 | 837 | 0 | 0 | 0 | 9 | NT | NT |
| P24 | NT | NT | 896 | 6833 | 8 | 101 | 4 | 38 | NT | NT |
| P27 | NT | NT | 3039 | 8816 | 132 | 230 | 57 | 49 | NT | NT |
| P28 | NT | NT | 99 | 135 | 2 | 7 | 3 | 11 | NT | NT |
| P30 | NT | NT | 2332 | >15000 | 189 | 785 | 28 | 734 | NT | NT |
| P32 | NT | NT | >15000 | >15000 | 233 | 805 | 40 | 584 | NT | NT |
| P33 | NT | NT | >15000 | >15000 | 249 | 452 | 11 | 18 | NT | NT |
| Mean | 224,75 | 1110,38 | 2723,04 | 9766,36 | 82,07 | 262,37 | 39,02 | 168,68 | 463,58 | 3434,50 |
| St Dev | 292,13 | 679,47 | 2897,37 | 7479,22 | 64,68 | 220,85 | 39,03 | 170,02 | 614,21 | 2968,63 |

Par rapport à la population SEP précédemment étudiée, une différence est déjà observée spontanément en culture pour certains patients (illustrée dans les figures 13 et 14 pour l'IL-10 et l'IL-12p40). Ceci témoigne d'une donnée inattendue, à savoir que, bien que la SCZ ne soit pas une maladie inflammatoire systémique et encore moins autoimmune, certains patients SCZ présentent un taux d'activation immunologique spontanée dans leur PBMCs qui dépasse aussi bien celui des témoins sains que celui des SEP dans les mêmes conditions. Ceci apporte une donnée importante sur la notion d'activation systémique de l'immunité chez ces patients et donc, confirme la réalité de cette composante immunologique pro-inflammatoire dans cette maladie.

La réponse à la stimulation par Env-SU est globalement plus augmentée dans la série de patients SCZ et certains patients répondent avec des taux de sécrétions de cytokines nettement supérieurs à la moyenne des témoins sains et même parfois supérieurs au maximum observé dans la série des témoins (comme illustré dans les figures 13 et 14, pour l'IL-10 et l'IL-12p40). Ceci confirme aussi que la réponse à Env-SU est significativement augmentée chez certains patients atteints de SCZ et que Env-SU est donc capable de révéler un biais immunologique mettant en jeu les composantes de l'immunité innée activée par la voie TLR4, chez certains de ces patients dont le statut clinique coïncide.

Cependant, chez ces patients, l'accroissement après stimulation par Env-SU relativement au taux spontané de cytokines (taux stimulé - taux spontané / taux spontané) est globalement plus faible chez les patients SCZ que chez les témoins sains; ceci même quand le taux de sécrétion induit par ENV-1 dépasse celui de tous les témoins sains (comme illustré dans la figure 14b et c, pour l'IL-12p40). Ceci apporte un élément nouveau au regard de ce qui a été décrit dans une pathologie comme la SEP, en relation avec le rôle étiopathogénique d'un élément rétroviral de la famille MSRV/HERV-W. En effet, le rôle d'un élément rétroviral de cette même famille MSRV/HERV-W dans la SCZ a aussi été mis en évidence et confirmé par plusieurs équipes indépendantes avec des approches différentes [3, 14, 31, 54], mais la SCZ n'est pas une maladie avec une composante pathologique autoimmune comme la SEP. Ainsi les résultats obtenus avec la protéine Env-SU sur les PBMCS de patients SCZ montrent que, bien que les conséquences en aval de l'activation immunologique qui concernent les lymphocytes T (cellules responsables de l'autoimmunité) soient différentes de la SEP, une voie d'activation précoce de l'immunité innée passant par le récepteur TLR4 (qui n'est pas présent sur les lymphocytes T) peut néanmoins constituer une voie pathogénique initiale commune entre ces deux maladies et la famille rétrovirale MSRV/HERV-W.

Comme cela a été évoqué précédemment, le rôle d'une protéine d'enveloppe MSRV/HERV-W chez ces patients devient maintenant objectivé par la réactivité immunologique particulière de leurs cellules mononuclées sanguines (PBMCs) à la protéine Env-SU.

Les différences les plus intéressantes et les plus pertinentes au regards des données cliniques des patients atteints de SCZ, à ce stade de l'étude, ont été observées pour l'IL12p40 (Figure 14).

En effet, il a été vérifié que les patients présentant un taux induit d'IL-12p40 induit par Env-SU parmi les plus élevés (ici, supérieur à 400 pg/ml, donc au taux maximal des témoins sains testés dans cette série) comprennent tous les patients résistants aux thérapeutiques antipsychotiques de la série testée et des formes de schizophrénie particulièrement évolutives.

Ceci montre que, avec un profil différent de celui obtenu précédemment avec les patients SEP, il existe au moins une sous-population de patients atteints de formes évolutives et/ou résistantes aux traitements existants qui est identifiable et caractérisée par une sécrétion supérieure à la moyenne d'IL12p40 induite par Env-SU.

Le fait qu'au moins l'IL12p40 induite par l'activation Env-SU dans les PBMC de patients SCZ est maximale dans les formes évolutives et/ou résistantes aux traitements actuels, met en évidence, outre l'association entre des critères d'évolution et de gravité de la maladie, une cible thérapeutique nouvelle pour ces patients, à savoir la protéine Env MSRV/HERV-W associée à ce biais immunologique chez les patients.

Comme cela est par ailleurs démontré pour les modèles de SEP, des anticorps capables d'inhiber l'activation du système immunitaire avant l'engagement de la voie « amont « médiée par le récepteur TLR4, ont un intérêt thérapeutique et leur cible est très intéressante dans le contexte clinico-biologique nouvellement identifié.

Contrairement à la SEP, où ces effets ont pour conséquence ultérieure une réactivité autoimmune qui cible les composants antigéniques de la myéline, les médiateurs produits à ce stade précoce d'activation de l'immunité innée (voie TLR4) avec un taux spontané *ex vivo* plus élevé que dans la SEP ont un potentiel excitotoxique sur les neurones corticaux [17, 21, 23-25, 29, 50, 51, 55].

Ainsi, l'activation d'un provirus MSRV/HERV-W par différents co-facteurs peut activer l'expression de la protéine Env MSRV/HERV-W dans des cellules du cerveau [56] et, selon la nature du co-facteur et les circonstances, cibler des territoires différents du cerveau. Dans ces conditions une activation dans les régions du cortex frontal peut provoquer une excitotoxicité neuronale se traduisant par des hallucinations diverses selon les territoires atteints.

Dans le cas d'une activation dans la substance blanche (myélinisée) l'inflammation précoce produite par la protéine Env au niveau des macrophages et/ou des microgliocytes est susceptible de stimuler une dégradation de la myéline et donc, après contribution des lymphocytes T, d'induire une autoimmunité contre les auto-antigènes de la myéline.

Ces différentes notions mises en évidence sont illustrées dans la figure 9 . Ceci montre que, dans le contexte clinico-biologique ainsi identifié chez des patients atteints de schizophrénie, il est utile d'inhiber cette composante inflammatoire neurotoxique en relation évidente avec les symptômes de la maladie.

De plus, comme cela est aussi illustré dans la figure 8, le niveau auquel ces anticorps inhibent les effets biologique de la protéine Env est bien en amont de tous les médiateurs pathologiques qui sont produits en aval et qui sont les cibles habituelles des thérapeutiques anti-inflammatoires classiques (cytokines, radicaux-libres, composés rédox, enzymes, prostaglandines, protéines et lipides pro-inflammatoires, lymphocytes T activés, etc...). A ce stade, le seul agoniste est la protéine Env MSRV/HERV-W elle-même et son empêchement d'activer le récepteur TLR4 bloque la voir d'entrée initiale des différentes cascades immunobiologiques qui en résultent en aval, comme illustré dans les figures 8 et 9.

Il est à la portée de l'homme de l'art d'entreprendre les travaux de développement pré-clinique, comme :

- l'humanisation des anticorps monoclonaux, selon les procédés mis en ouvre pour des anticorps thérapeutiques connus comme le REMICAD anti-TNF-alpha. L'optimisation du passage intracérébral d'anticorps thérapeutiques est réalisée selon des techniques maintenant bien connues dont la plupart sont décrites dans de nombreuses publications scientifiques et médicales comme, par exemple, décrit par Merlo et coll. ou par Pranzatelli [57, 58].
- la vérification de l'activité inhibitrice de ces anticorps humanisés ou modifiés avec le test d'activation pro-inflammatoire par la protéine Env-SU sur les PBMCs, tel que décrit dans la présente invention.
- la vérification de l'effet thérapeutique des anticorps sur des modèles animaux mettant en évidence les effets comportementaux de l'expression anormale de la protéine Env MSRV/HERV-W dans le cerveau [59].

Ainsi, les éléments de la présente invention, à savoir :

- la mise en évidence du récepteur « TLR4 » de la protéine d'enveloppe MSRV/HERV-W comme voie d'entrée de l'activation pro-inflammatoire « amont » au niveau des cellules de l'immunité innée
- le test cellulaire qui permet de détecter et mesurer ces effets
- les anticorps monoclonaux anti-Env capables d'inhiber les effets de cette protéine
- les évidences biologiques de ces effets au niveau des cellules immunitaires sanguine prélevées ex vivo chez les patients atteints de schizophrénie
- les évidences biologiques reliant ces effets à la pathologie permettent à l'homme de l'art de réaliser, avec les connaissances, les techniques et les modèles animaux connus à ce jour, les étapes de développement pré-clinique et d'aborder les études cliniques chez l'homme dans les conditions appropriées. De plus, les tests biologiques décrits dans la présente invention permettent une investigation biologique ex vivo des paramètres ciblés par ces anticorps thérapeutiques, avant, pendant et après le traitement des patients, par une simple prise de sang.

Un tel guidage thérapeutique apporte un avantage très important à la définition des patients éligibles pour un traitement à un moment donné ou dans un sous-groupe donné et permet d'ajuster la thérapeutique en dose et fréquence en fonction des résultats biologiques.

**Modèles animaux**

Réalisation d'un modèle d'étude de la distribution pharmacocinétique et de la toxicologie des anticorps thérapeutiques et de contrôles dans un modèle animal.

**Anticorps .**

1. Nature des Anticorps :

Pour éviter une dégradation hépatique trop rapide et trop importante, les anticorps d'intérêts sont utilisés sous forme de fragments de type Fab' ou Fab2, obtenus à partir des anticorps monoclonaux selon les techniques connues de l'homme de l'art [60]. Les anticorps anti-Env MSRV/HERV-W, inhibiteurs de l'effet pro-inflammatoire médié par TLR4, sont les anticorps 13H5A5 et 3B2H4. L'anticorps témoin anti-Gag MSRV/HERV-W est l'anticorps 3H1H6.

2. Protocole de Marquage des anticorps :

Les fragments sont tout d'abord dilués. 100 $\mu$l à la concentration de 1 $\mu$g/$\mu$l sont ainsi préparés puis mis en contact avec de l'iodure de sodium (NaI$^{125}$ NEN, au 5mCi/50 $\mu$l) adsorbé sur des billes (Iodobeads n°28665 Pierce Rockford, Illinois USA), comme préconisé par le fabriquant.
Après 10 minutes d'incubation, la solution est prélevée et transférée dans un tube dépourvue de billes. Cette opération permet de stopper la réaction et ainsi d'éviter l'oxydation du site actif de l'anticorps ce qui conduirait à une perte de fonction.
L'échantillon est ensuite neutralisé par 10 $\mu$l d'une solution de pyrosulfite de sodium 4mg/ml (Fluka). Enfin, l'iodure de sodium est entrainé à l'aide de 10$\mu$l d'un entraîneur froid dosé à 250 nM/ml.

3. Purification

La purification est effectuée par la technique de séparation sur colonne échangeuse d'ions. Cette méthode utilise une colonne échangeuse d'anions, destinée à fixer l'iode libre. Elle est activée dans un premier temps, à l'aide de 2 ml d'une solution de NaCl 0,9% (Aguettant), le tampon de migration. La purification est effectuée en quatre passages, entraînés par 0,5 mL de NaCl 0,9%. La radioactivité contenue dans les quatre tubes est ensuite comptée.

4. Rendement

Les résultats sont reportés dans le tableau 3 qui représente le pourcentage de récupération de chaque anticorps après la purification.
Le rendement de marquage est correct après 10 minutes d'incubation. Il n'augmente pas avec le temps. Le pourcentage obtenu pour l'ensemble des fragments est compris entre 70 et 80%.
La purification par échanges d'ions permet une bonne récupération des anticorps marqués. Pour le fragment 3B2H4 Fab2 le rapport n'est que de 50%.

**Evaluation de la biodistribution :**

1. Souris

Les animaux concernés par l'expérience sont des souris BALB/c de 7 semaines fournies par les Laboratoires Charles River (Wilmington, North Carolina, USA,). Leur bon état de santé est garanti par le fournisseur.
Elles sont entretenues en attendant l'expérimentation dans un local tempéré et éclairé par cycles avec précaution.

2. Protocole

Les lots sont constitués de 3 souris blanches BALB/c âgées de 7 semaines pour chaque fragment testé.
Dans une première étape, elles sont anesthésiées à l'aide de pentobarbital ou d'un mélange volume à volume de kétamine 2%-xylamine10g/100ml administré à la dose de 1$\mu$l/g de poids.
Puis elles sont injectées par voie intraveineuse (IV) avec 0,15 mg d'Anticorps marqués à 700 $\mu$Ci/mg (les souris

sont inoculées avec les fragments d'un des trois anticorps testés (3B2H4, 13H5A5 et 3H1H6).

Une lecture est effectuée à 10, 45, 90 et 210 minutes après l'injection.

Au bout de ces 210 minutes, la souris est sacrifiée et sont prélevés : la rate, le foie, les reins, le cerveau, le coeur, les poumons et le sang. La queue est également gardée pour ajuster les valeurs obtenues.

3. Résultats

Aucune pathologie tissulaire évocatrice d'une toxicité aiguë liée à ces anticorps n'a été observée.

Les résultats de biodistribution des anticorps sont reportés dans le tableau 4 qui représente la répartition de la dose d'anticorps marqué dans les différents organes 210 minutes après l'injection IV.

Le tableau 4 met en évidence qu'aucun des fragments testés ne se fixe de façon anormale dans un tissu.

Ces résultats démontrent que les anticorps n'ont pas de toxicité aiguë, sont dosables dans les fluides et les tissus biologiques, et que leur répartition correspond à celle attendue par l'homme du métier.

Ainsi, toute optimisation de la biodistribution de ces anticorps et ou toute vérification de ces constantes après leur modification, peut être évaluée pour sa pertinence pharmacocinétique et toxicologique selon un même protocole ou son équivalent.

Tableau 3

| Pourcentage de récupération des fragments d'anticorps au cours du marquage par l'Iode 125 | | | | | |
|---|---|---|---|---|---|
| | Anticorps | | | | |
| | 3H1H6 | | 3B2H4 | | 13H5A1 |
| Pourcentages de récupération | Fab' | Fab2 | Fab' | Fab2 | Fab' |
| Après fixation | 72,69% | 82,00% | 74,36% | 75,95% | 74,22% |
| Après purification | 62,70% | 73% | 75,10% | 41,70% | 76,20% |

Tableau 4

| Pourcentage moyen de la dose d'anticorps retrouvée dans les différents organes 210 minutes après l'injection chez la souris BALB/c | | | | |
|---|---|---|---|---|
| | 3H1H6 Fab' | 3B2H4 Fab' | 3B2H4 Fab2 | 13H5H1 Fab' |
| Rate | 1,28 | 1,68 | 2,26 | 1,30 |
| Cerveau | 0,18 | 0,28 | 0,60 | 0,17 |
| Rein | 7,07 | 16,34 | 12,88 | 20,64 |
| Foie | 1,31 | 1,24 | 1,80 | 0,97 |
| Sang | 2,98 | 3,28 | 5,54 | 2,87 |
| Coeur | 0,00 | 1,30 | 1,87 | 0,95 |
| Poumons | 0,00 | 2,14 | 2,68 | 0,60 |

Parallèlement à l'étude directe sur les patients, des modèles animaux ont été réalisés qui permettent de confirmer que :

- La pathologie « pro-inflammatoire » correspondant aux voies d'activation « immunité innée seule » (modèle SCID-hu et protéine Env-SU) ou « immunité innée et effet superantigène sur les lymphocytes T (modèle SCID-hu et virion) produites par une protéine Env MSRV/HERV-W est bien observée *in vivo* et analysable par des critères objectifs. La voie « immunité innée/ voie d'activation TLR4 +/- CD14 » étant l'objet de cette invention et présentant un avantage de blocage en amont de la cascade pro-inflammatoire.
- La pathologie autoimmune dirigée contre des auto-antigènes de la myéline comme dans la SEP est bien obtenue avec la protéine Env MSRV/HERV-W (modèle EAE MOG Env-SU) et analysable.

- L'utilisation d'anticorps monoclonaux anti-Env sélectionnés pour leurs propriétés inhibitrices et de leurs fragments porteurs de la spécificité de reconnaissance immunologique est compatible avec une composition thérapeutique présentant une distribution organique mesurable et une absence de toxicité organique chez l'animal (Modèle BALB/c anticorps radiomarqués).

- L'utilisation thérapeutique d'anticorps anti-Env obtenus par une sélection préalable des monoclonaux anti-Env dans le test d'activation « cellulaire » de la voie TLR4/immunité innée. Cette utilisation est illustrée dans un modèle tel que l' « EAE » (Encéphalomyélite Allergique Expérimentale) en présence d'autoantigène « MOG » (Myelin Oligodendrocyte Glycoprotein) induite par Env et permet d'inhiber, au delà de l'inhibition des la phase d'activation pro-inflammatoire bien décrite ici, les conséquences pathologiques beaucoup plus en aval (cf. : inhibition par les anticorps anti-Env dans le modèle EAE-MOG).

De plus, après une sélection des anticorps anti-Env inhibiteurs de l'effet pro-inflammatoire par le test cellulaire *in vitro* décrit dans la présente invention, les modèles animaux permettent de sélectionner parmi cette première sélection, les anticorps thérapeutiques qui n'ont pas d'effet secondaires néfastes à leur utilisation thérapeutique dans le contexte pathologique. En effet, comme cela a été montré pour un anticorps potentialisant l'atteinte neurologique dans l'exemple « EAE-MOG » (cf. : exemple EAE-MOG et anticorps 3H1H6), certains anticorps peuvent se révéler inadéquats pour la thérapeutique quelque soit leur spécificité et, même s'ils inhibent une cible pathologiques, ces effets secondaires doivent les faire éliminer ou modifier avant une utilisation thérapeutique dans un contexte particulier. Les outils mis au point lors de la présente invention permettent donc d'identifier ces effets néfastes éventuels dans le contexte pathologique d'utilisation et contribuent donc de manière originale et adaptée, à la sélection et à la validation des anticorps thérapeutiques adéquats.

**Modèles inflammatoires sur cellules et animaux :**

**Matériel et méthodes**

Protéines et toxines

Le lipopolysaccharide (LPS) de la souche *E. coli* 026 :B6 a été obtenu chez Sigma (St Louis, Mi). La protéine recombinante Env-SU représente une fraction de la protéine d'enveloppe entière de MSRV, ENV pV14, d'environ 33kDa et 287 acides aminés. Env-SU a été produite chez *E.coli,* purifiée par chromatographie et analysée par western blot (Protein Expert, Grenoble). Un test LAL (lymulus amebocyte lysate, Clean cell, Bouffere, France) a été effectué afin de détecter l'éventuelle présence d'endotoxines. Les résultats furent négatifs, en dessous du seuil de détection de 5UI/ml. Le tampon utilisé pour la conservation de la protéine sera utilisé dans les expériences comme témoin négatif. Il est constitué de Tris pH8 50mM, NaCl 0,3M, de $\beta$-mercaptoéthanol 1mM, sucrose 2%, glycérol 2% et d'urée 5,3mM.

Culture cellulaire

Préparation de PBMCs

Les PBMCS ont été préparés à partir de sang frais total citraté de donneurs sains (poches de sang total citraté, Centre de Transfusion Sanguine de Valence, France) par gradient de densité sur Ficoll (Amersham Biosciences, Freiburg, Allemagne). La dilution de 25 ml de sang avec 10ml de PBS-2% SVF (sérum de veau foetal), délicatement déposé sur 15 ml de Ficoll, est centrifugé à 2400 tpm pendant 20 min. à température ambiante (TA). Les anneaux cellulaires sont récupérés et les PBMCs sont lavés trois fois avec 50ml de PBS-2%SVF (Figure 11). Après comptage au bleu trypan, les cellules sont congelées à - 80°C dans un mélange 90% SVFd-10%DMSO et utilisées directement en culture pour des tests cellulaires ou pour injection chez la souris.

Préparation des suspensions de splénocytes murins

Après sacrifice des souris par dislocation cervicale, les rates sont prélevées et broyées sur un filtre en métal dans du RPMI. Les suspensions cellulaires sont centrifugées à 1200 tpm pendant 10 min. à 4°C. Le culot cellulaire est ensuite repris dans environ 4ml de sérum physiologique ou de SVFd (pour, respectivement injection aux souris C57Bl6 ou congélation). Un comptage au bleu trypan est effectué puis la concentration des cellules est ajustée. Les cellules sont ensuite congelées ou utilisées pour les modèles animaux. Pour l'injection aux souris, de la gentamicine est ajoutée à une concentration de 0,2 mg/ml. Enfin, 500 $\mu$l, soit $50.10^6$ cellules sont injectées par voie IP (intrapéritonéale) à chacune des souris C57Bl6.

Congélation et décongélation de cellules

Les suspensions cellulaires sont lavées une fois dans 50 ml de PBS (bioMérieux, France) à 4°C et centrifugées à 1400 tpm pendant 7min à 4°C. Les cellules sont ensuite reprises dans quelques ml de SVF et comptées. La concentration cellulaire est ensuite ajustée à 20.10$^6$ cellules en général. 500 $\mu$l de cette solution sont déposés en cryotube puis 500 $\mu$l de solution de congélation (80%SVFd-20%DMSO (Sigma)) sont ajoutés. Ainsi, les cellules sont conservées dans 1ml de solution 90%SVF-10%DMSO. Les cryotubes sont placés dans une boîte à congélation contenant de l'isopropanol pour une descente lente en température et placées à -80°C.

Les suspensions cellulaires sont décongelées au bain marie à 37°C. Les tubes sont lavés à l'alcool avant ouverture. Les cellules sont rapidement transférées dans 50 ml de RPMIc-10%SVFd, centrifugées à 1400 tpm pendant 7 min. à 4°C puis deux autres lavages sont effectués avec du RPMIc-10%SVF.

Entretien des cultures

Les cultures de cellules (PBMCs) sont incubées en atmosphère humide sous 5% de $CO_2$ à 37°C. Le milieu de culture utilisé est constitué de RPMI 1640 (Gibco, Rockeville, MD) supplémenté avec 1% de L-glutamine, 1% de penicilline-streptomicine, 1% de pyruvate de sodium, 1% d'acides aminés non essentiels (Sigma) ainsi que de 10% de sérum de veau fétal (Biowest, Nuaille, France) décomplémenté (SVFd) par chauffage à 56°C pendant 30 min.

Stimulations cellulaires

Les suspensions cellulaires (PBMCs ou splénocytes) sont décongelées et un comptage au bleu Trypan est effectué : la concentration cellulaire est ajustée à 1.10$^6$ cellules/ml. Les cultures s'effectuent dans des plaques 48 puits (500 $\mu$l de suspension cellulaire par puit) ou dans des plaques 24 puits (1 ml de suspension cellulaire par puit). Après dépôt des cellules en plaque, les différentes substances à tester sont ajoutées et les cellules sont incubées pour des périodes de temps variées. La récolte des surnageants s'effectue par centrifugation des suspensions cellulaires à 6000 tpm pendant 10 min. à TA. Ils sont ensuite congelés en tube ependorf à -20°C. Sauf indiqué, les concentrations de Env-SU et de LPS utilisées pour les tests cellulaires ont été de 1 $\mu$g/ml. Pour quelques expériences, Env-SU et le LPS ont été bouillis pendant 30 min. La polymixine B (PB) a été utilisée à 25 g/ml, et préincubée 45 min. à 37°C avec les cellules avant addition du tampon, LPS ou Env-SU. Pour les expériences nécessitant l'utilisation d'anticorps, des préincubations à 4°C ou 37°C des cellules avec les anticorps ou de Env-SU, du LPS ou du tampon avec les anticorps ont été nécessaires, pendant différentes périodes de temps. Les anticorps monoclonaux IgG anti-Env-SU (13H5A5 et 3B3H4) et anti-Gag (3H1H6) (bioMérieux) ont été obtenus par culture d'hybridomes après immunisation des souris avec respectivement les protéines Env-SU ou Gag recombinantes. La spécificité des anticorps anti-Env-SU a été vérifiée par ELISA. Sauf indiqué, les concentrations de 13H5A5, 3B2H4 et 3H1H6 utilisées pour les tests cellulaires ont été de 30$\mu$g/ml.

**Modèles animaux**

Entretien de souris

Les souris C57Bl6, BalbC ou SCID (Charles River, L'Arbres le, France) sont achetée à l'âge de 5 ou 6 semaines et sont gardées une semaine au repos après réception. Elles sont abritées dans des cages stériles filtrantes à une température de 24°C. Toutes les manipulations sont effectuées sous hôte à flux laminaire.

Humanisation des souris SCID et préparation des souris C57Bl6

Après une semaine d'adaptation, les souris SCID reçoivent une injection intra-péritonéale (IP) de 50.10$^6$ PBMCs frais humains dans 2ml de RPMI sans rouge de phénol (Eurobio, Les Ulis, France) additionnés de gentamicine à une concentration de 0,25 mg/ml, et sont de nouveau laissées une semaine au repos. Afin de garantir une bonne humanisation, 50 $\mu$l d'anticorps anti-NK (25 $\mu$l d'anticorps pur dilués dans 25microl d'eau physiologique) sont injectés par voie RO deux jours avant l'injection des PBMCs. Une semaine après l'humanisation, du sang est prélevé par voie RO sur chaque souris et le sérum conservé à -80°C afin de pouvoir tester le niveau d'humanisation des souris.

Les souris C57Bl6 reçoivent après une semaine d'adaptation, une injection intrapéritonéale (IP) de 50 10$^6$ splénocytes frais murins dans 2 ml de RPMI sans rouge de phénol additionnés de gentamicine à une concentration de 0,25mg/ml et sont de nouveau laissées une semaine au repos. Lors des injections IP, chez les SCID comme chez les C57Bl6, le liquide se résorbe rapidement mais quelques pertes sont observées.

Dosage des IgG humaines chez les souris SCID-humanisées

Le dosage des IgG humaines dans le sérum des souris SCID est effectué par la méthode d'immuno-diffusion radiale en suivant les instructions du fabricant (The binding site, Birmingham, UK). La mesure du diamètre du précipité 96h après le dépôt du sérum sur le gel permet, grâce à une courbe de calibration, de relier son carré à la concentration en IgG de l'échantillon testé.

Injections des différentes substances et prélèvements sur les souris

A J0, les protéines (Env-SU), toxines (LPS) ou tampon sont attribués aux souris par injection IP après dilution des substances dans 1ml d'eau physiologique (Fresenius Kabi, Bad Homburg, Allemagne) à la concentration voulue. Pour les injections d'anticorps anti-Env-SU ou anti-GAG, ceux-ci sont préalablement incubés 3h à 4°C avec Env-SU, le LPS ou le tampon. Les souris témoins reçoivent 1 ml d'eau physiologique. Les prélèvement ont lieu quelques heures (1h, 2h) ou jours (24, 48, 72h) plus tard. Après anesthésie des souris à l'éther, le maximum de sang (1 ml environ) est prélevé par voie rétro-orbitaire (RO) avec une pipette pasteur. 2ml d'eau physiologique sont ensuite injectés dans la cavité intra-péritonéale (IP), et après massage de l'abdomen, le maximum de liquide est retiré (1 à 1,5 ml). Enfin, les souris sont sacrifiées par dislocation cervicale et la rate est prélevée. Les différents protocoles utilisés sont présentés figures 12, 13, et 14. Toutes les souris sont observées cliniquement jusqu'à la fin de l'expérimentation. Sont notés particulièrement les signes d'inflammations et les signes d'atteintes du système nerveux.

Traitements des prélèvements sur les souris

La rate prélevée est divisée en deux fragments. Une partie de celle-ci est mise en suspension par broyage sur grille dans du RPMI. Après centrifugation à 1200 tpm pendant 10 min. à 4°C, les cellules sont reprises dans 50 ml de PBS à 4°C puis centrifugées et congelées en cryotube dans 1 ml de solution de congélation (DMSO 10%-SVF 90%). Une autre partie de la rate est congelée telle quelle dans un ependorf à -80°C. Le liquide retiré en IP est centrifugé à 6000 tpm pendant 10 min. à TA afin de retirer le culot cellulaire et congelé à -80°C en tube ependorf. Après lavage en PBS, les cellules retirées de la cavité péritonéale sont à leur tour congelées. Le sang est centrifugé à 6000 tpm pendant 10 min. à TA afin de récupérer le sérum. Celui-ci ainsi que le culot cellulaire sont congelés séparément en tube ependorf à -80°C.

Traitement des résultats

Marquage cellulaire et cytométrie en flux

Les suspensions cellulaires sont décongelées et les cellules sont reprises dans 50ml de PBS-2%SVFd-1mMEDTA et centrifugées à 1400 tpm pendant 7 min. à 4°C. Un comptage au bleu Trypan est effectué et les cellules sont déposées en plaque 96 puits (environ $1.10^6$ par puits). Après centrifugation de la plaque à 4000 tpm pendant 1 min. à 4°C, les surnageants sont éliminés et 50 $\mu$l du cocktail d'anticorps marqueurs de surface (dilution en PBS-2%SVFd-1mMEDTA) sont ajoutés dans chaque puit. Les cellules sont resuspendues et incubées 30 min. à 4°C. Elles sont ensuite lavées par ajout de 100 $\mu$l de PBS-2%SVF-1mMEDTA par puits et centrifugées à 4000 tpm pendant 1 min. à 4°C. Les surnageants sont éliminés et 200 $\mu$l de PBS-2%SVF-1mMEDTA sont ajoutés par puits. Les cellules sont resuspendues et transférées dans des tubes pour l'analyse par FACS (Figure 15). Pour les anticorps nécessitant un second marquage streptavidine-APC, le même cycle est effectué une nouvelle fois. Les anticorps (Pharmingen, San Diego, CA) et dilutions utilisés pour le marquage des cellules murines sont : CD3 - FITC (1/500), CD4 - PE (1/1000), CD8 - cy-chrome (1/600), CD25 - APC (1/1000), CD69 - APC (1/500, biotinylé au départ). Pour le marquage des cellules humaines, 2$\mu$l de chaque anticorps sont utilisés : CD3-cy-chrome, CD4-APC, CD8-PE, CD25-PE, CD69-FITC.

Dosages de cytokines

Les surnageants de cultures, sérums et liquides issus des lavages intra-péritonéaux ont été conservés à -20°C avant le dosage des cytokines par ELISA. Les dosages de cytokines humaines ou murines (TNF-$\alpha$ et IL-6) par la méthode ELISA ont été effectués en suivant les instructions du fabriquant (Pharmingen).

Sélection des anticorps anti-Enveloppe inhibiteurs de l'effet pro-inflammatoire induit par la protéine d'enveloppe MSRV au niveau des cellules de l'immunité innée (via la voie d'activation TLR4)

Différents anticorps anti-enveloppe MSRV/HERV-W produits dans le laboratoire des anticorps monoclonaux de la société bioMérieux ont été testés dans des cultures de cellules mononuclées sanguines (PBMC) de donneurs sains avec un dosage de cytokines (IL-6 et/ou TNF-alpha), en absence ou en présence de protéine Env-SU, afin de déterminer leur effet sur l'activation des monocytes/macrophages présents dans la culture, par une voie que nous avons vérifié par ailleurs être celle du récepteur « TLR4 ». Ceci, selon les protocoles décrits dans la présente invention et notamment avec les anticorps produits par les hybridomes 3H10F10, 13H5A5, 6A2B2, 2A12A5, 3C1D5 et 3B2H4.

Certains anticorps anti-Env n'avaient pas d'activité inhibitrice détectable dans ces tests (6A2B2 et 2A12A5), ou une activité inhibitrice modérée (3C1D5) ou encore une activité inégale selon les expériences faites avec des PBMC de donneurs différents (3H10F10). Des exemples de ces tests sont montrés dans la figure 15 (15a, b, c et d) qui illustrent les effets de ces anticorps vis-à-vis de l'activation pro-inflammatoire produite par la protéine Env-SU dans différentes expériences.

De plus, un anticorps comme celui produit par l'hybridome 2A12A5, en l'absence d'activité inhibitrice sur la protéine Env-SU, a paradoxalement produit une stimulation immunologique non spécifique dans le test, même en l'absence de protéine Env-SU.

Des anticorps contrôles testés dans ces mêmes tests, n'ont pas montré d'activité inhibitrice, ni stimulatrice particulière, notamment l'anticorps anti-GAG 3H1H6.

Les anticorps monoclonaux anti-Env MSRV/HERV-W produits par les hybridomes 13H5A5 et 3B2H4 se sont avérés constamment inhibiteurs de l'effet de la protéine Env-SU sur les PBMC de différents donneurs humains sains, sans aucun effet paradoxal pro-inflammatoire ni variation significative entre des tests réalisés avec les PBMC de différents donneurs sains, décelable dans les conditions réalisées.

Un exemple de l'activité inhibitrice des anticorps 3B2H4 et 13H5A5 et de l'absence d'effet de l'anticorps anti-GAG MSRV 3H1H6 est présenté dans la figure 15a. Les conditions de spécificité de l'inhibition sont validées par rapport à un autre ligand stimulant cette voie d'activation (LPS) sur lequel ces anticorps n'ont pas d'effet. La figure 15b montre que les conditions de spécificité d'activation par la protéine Env-SU sont validées par l'absence d'effet d'une protéine témoin (mock) produite et purifiée dans des conditions identiques ainsi que par les tests confirmant l'absence de contamination de l'échantillon Env-SU utilisé par du LPS bactérien (Inhibition par le chauffage à 100°C qui dénature les protéines et pas le LPS, et absence d'inhibition par la polymixine B qui inhibe l'effet du LPS).

Ainsi, après avoir testé différents les différents anticorps monoclonaux obtenus par bioMérieux contre les protéines d'enveloppe MSRV/HERV-W, le test cellulaire mis au point et développé dans la présente invention a permis d'identifier ceux qui sont capables d'inhiber l'effet pro-inflammatoire activant la voie TLR4 et de sélectionner parmi les anticorps inhibiteurs, ceux qui ont un potentiel inhibiteur le plus proche de 100%. Parmi ces anticorps, les anticorps 3B2H4 et 13H5A5 sont préférés. L'utilité de ces anticorps ou d'autres anticorps qui peuvent produits par des techniques classiques à la portée de l'homme du métier, telles que décrites précédemment, est donc confirmée.

Effet de la protéine Env MSRV sur le système immunitaire humain, dans un modèle animal greffé avec un système lymphoïde humain fonctionnel.

- Préparation des souris :

L'expérience suivante concerne 17 souris femelles C57Bl6 de 6 semaines. La première étape consiste à injecter en IP, 50 millions de PBLs humains. Les souris sont préalablement irradiées et reçoivent une injection d'anticorps anti NK (Firouzi et al., Journal of Neurovirology 2003). Les souris sont laissées au repos pendant une semaine, temps nécessaire à la stabilisation du système immunitaire.

- Préparation des PBLs humains.

Les suspensions cellulaires sont regroupées dans un tube de 50 ml, centrifugées pendant 10 min. à 4°C et à 1200 tpm. Le culot cellulaire est ensuite repris dans environ 4 ml de sérum physiologique. Un comptage au bleu trypan est effectué puis la concentration des cellules est ajustée. De la gentamicine est ajoutée à une concentration de 0,2mg/ml. Enfin, 500$\mu$l, soit 50.10$^6$ cellules sont injectées par voie IP à chacune des 17 souris C57Bl6. Constitution des lots d'inoculation :

Les souris sont réparties par groupe de 3 ou 4. Chaque lot ainsi formé est nommé de la façon suivante : 3 * « T » pour le groupe témoin négatif qui recevra du tampon ENV1.

3 * « LPS » pour le groupe qui recevra une injection de LPS, témoins positifs d'une réaction inflammatoire.

3 * « Env » pour le lot à qui sera injecté une solution de la protéine d'enveloppe du virus MSRV.

4 * « 2GR412 » pour le lot qui sera infecté par le virus MSRV inactivé par la chaleur 30 min. à 56°C (pour tester l'effet de la protéine d'enveloppe du virion, en l'absence de réplication virale).

4 * « GRE» pour le lot qui sera infecté par le virus GRE inactivé par la chaleur et fortement dilué dans témoin négatif (pour évaluer un effet éventuel de contamination d'un échantillon biologique par ce virion, dans le cadre de pools sanguins pour la transfusion de produits dérivés contenant un donneur virémique).

Les souris de chaque lot sont infectées par un volume de solution adéquate en une seule administration IP (les quantités de LPS et de Env correspondent à la concentration utilisée pour les tests sur PBMCs). Les solutions seront titrées de la façon suivante :

« LPS » : 50 μg/souris ;« Env » : 50 μg/souris (injection de 500 μl) ;« 2GR412 » et « GRE » : 100 μl du culot d'ultracentrifugation/souris.

Toutes les dilutions nécessaires sont réalisées dans du liquide physiologique stérile.

Observations et prélèvements :

J+1h/J+2h/J+24h/J3: Sacrifices et prélèvements

Dans chaque groupe, une souris est sacrifiée. Injection en IP de 2ml d'eau physiologique, tapoter le ventre et retirer le maximum de liquide (1-1,5ml maximum). Centrifuger la suspension (6000 tpm/10min/TA) afin de retirer l'éventuel culot cellulaire et congeler à -80°C en tube ependorf.

Le maximum de sang est prélevé par voie rétro-orbitaire avec une pipette pasteur sur tube héparine. Centrifugation du sang, à 6000 tpm/10 min./TA. Récupérer et congeler le plasma et le culot cellulaire séparément en tube ependorf à -80°C.

La rate prélevée est divisée en deux fragments. Une partie est mise en suspension (afin de réaliser son phénotypage humain et murin par FACS) : broyage sur grille dans environ 10ml de RPMI, centrifugation à 1200 tpm/4°C/10 min, lavage avec environ 15ml de PBS/4°C puis centrifugation et congélation en cryotube dans 1ml de solution de congélation (DMSO 10%-SVF 90%) et une autre partie est congelée telle quelle dans un ependorf à -80°C (pour une éventuelle PCR).

Cette opération est réalisée au bout de 24h et 48h, sauf signes cliniques apparus plus tôt (surveiller les souris et les récupérer juste avant la mort). Pour les deux souris restantes des lots « 2GR412 » et « GRE », les souris sont sacrifiées au bout de 15-20 jours ou récupérées dès leur mort si elle apparaît avant.

Cette répartition des prélèvements permet de couvrir les réactions immunitaires immédiates (2h), précoces (24h) et retardées (10-15jours).

Sur les prélèvements de liquides biologiques sont réalisés des dosages de cytokines humaines et murines (Il-6 et TNFα) ainsi que le dosage de la protéine « Env » ou/et le titrage du virus par ELISA et par bioessai sur des cultures cellulaires.

Ces analyses permettent d'évaluer la réaction immunitaire : inflammation (cytokines) et répartition cellulaire (FACS) et de rechercher une réplication virale (ELISA, bioessai).

Observations cliniques

Toutes les souris sont observées cliniquement jusqu'à la fin de l'expérimentation. Sont notés particulièrement les signes d'inflammations et les signes d'atteintes neurologiques.

**Résultats :**

Cette partie a pour but d'étudier *in vivo* sur un modèle SCID-humanisée les propriétés de Env-SU grâce aux paramètres déterminés par une étude de faisabilité préalablement réalisé.

Effets pro-inflammatoires de Env-SU sur des cultures de PBMCs

Dans un premier temps nous avons étudié la cinétique de production de TNF-alpha induite par Env-SU et le LPS dans une culture de PBMCs humains. Les protéines et toxines ont été utilisées à une concentration de 1 μg/ml (Figure 16). Nous pouvons observer que la production de cytokine atteint un pic à 2h post-injection puis diminue progressivement pour devenir nulle après 48h. La détection de TNF-alpha post-injection par Env-SU est très significative. En effet, les cellules incubées avec du tampon ne donnent lieu qu'à une très faible production de TNF-alpha, atteignant à peine 30 pg/ml à 2h. La stimulation par Env-SU donne lieu à une production de 550, 350 puis 160 pg/ml à respectivement 2h, 24h et 48h post-stimulation. L'action du LPS est légèrement supérieure, donnant lieu à une production de 650, 180 puis 50 pg/ml à 2h, 24h et 48h. Cette étude a permis de confirmer les propriétés pro-inflammatoires de la protéine Env-SU sur les PBMCs, comme déjà montré. Elle permet en plus de voir que la production de TNF-alpha est maximale à 2h post-injection. Ces données permettent de définir la cinétique de prélèvements à adopter pour l'étude sur modèle SCID, à savoir 2h, 24h et 48h.

Effets pro-inflammatoires de Env-SU chez la souris SCID-humanisée (SCID-h)

Après avoir constaté les effets pro-inflammatoires provoqués par la protéine Env-SU sur différentes cultures cellulaires, humaines et murines, nous avons évalué in vivo la pathogénicité de ces mêmes substances sur les souris SCID-hu. Un groupe de 16 souris a été greffé en IP avec $50.10^6$ PBMCs humains après avoir reçu 50 microlitres d'anti-NK par voie rétro-orbitaire. Une semaine plus tard, du sang de chaque souris fut prélevé afin de doser les IgG humaines dans leur sérum, dans le but de valider l'humanisation des souris. Nous avons pu déterminer par dosage en immuno-diffusion radiale que la concentration en IgG humaines dans le sérum de toutes les souris SCID-hu était très supérieure à 4,5 mg/l. La demi-vie des IgG chez une souris SCID-hu est de 12 jours. Dans notre cas, nous pouvons donc affirmer que l'humanisation des souris a été réussie.

Les souris sont ensuite réparties en cinq lots, chaque lot comprenant trois souris injectées avec, respectivement 0,2 ml de tampon, 50 $\mu$g d'Env-SU ou 50 $\mu$g de LPS, dilués dans 2 ml d'eau physiologique (Une des souris de chaque lot fut sacrifiée à 2h, 24h et 48h après les injections. Toutes les souris sont restées vivantes et aucun signe extérieur d'atteinte du système nerveux n'a été visible jusqu'à leur sacrifice.

Le dosage des cytokines TNF-alpha et IL-6 humaines et murines a été réalisé par ELISA. Les résultats de la Figure 17 montrent que la production de cytokines, humaines ou murines, suit la même tendance : elle est brusquement détectée à 2h post-injection puis elle devient nulle dans les jours qui suivent. Globalement, cette cinétique est identique à celle observée *in vitro* sur des PBMCs. Les souris injectée avec du tampon de montrent pas de production significative de cytokines. Le dosage du TNF-alpha murin ne révèle qu'un seul pic important : la souris ayant reçu du LPS montre un taux de TNF-$\alpha$ supérieur à 1000 pg/ml dans son sérum. Concernant le dosage de l'IL-6 murine, un taux d'environ 20000 pg/ml est atteint dans le liquide IP des souris injectées avec de l'Env ou du LPS. Les concentrations retrouvées dans le sérum de ces souris atteignent respectivement 6200 pg/ml et plus de 20000 pg/ml.

Le dosage des cytokines humaines révèle une détection de cytokines dans le liquide IP plus élevée que dans le sérum. En effet, les souris ayant été greffées seulement une dizaine de jours précédant l'administration des protéines et toxines, les PBMCs n'ont eu que peu de temps pour migrer et coloniser la rate ainsi que les organes lymphoïdes secondaires (le taux de cellules migrant reste relativement faible). De plus, Env-SU cible principalement les monocytes qui se différencient rapidement en macrophages dans les tissus. Ces cellules sont très adhérentes et vont préférentiellement venir se « coller » au péritoine plutôt que de coloniser les organes lymphoïdes secondaires. Il paraît donc logique de retrouver une production plus importante de cytokines dans le lieu même de greffe des cellules, dans la cavité intrapéritonéale (IP).

Le dosage du TNF-$\alpha$ humain révèle une concentration atteignant 1400 pg/ml en IP pour la souris injectée avec de l'Env-SU et 3000 pg/ml en IP, ainsi que 1200 pg/ml dans le sérum pour la souris injectée avec du LPS. Les mêmes tendances sont observées pour le dosage de l'Il-6 avec 1700 pg/ml détectés en IP chez la souris injectée avec de l'Env-SU et 9600 pg/ml et 1400 pg/ml respectivement en IP et dans le sérum de la souris injectée avec du LPS.

La décision de doser les cytokines murines chez des souris SCID-hu peut paraître surprenante, puisque celles-ci sont dépourvues de Lymphocytes T et B. Cependant, la population des monocytes/macrophages reste active et contribue à la production de TNF-$\alpha$ et d'IL-6 chez ces souris. Ainsi, il est montré que les concentrations en IL-6 murine détectées sont toujours supérieures aux concentrations en IL-6 humaine, ce qui n'est pas le cas pour le TNF-$\alpha$. Ce dernier point illustre donc parfaitement *« in vivo »* l'effet direct de l'enveloppe MSRV sur la composante de l'immunité innée, en l'absence de lymphocytes fonctionnels dans ce modèle SCID (pour la partie murine).

L'objet de cette étude était d'évaluer in vivo la pathogénicité associée à la protéine d'enveloppe recombinante, Env-SU, après avoir apporté la preuve d'une pathogénicité *in vitro* sur des PBMCs humains. Les effets pro-inflammatoires de la protéine Env-SU et du LPS, caractérisés par une production massive et ponctuelle (2h post-injection) de TNF-$\alpha$ et/ou d'IL-6 sont observés chez les souris SCID-hu. Suite aux résultats obtenus, on peut valider les protocoles expérimentaux de prélèvements et d'analyses de la production en cytokines (dans le sérum et par lavage IP) pour l'utilisation sur les SCID-humanisées, tels que mis au point sur un modèle murin dans une étude de faisabilité « technique » préalable sur les souris C57Bl6.

Modèle EAE

Le modèle EAE est un modèle animal de sclérose en plaques reposant sur l'induction en périphérie d'une autoimmunité dirigée contre des déterminants de la myéline.

Ce modèle est, à ce jour, le modèle de référence utilisé pour tous les protocoles de validation « pré-clinique » de molécules thérapeutiques destinées à traiter la sclérose en plaques.

Ce modèle est caractérisé par la présence de lymphocytes T autoréactifs et une démyélinisation conduisant à des signes neurologiques graves.

Son développement repose classiquement sur l'injection à des souris C57bl6 d'un peptide de myéline couplé à un adjuvant adéquat (adjuvant complet de Freund) et associé à une injection de toxine pertussis.

L'adjuvant qui est composé de mycobactéries inactivées permet la rupture de tolérance contre la myéline injectée et favorise le développement des lymphocytes T autoréactifs.

La toxine pertussis favorise l'ouverture de la barrière hémato-encéphalique mais joue également un rôle dans la rupture de tolérance.

Il a été montré que Env-SU activait le système immunitaire inné via le récepteur TLR4 et était capable d'induire le développement de réponses lymphocytaires de type Th1. Env-SU pourrait donc jouer un rôle d'adjuvant pour le déclenchement des mécanismes d'autoimmunité et de démyélinisation associés à la SEP. Ce rôle potentiel a été étudié dans le modèle EAE.

Trois expériences différentes ont été réalisées.

1 - Expérience préliminaire

Matériel et méthode

Le principe actif de l'adjuvant complet de Freund (mycobactéries inactivées) normalement utilisé pour le modèle de sclérose en plaques « EAE » classiquement réalisé, a été remplacé par la fraction Env-SU de la protéine d'enveloppe de MSRV.

Matériel

Huit souris C57Bl6 (Charles River).
Peptide de myéline MOG (Myelin Oligodendrocyte Glycoprotein) 35-55 immunogrades de Neosystem.
Adjuvant complet de Freund (CFA) de SIGMA.
Adjuvant incomplet de Freund (IFA) de SIGMA.
Pertussis Toxin (Salt free bordetella pertussis) de Calbiochem.
Env-SU de Protein Expert.

Méthode :

Injection sous cutanée de 200 $\mu$l de :

-   Contrôle positif :

MOG 150 $\mu$g + CFA : 3 souris testées.

-   Contrôle négatif :

MOG 150 $\mu$g + IFA : 2 souris testées.

-   Env-SU :

MOG 150 $\mu$g + IFA + Env-SU (50 $\mu$g) : 3 souris testées ;

puis injection de 200ul (IV) de toxin pertussis (200 ng) à J0 et J2.

Les signes neurologiques sont ensuite mesurés quotidiennement. Les différents stades sont listés ci-dessous en fonction des signes neurologiques observés.

Le stade 0 signifie pas de signes cliniques,
le stade 1 signifie queues molles,
le stade 2 signifie troubles de la démarche,
le stade 3 signifie paralysie partielle des membres postérieurs,
le stade 4 signifie paralysie totale des membres postérieurs,
le stade 5 signifie paralysie des membres postérieurs et paralysie partielle des membres antérieurs,
le stade 6 signifie animaux moribonds ou morts.

Résultats :

MOG (150 μg) + CFA : 2 souris sur 3 ont développé la maladie (stade 4).
MOG (150 μg) + IFA : aucun signe observé.
MOG (150 μg) + Env-SU (50 μg) : 3 souris sur 3 ont développé la maladie (stades 1 à 6).

Les résultats de l'étude préliminaire sont représentés dans la figure 18.

Cette étude préliminaire montre que Env-SU , qui active le système immunitaire via le récepteur TLR4, peut être utilisée comme adjuvant pour le développement du modèle de la SEP, l'EAE.

Le contrôle positif avec un adjuvant « classique » (ACF) valide l'expérience. Le contrôle négatif avec un adjuvant incomplet sans potentiel inducteur d'autoimmunité (AIF) valide la nécessité de stimuler le système immunitaire selon des voies spécifiques pour induire une réaction autoimmune.

Ainsi, dès ce stade préliminaire, il est évident que la protéine Env du rétrovirus MSRV /HERV-W est donc bien capable de provoquer une sensibilisation autoimmune avec un effet sur le système nerveux central comme l'adjuvant « expérimental » actuellement utilisé pour le modèle EAE.

La différence majeure avec l'ACF, dont le principe actif est un lysat de Mycobacterium tuberculosis, est que cette bactérie n'est nullement associée à la sclérose en plaque chez l'homme alors que le rétrovirus MSRV et ses analogues génétiques de la famille HERV-W sont clairement associés à la sclérose en plaques chez l'homme [2, 7, 8, 10, 61-63]. De plus, l'expression et la circulation dans les fluides biologiques de virions porteurs de cette protéine d'enveloppe est corrélée avec la progression de la maladie[10].

En conséquence et dès ce stade préliminaire, il est évident que tout agent thérapeutique susceptible d'inhiber le potentiel immunologique « inducteur d'autoimmunité » de la protéine d'enveloppe Env de cette famille rétrovirale est particulièrement intéressant, qui plus est s'l a été sélectionné pour son activité inhibitrice des effets anti-inflammatoires tels que décrits dans la présente invention avec les tests cellulaires *in vitro.* En effet, les anticorps monoclonaux dirigés contre les protéines Env MSRV/HERV-W, sont implicitement inhibiteurs des effets « inducteurs d'autoimmunité » de ces protéines lorsqu'elles sont exprimées à la surface des virions détectables chez les patients atteints de SEP [8, 10, 62]. Leur utilisation en thérapeutique humaine est évidente et techniquement à la portée de l'homme de l'art, selon des modalités connues pour des anticorps thérapeutiques actuellement autorisés et commercialisés pour la thérapie humaine, comme l'anticorps anti-TNF alpha commercialisé sous le nom de REMICAD qui est prescrit pour le traitement de la Polyarthrite Rhumatoïde notamment. Il est intéressant, en outre, de noter ici que cet anticorps thérapeutique, disponible commercialement, cible un produit « aval » de la cascade d'activation pro-inflammatoire, alors que selon l'invention, la cible thérapeutique est inhibée bien avant l'induction de TNF-alpha via le TLR4 notamment.

Il est aussi important de noter que les agents thérapeutiques actuellement proposés dans la thérapie de la SEP (corticostéroides, interféron beta ou autres) agissent sur une partie limitée seulement des composantes pro-inflammatoires produites consécutivement à l'initiation de la cascade immunopathologique, ce qui explique leur efficacité partielle et relative dans le traitement des patients.

Par contre, en inhibant l'effet primaire de la protéine Env MSRV/HERV-W avant l'activation de la voie du récepteur TLR4 et donc de l'immunité innée mise en jeu dans cette phase initiale, le seul agoniste immunopathogénique présent à ce stade est inhibé, ce qui n'est plus le cas avec la multitude des produits pro-inflammatoires sécrétés après activation primaire de cette voie (Figure 8). Cet avantage « biologique » apporte un potentiel d'efficacité unique chez les malades, d'autant plus qu'il cible un agent « clé » dans l'étiopathogénie de la SEP, et non un seul des sous-produits d'activation dont l'efficacité a été mesurée dans un modèle EAE induit par l'agent de la tuberculose (*M. tuberculosis* dans l'Adjuvant Complet de Freund) qui n'a rien à voir avec la maladie humaine SEP.

2 - Expérience 2.

Le même type d'expérience a été effectué afin de confirmer les résultats préliminaires observés.

Méthode

Injection sous cutanée de 200 μl de :

- MOG (150 μg) + CFA : 4 souris testées.
- MOG (150 μg) + IFA : 3 souris testées.
- MOG (150 μg) + IFA + Env-SU (50 μg) : 4 souris testées.
- MOG (150 μg) + IFA + LPS (20ug) : 4 souris testées ;

puis injection de 200 μl (IV) de toxin pertussis (200 ng) à J0 et J2.

Mesure des signes cliniques.

Les rates de toutes les souris ont ensuite été récupérées puis des suspensions cellulaires ont été congelées.
Les cerveaux de 2 souris ont été récupérés puis congelés après transfusion au PFA 4%. (Cerveau d'une souris Env stade 3 ; Cerveau d'une souris LPS stade 0).
Des lésions inflammatoires caractéristiques ont été visualisées par analyse histologique dans le cerveau de la souris ayant reçu la protéine Env et non dans celui de la souris ayant reçu l'injection de LPS.

Résultats :

Suivi des signes neurologiques :

- MOG (150 μg) + CFA : 4 souris sur 4 ont développé la maladie (stades 2 à 6).
- MOG (150 μg) + IFA : Aucun signe observé.
- MOG (150 μg) + Env-SU (50 μg) : 4 souris sur 4 ont développé la maladie (stades1 à 5).
- MOG (150 μg) + LPS (20 μg) : Aucun signe observé.

Les résultats sont illustrés dans la Figure 19.
Ces résultats confirment que Env-SU peut avoir un rôle adjuvant dans l'induction des signes neurologiques observés lors du développement du modèle de SEP qu'est l'EAE.
En plus des contrôles précédents, du LPS (lipoplysaccharide bactérien) a été utilisé, car il stimule le même récepteur à la surface des cellules présentatrices de l'antigène que la protéine Env MSRV: TLR4. L'absence d'effet inducteur d'autoimmunité du LPS dans ces conditions, montre que la potentialité immunologique de la protéine Env est bien supérieure à celle d'autres ligands du TLR4 et que, logiquement, un inhibiteur ciblant cette protéine sera un meilleur outil thérapeutique que des molécules inhibant non-spécifiquement certaines voies activées par cette dernière.

Etudes fonctionnelles :

Les rates ont été décongelées puis les splénocytes ont été re-stimulés avec du peptide MOG in vitro, puis la production d'IFN-g a été mesurée (Cinétique et dose réponse).
$2 \times 10^6$ splénocytes /ml de c-RPMI + 10% FCS.
Les moyennes de 3 souris pour Env et 2 pour IFA et LPS sont présentées.
La figure 20 illustre la réponse en fonction de la dose de MOG (effet-dose), par le dosage de l'interféron gamma qui signe l'activation des lymphocytes T en présence de l'autoantigène spécifique et montre bien la réalité de la réponse autoimmune induite dans ce modèle par la protéine Env MSRV/HERV-W, en utilisant le fragment Env-SU qui stimule spécifiquement la voie TLR-4 dans ces conditions. Dans ce modèle de SEP, la réponse des lymphocytes T ne résulte pas de l'activation directe par Env MSRV/HERV-W, par exemple par le récepteur T (TCR) comme dans le cas d'un superantigène, mais bien en amont au niveau des cellules de l'immunité innée (monocytes/macrophages, cellules dendritiques etc..) comme les nombreux résultats de la présente invention le montrent (absence de sécrétion d'IFN-gamma, stimulation des monocytes et cellules dendritiques purifiées, stimulation des cytokines « macrophagiques » murines dans le modèle SCID qui ne comporte pas de lymphocytes murins fonctionnels, cinétique IL-6 parallèle à celle du LPS, absence d'IL-6 et de TNF-alpha mais bien d'Interféron gamma induit dans les mêmes conditions par un superantigène de référence -SEB-, etc...).
Ceci est encore confirmé par l'étude de la cinétique dans le temps de la réponse lymphocytaire T autoimmune contre l'antigène de myéline MOG ajouté à 10 microg/ml dans le milieu de culture utilisé pour tester les splénocytes prélevés sur les animaux « EAE/MOG/Env-SU » et les contrôles « sans Env » avec adjuvant incomplet de Freund (IFA, sans extrait de *M. tuberculosis*) et MOG. Ceci est illustré par la figure 21 qui montre la progression très significative de la réponse autoimmune anti-MOG au cours du temps dans les seules souris ayant reçu de l'Env-SU.
Ces résultats montrent donc bien qu'une stimulation par Env-SU associée à un autoantigène permet, en aval de la cascade initiée par Env-SU au niveau du récepteur TLR4 sur les cellules présentatrices de l'antigène (APC dont font partie les monocytes/macrophages, les cellules dendritiques, les microgliocytes cérébraux,..), le développement de lymphocytes T autoréactifs qui sont à l'origine exclusive de l'Interféron gamma (IFN-g) relargués en quantités très élevées (voir graphiques) et donc d'une autoimmunité médiée par ces lymphocytes T *in vivo.*

3 - Expérience 3.

Le même type d'expérience a été effectué afin de confirmer les résultats préliminaires observés amis également de tester in vivo les effets thérapeutiques (sur la conséquence clinique mesurée dans le modèle) des anticorps anti Env-SU (représentés ici par l'anticorps monoclonal 3B2H4), parallèlement à des anticorps de même isotype mais sans spécificité équivalente (représentés ici par le monoconal anti-GAG, 3H1H6).

Méthode.

Injection sous cutanée d'une dose 200 microlitres d'anticorps à 5 microgramme/ml, soit 1 microgramme d'anticorps par souris d'environ 20 grammes, soit de 50 microgrammes par Kg :

- MOG (150 μg) + CFA : 5 souris testées.
- MOG (150 μg) + IFA : 5 souris testées.
- MOG (150 μg) + IFA + Env-SU (50 μg) : 5 souris testées.
- MOG (150 μg) + IFA + Env-SU (50 μg) : 5 souris testées. Ces souris ont en plus reçu 1 mg Ac 3B2H4 IV (200 μl) ;

puis injection de 200ul (IP) de toxin pertussis (200 ng) à J0 et J2 et mesure des signes cliniques.

Résultats :

Les résultats obtenus sont illustrés dans la figure 22.

Afin de tester des conditions plus proches du développement d'une autoimmunité progressive comme dans la SEP, les résultats sont obtenus dans des conditions plus « modérées » que les précédents car la toxine a été injectée par voie IP et non IV comme précédemment.

Le témoin positif « MOG+CFA » correspond ici à 4/5 souris avec des signes cliniques et que le témoin négatif « MOG+IFA » correspond à 0/5 souris atteinte.

Avec la protéine Env comme avec les témoins positifs « MOG+CFA », le score clinique moyen est ici réduit par rapport aux conditions précédentes. Cependant la nette réduction de l'effet pathogène de cette protéine en présence d'anticorps anti-Env est illustrée par l'atteinte minimale observée chez les souris traitées.

On note ainsi que l'anticorps « anti-Gag » n'a aucun effet inhibiteur sur l'effet immunopathologique induit par la protéine Env, voire même potentialise un peu cette effet par sa présence, alors que l'anticorps 3B2H4 a un très net effet inhibiteur qui rapproche le plus la courbe observée de celle des témoins négatifs « MOG+CFA ». L'effet inhibiteur in vivo des effets « inducteurs d'autoimmunité médiée par les lymphocytes T » de type EAE est donc lié à la présence de l'anticorps 3B2H4 anti-Env MSRV/HERV-W.

Dans le modèle de sclérose en plaques « EAE », ce sont les effets cliniques (atteinte neurologique) qui sont mesurés en particulier et pas seulement les paramètres biologiques associés. L'effet étudié n'est donc plus seulement un effet biologique tel que décrit précédemment, mais sa traduction clinique dans le cadre d'un modèle pathologique dédié. Donc ce qui est mesuré ici est bien un effet thérapeutique. Or, il est bien connu de l'homme de l'art que ce sont là les limites qualitatives de la validation thérapeutique « pré-clinique» pour la thérapie humaine, car toute validation thérapeutique ultérieure sur la maladie humaine doit se faire sur l'homme sur la base des critères obtenus sur un modèle animal.

Une fois les agents thérapeutiques candidats identifiés et sélectionnés et les modèles animaux dédiés mis au point et validés, comme dans la présente invention, une extension « quantitative » des séries de test effectués peut implicitement être effectuée en développant les outils et les modèles déjà obtenus avec des contrôles adaptés et bien connus de l'homme de l'art communs aux études pharmacologiques, afin de répondre aux critères pré-cliniques.

Les éléments obtenus sont donc nécessaires et suffisans pour finaliser les validations pré-cliniques et développer une expérimentation thérapeutique chez l'homme.

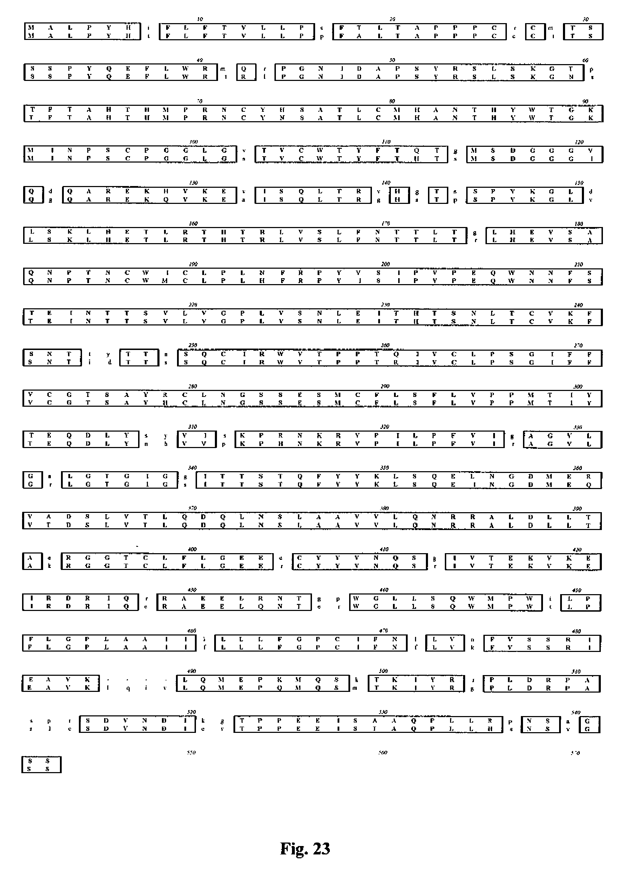

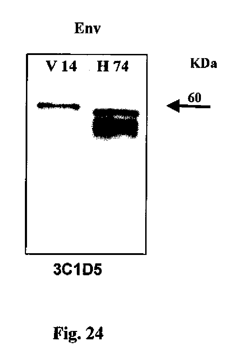

Par ailleurs, l'analyse de séquence aminoacide des protéines ENV MSRV et HERV-W7q (syncitine) montrent la forte homologie et la conservation des principaux motifs aminoacide dans la famille MSRV/HERV-W (Figure 23). Ceci se traduit par une réactivité croisée aux anticorps monoclonaux anti-ENV (Figure 24) .

L'analyse des séquences (Cf. Figure 25) permet aussi d'évaluer des régions antigéniques d'intérêt dans la séquence de la protéine ENV-SU référencée en SEQ ID NO : 1 correspondant aux régions définies par les acides aminés 122-131 (inclus) et/ou 312-316 (inclus) et/ou 181-186 (inclus).

En conséquence, il a été confirmé dans ce modèle animal de SEP, qu'un anticorps monoclonal dirigé contre la protéine d'enveloppe Env des rétrovirus de la famille MSRV/HERV-W et, en particulier, de son membre prototype MSRV, notamment sélectionné pour ses propriétés inhibitrices de la voir pro-inflammatoire initiée par le récepteur TLR4 dans un test cellulaire, constitue un agent thérapeutique capable d'inhiber le potentiel immunopathologique, notamment « inducteur d'autoimmunité », de la protéine d'enveloppe ENV de cette famille rétrovirale.

Il est donc maintenant prouvé que :

1) Les virions MSRV enveloppés sont détectés chez les patients atteints de Sclérose en plaques [4, 8, 10, 62, 64]

2) Leur expression corrèle l'évolution de la maladie [10]

3) La réponse immunologique à la protéine Env MSRV corrèle la progression et la gravité de la maladie [65]

4) Les virions MSRV possèdent un ARN codant pour la protéine Env MSRV [66]

5) Les protéines Env de la famille MSRV/HERV-W ont une très forte homologie au niveau de leur séquence aminoacide et au niveau des séquences génétiques qui les codent [2, 5, 66]

6) La protéine Env MSRV et la protéine Env codée par la copie HERV-W dans la région du chromosome humain 7q21-22 (HERV-W7q), ont des propriétés pro-inflammatoires *in vitro* et *in vivo* (exemples de la présente demande de brevet et [11, 12, 59]

7) La protéine Env MSRV est capable de reproduire le modèle animal bien connu de Sclérose en Plaques (SEP), à savoir l'encéphalomyélite allergique expérimentale (EAE), en présence d'un autoantigène du système nerveux central issu de la myéline (Myelin Oligodendrocyte Glycoprotein, MOG, exemple de la présente demande de brevet).

8) Ce modèle expérimental est habituellement provoqué artificiellement par un extrait antigénique de *Mycobacterium tuberculosis,* agent bactérien de la tuberculose, sans rapport avec l'étiologie de la Sclérose en Plaque humaine. Son obtention avec la Protéine d'enveloppe du rétrovirus MSRV, appartenant à la famille rétrovirale endogène HERV-W, dont l'expression est détectable en corrélation avec la maladie sous forme de virions [8, 10, 62] ou sous forme de protéine Env spécifiquement exprimée dans les lésions de démyélinisation caractéristiques de la SEP [59, 67], constitue un modèle animal nouveau et unique permettant à l'étude des agents thérapeutiques ciblant un agent rétroviral impliqué dans l'immunopathogénie de la maladie.

9) On retrouve bien les effets pro-inflammatoire associés à l'activation des lymphocytes T décrits sur les cellules humaines, au niveau des lymphocytes T murins du modèle EAE induit par la protéine Env MSRV ainsi que l'attestent les dosages de production d'interféron gamma (exemple de la présente demande de brevet).

10) Les effets pro-inflammatoires de la protéine Env MSRV sont médiés par les cellules lymphoides et les cellules présentatrices de l'antigène, donc par les sytème immunitaire (exemples de la présente demande de brevet, [11, 12]).

11) Des anticorps monoclonaux anti-Env MSRV (3B2H4 et 13H5A5) sont capable d'inhiber spécifiquement les effets pro-inflammatoires de la protéine Env MSRV sur des cellules lymphoides (lymphocytes et monocytes) sanguines humaines (exemples de la présente demande de brevet).

12) L'effet « inhibiteur spécifique » d'un anticorps monoclonal (3B2H4) dirigé contre la protéine Env MSRV est confirmé dans le modèle animal de l'EAE induit par ENV MSRV. Cet effet se traduit par une amélioration clinique notable des animaux traités par rapport aux animaux non-traités ou traités par un anticorps non pertinent de même isotype. (exemple de la présente demande de brevet).

Les anticorps monoclonaux anti-Env MSRV/HERV-W peuvent donc avoir un effet inhibiteur de l'inflammation, de l'autoimmunité et des troubles cliniques neurologiques induits par une telle protéine d'un agent rétroviral associé à la maladie.

Il est donc évident que les anticorps dont les propriétés ont été vérifiées *in vitro* et *in vivo,* constituent de nouveaux agents thérapeutiques de la maladie humaine, sclérose en plaques, comme tels ou améliorés par les techniques biologiques, notamment de génie génétique.

Les tests cellulaires et les modèles animaux appropriés à l'évaluation pré-clinique de ces anticorps thérapeutiques sont ici décrits et permettent maintenant à l'homme de l'art de réaliser les étapes de validation requises avant les essais thérapeutiques chez l'homme et de les adapter à différentes pathologies associées à la famille rétrovirale MSRV/HERV-W.

## REFERENCES BIBLIOGRAPHIQUES

1. Conrad, B., et al., A human endogenous retroviral superantigen as candidate autoimmune gene in type I diabetes. Cell, 1997. 90(2): p. 303-13.

2. Perron, H., et al., Molecular identification of a novel retrovirus repeatedly isolated from patients with multiple sclerosis. The Collaborative Research Group on Multiple Sclerosis. Proc Natl Acad Sci USA, 1997. 94(14): p. 7583-8.

3. Deb-Rinker, P., et al., Molecular characterization of a MSRV-like sequence identified by RDA from monozygotic twin pairs discordant for schizophrenia. Genomics, 1999. 61(2): p. 133-44.

4. Perron, H., et al., Isolation of retrovirus from patients with multiple sclerosis [letter]. Lancet, 1991. 337(8745): p. 862-3.

5. Blond, J.L., et al., Molecular characterization and placental expression of HERV-W, a new human endogenous retrovirus family. J Virol, 1999. 73(2): p. 1175-85.

6. Perron, H., et al., Particle-associated retroviral RNA and tandem RGH/HERV-W copies on human chromosome 7q: possible components of a 'chain-reaction' triggered by infectious agents in multiple sclerosis? J Neurovirol, 2000. 6(Suppl 2): p. S67-75.

7. Dolei, A., et al., Multiple sclerosis-associated retrovirus (MSRV) in Sardinian MS patients. Neurology, 2002. 58(3): p. 471-3.

8. Garson, J.A., et al., Detection of virion-associated MSRV-RNA in serum of patients with multiple sclerosis [letter] [see comments]. Lancet, 1998. 351(9095): p. 33.

9. Olsson, P., et al., Retroviral RNA related to ERV9/MSRV in a human serum: a new sequence variant. AIDS Res Hum Retroviruses, 1999. 15(6): p. 591-3.

10. Sotgiu, S., et al., Multiple sclerosis-associated retrovirus and MS prognosis: an observational study. Neurology, 2002. 59(7): p. 1071-3.

11. Perron, H., et al., Multiple sclerosis retrovirus particles and recombinant envelope trigger an abnormal immune response in vitro, by inducing polyclonal Vbeta16 T-lymphocyte activation. Virology, 2001. 287(2): p. 321-32.

12. Firouzi, R., et al., Multiple Sclerosis Associated Retrovirus Particles Cause T-Lymphocyte Dependent Death with Brain Hemorrhage, in Humanized SCID Mice Model. Journal of Neurovirology, 2003. 9: p. 79-93.

13. Lin, A., et al., The inflammatory response system in treatment-resistant schizophrenia: increased serum interleukin-6. Schizophr Res, 1998. 32(1): p. 9-15. 6: Stevens JR. Neuropathology of schizophren...[PMID: 7125843]Related Articles, Links.

14. Karlsson, H., et al., Retroviral RNA identified in the cerebrospinal fluids and brains of individuals with schizophrenia. Proc Natl Acad Sci U S A, 2001. 98(8): p. 4634-9.

15. Perron, H., Microbial Agents triggering Endogenous Retroviruses within Genetic Susceptibility Loci, Resulting in Expression of Superantigen and Gliotoxic Molecules: a Plausible " Immunovirogenetic " Cascade causing Multiple Sclerosis ? Modern Aspects of Immunobiology, 2001. 1(5): p. 198-203.

16. Liu, Y., et al., Dextromethorphan protects dopaminergic neurons against inflammation-mediated degeneration through inhibition of microglial activation. J Pharmacol Exp Ther, 2003. 305(1): p. 212-8. 2: Gao HM et al. Synergistic dopaminergic neur...[PMID: 12598611]Related Articles, Links.

17. Morimoto, K., T. Murasugi, and T. Oda, Acute neuroinflammation exacerbates excitotoxicity in rat hippocampus in vivo. Exp Neurol, 2002. 177(1) : p. 95-104. 5: Stoll G. Inflammatory cytokines in the...[PMID: 12407295]Related Articles, Links.

18. Guillemin, G.J. and B.J. Brew, Implications of the kynurenine pathway and quinolinic acid in Alzheimer's disease. Redox Rep, 2002. 7(4): p. 199-206. 7: Kim EJ et al. Neuroprotective effects of pr...[PMID: 12237868]Related Articles, Links.

19. Kim, W.G., et al., Regional difference in susceptibility to lipopolysaccharide-induced neurotoxicity in the rat brain: role of microglia. J Neurosci, 2000. 20(16): p. 6309-16. 15: Czlonkowska A et al. Inflammatory changes in the s...[PMID: 10894230]Related Articles, Links.

20. Licinio, J. and M.L. Wong, The role of inflammatory mediators in the biology of major depression: central nervous system cytokines modulate the biological substrate of depressive symptoms, regulate stress-responsive systems, and contribute to neurotoxicity and neuroprotection. Mol Psychiatry, 1999. 4(4): p. 317-27. 18: Cotter RL et al. Insights into the neurodegene...[PMID: 10204569]Related Articles, Links.

21. Cotter, R.L., et al., Insights into the neurodegenerative process of Alzheimer's disease: a role for mononuclear phagocyte-associated inflammation and neurotoxicity. J Leukoc Biol, 1999. 65(4): p. 416-27. 19: Heese K et al. Inflammatory signals induce n...[PMID: 9453564]Related Articles, Links.

22. Heese, K., C. Hock, and U. Otten, Inflammatory signals induce neurotrophin expression in human microglial cells. J Neurochem, 1998. 70(2) : p. 699-707. 20: Sasser LB et al. Subchronic toxicity evaluatio...[PMID: 8600286]Related Articles, Links.

23. Chao, C.C., et al., Interleukin-1 and tumor necrosis factor-alpha synergistically mediate neurotoxicity: involvement of nitric oxide and of N-methyl-D-aspartate receptors. Brain Behav Immun, 1995. 9(4): p. 355-65. 22: Chao CC et al. Tumor necrosis factor-alpha p...[PMID: 7705222]Related Articles, Links.

24. Chao, C.C. and S. Hu, Tumor necrosis factor-alpha potentiates glutamate neurotoxicity in human fetal brain cell cultures. Dev Neurosci, 1994. 16(3-4): p. 172-9.

25. Bal-Price, A. and G.C. Brown, Inflammatory neurodegeneration mediated by nitric oxide from activated glia-inhibiting neuronal respiration, causing glutamate release and excitotoxicity. J Neurosci, 2001. 21(17): p. 6480-91. 5: Obrenovitch TP. Quinolinic acid accumulation ...[PMID: 11462760]Related Articles, Links.

26. Obrenovitch, T.P., Quinolinic acid accumulation during neuroinflammation. Does it imply excitotoxicity? Ann N Y Acad Sci, 2001. 939(1-10.): p. Law A et al. Say NO to Alzheimer's disease...[PMID: 11245887]Related Articles, Links.

27. Werner, P., D. Pitt, and C.S. Raine, Glutamate excitotoxicity--a mechanism for axonal damage and oligodendrocyte death in Multiple Sclerosis? J Neural Transm Suppl, 2000(60): p. 375-85. 8: Pitt D et al. Glutamate excitotoxicity in a...[PMID: 10613826]Related Articles, Links.

28. Pitt, D., P. Werner, and C.S. Raine, Glutamate excitotoxicity in a model of multiple sclerosis. Nat Med, 2000. 6(1): p. 67-70. 9: Carlson NG et al. Inflammatory cytokines IL-1 a...[PMID: 10490998]Related Articles, Links.

29. Carlson, N.G., et al., Inflammatory cytokines IL-1 alpha, IL-1 beta, IL-6, and TNF-alpha impart neuroprotection to an excitotoxin through distinct pathways. J Immunol, 1999. 163(7): p. 3963-8. 10: Wang YS et al. The bacterial endotoxin lipop...[PMID: 9930737]Related Articles, Links.

30. Wang, Y.S. and T.D. White, The bacterial endotoxin lipopolysaccharide causes rapid inappropriate excitation in rat cortex. J Neurochem, 1999. 72(2): p. 652-60. 11: Chao CC et al. Tumor necrosis factor-alpha p...[PMID: 7705222]Related Articles, Links.

31. Yolken, R.H., et al., Endogenous retroviruses and schizophrenia. Brain Res Brain Res Rev, 2000. 31(2-3): p. 193-9.

32. Kleine, T.O., et al., Approach to discriminate subgroups in multiple sclerosis with cerebrospinal fluid (CSF) basic inflammation indices and TNF-alpha, IL-Ibeta, IL-6, IL-8. Brain Res Bull, 2003. 61(3): p. 327-46. 2: Aarli JA. Role of cytokines in neurolog...[PMID: 12871095]Related Articles, Links.

33. Aarli, J.A., Role of cytokines in neurological disorders. Curr Med Chem, 2003. 10(19): p. 1931-7. 3: Vladic A et al. Cerebrospinal fluid and serum... [PMID: 12445803]Related Articles, Links.

34. Miljkovic, D., et al., Nitric oxide metabolites and interleukin-6 in cerebrospinal fluid from multiple sclerosis patients. Eur J Neurol, 2002. 9(4): p. 413-8. 5: Clerici M et al. Single-cell analysis of cytok...[PMID: 11730945]Related Articles, Links.

35. Fedetz, M., et al., The -174/-597 promoter polymorphisms in the interleukin-6 gene are not associated with susceptibility to multiple sclerosis. J Neurol Sci, 2001. 190(1-2): p. 69-72. 7: Stelmasiak Z et al. IL-6 and sIL-6R concentration...[PMID: 11535934]Related Articles, Links.

36. Vandenbroeck, K., et al., High-resolution analysis of IL-6 minisatellite polymorphism in Sardinian multiple sclerosis: effect on course and onset of disease. Genes Immun, 2000. 1(7): p. 460-3. 9: Stelmasiak Z et al. Interleukin-6 concentration i...[PMID: 11208463]Related Articles, Links.

37. Stelmasiak, Z., et al., Interleukin-6 concentration in serum and cerebrospinal fluid in multiple sclerosis patients. Med Sci Monit, 2000. 6(6): p. 1104-8. 10: Schonrock LM et al. Interleukin-6 expression in h...[PMID: 11044583]Related Articles, Links.

38. Cornford, E.M. and M.E. Cornford, New systems for delivery of drugs to the brain in neurological disease. Lancet Neurol, 2002. 1(5): p. 306-15. 4: Schmidt J et al. Drug targeting by long-circul...[PMID: 12805101]Related Articles, Links.

39. Schmidt, J., et al., Drug targeting by long-circulating liposomal glucocorticosteroids increases therapeutic efficacy in a model of multiple sclerosis. Brain, 2003. 126(Pt 8): p. 1895-904. 5: Fournier E et al. Therapeutic effectiveness of ...[PMID: 12767096]Related Articles, Links.

40. Pardridge, W.M., Blood-brain barrier drug targeting enables neuroprotection in brain ischemia following delayed intravenous administration of neurotrophins. Adv Exp Med Biol, 2002. 513(397-430.): p. Watanabe S et al. Chemotherapeutic targeting of...[PMID: 12575735]Related Articles, Links.

41. Watanabe, S., et al., Chemotherapeutic targeting of etoposide to regions of the brain on the basis of polyamine level. J Drug Target, 2002. 10(6): p. 457-61. 13: Lahiri DK et al. A critical analysis of new mo...[PMID: 12558063]Related Articles, Links.

42. Scherrmann, J.M., Drug delivery to brain via the blood-brain barrier. Vascul Pharmacol, 2002. 38(6): p. 349-54. 15: Wang JX et al. Enhanced brain targeting by s...[PMID: 12445558]Related Articles, Links.

43. Wang, J.X., X. Sun, and Z.R. Zhang, Enhanced brain targeting by synthesis of 3',5'-dioctanoyl-5-fluoro-2'-deoxyuridine and incorporation into solid lipid nanoparticles. Eur J Pharm Biopharm, 2002. 54(3): p. 285-90. 16: Mahar Doan KM et al. Passive permeability and P-gl...[PMID: 12438524]Related Articles, Links.

44. Hosoya, K., S. Ohtsuki, and T. Terasaki, Recent advances in the brain-to-blood efflux transport across the blood-brain barrier. Int J Pharm, 2002. 248(1-2): p. 15-29. 18: Mora M et al. Design and characterization o...[PMID: 12425459]Related Articles, Links.

45. Mora, M., et al., Design and characterization of liposomes containing long-chain N-acylPEs for brain delivery: penetration of liposomes incorporating GM1 into the rat brain. Pharm Res, 2002. 19(10): p. 1430-8.

46. Perron, H., et al., Herpes simplex virus ICP0 and ICP4 immediate early proteins strongly enhance expression of a retrovirus harboured by a leptomeningeal cell line from a patient with multiple sclerosis. J Gen Virol, 1993. 74(Pt 1): p. 65-72.

47. Soldan, S., et al., Association of human herpes virus 6 (HHV-6) with multiple sclerosis: increased IgM response to HHV-6 early antigen and detection of serum HHV-6 DNA. Nat Med, 1997. 3: p. 1394-1397.

48. Haahr, S., et al., Is multiple sclerosis caused by a dual infection with retrovirus and Epstein-Barr virus? Neuroepidemiology, 1992. 11(4-6): p. 299-303.

49. Bergström, T., O. Andersen, and A. Vahlne, Isolation of herpes simplex virus type 1 during first attack of multiple sclerosis. Ann Neurol, 1989. 26: p. 283-285.

50. Marx, C.E., et al., Cytokine effects on cortical neuron MAP-2 immunoreactivity: implications for schizophrenia. Biol Psychiatry, 2001. 50(10): p. 743-9. 2: Maes M et al. Effects of atypical antipsych...[PMID: 10706993]Related Articles, Links.

51. Minagar, A., et al., The role of macrophage/microglia and astrocytes in the pathogenesis of three neurologic disorders: HIV-associated dementia, Alzheimer disease, and multiple sclerosis. J Neurol Sci, 2002. 202(1-2): p. 13-23. 9: Jeohn GH et al. Go6976 protects mesencephalic...[PMID: 12076986]Related Articles, Links.

52. Gaser, C., et al., Ventricular enlargement in schizophrenia related to volume reduction of the thalamus, striatum, and superior temporal cortex. Am J Psychiatry., 2004. 161: p. 154-156.

53. Kurtzke, J., Disability rating scales in multiple sclerosis. Ann N Y Acad Sci., 1984. 436: p. 347-60.

54. Karlsson, H., et al., HERV-W-related RNA detected in plasma from individuals with recent-onset schizophrenia or schizoaffective disorder. Mol Psychiatry, 2004. 9: p. 12-13.

55. Qiu, Z. and D.L. Gruol, Interleukin-6, beta-amyloid peptide and NMDA interactions in rat cortical neurons. J Neuroimmunol, 2003. 139(1-2): p. 51-7. 2: Jenner P. Oxidative stress in Parkinson...[PMID: 12666096]Related Articles, Links.

56. Lafon, M., et al., Human Viral superantigens: to be or not to be transactivated ? Trends in Immunology, 2002. 23(5): p. 238-239.

57. Pranzatelli, M., Innovations in drug delivery to the central nervous system. Drugs Today (Barc). 1999. 35: p. 435-448.

58. Merlo, A., J. Mueller-Brand, and H. Maecke, Comparing monoclonal antibodies and small peptidic hormones for local targeting of malignant gliomas. Acta Neurochir., 2003. 88: p. 83-91.

59. Antony, J., et al., Human endogenous retrovirus glycoprotein-mediated induction of redox reactants causes oligodendrocyte death and demyelination. Nat Neurosci., 2004. 7(10): p. 1088-95.

60. Ng, P. and Y. Osawa, Preparation and characterization of the F (ab)2 fragments of an aromatase activity-suppressing monoclonal antibody. Steroids., 1997. 62: p. 776-81.

61. Perron, H., et al., In vitro transmission and antigenicity of a retrovirus isolated from a multiple sclerosis patient. Res Virol, 1992. 143(5): p. 337-50.

62. Serra, C., et al., Multiple sclerosis and multiple sclerosis-associated retrovirus in Sardinia. Neurol Sci, 2001. 22(2): p. 171-3.

63. Zawada M Fau - Liwien, I., et al., MSRV pol sequence copy number as a potential marker of multiple sclerosis. Pol J Pharmacol, 2003. 55(5): p. 869-75.

64. Perron H Fau - Garson, J.A., et al., Molecular identification of a novel retrovirus repeatedly isolated from. Proc Natl Acad Sci U S A, 1997. 94(14): p. 7583-8.

65. Rolland, A., et al., Correlation between disease severity and in vitro cytokine production mediated by MSRV (Multiple Sclerosis associated RetroViral element) envelope protein in patients with multiple sclerosis. J Neuroimmunol, 2004. **In press** (Published Online Dec.2004.).

66. Komurian-Pradel, F., et al., Molecular cloning and characterization of MSRV-related sequences associated with retrovirus-like particles. Virology, 1999. 260(1) : p. 1-9.

67. Perron, H., et al., Human Endogenous Retrovirus (HERV-W Env And Gag Proteins: Physiological Expression In Human Brain And Pathophysiological Modulation In Multiple Sclerosis Lesions. J. Neurovirology, 2005. In press.

**Revendications**

1. Procédé de détection et/ou de dosage de cytokines pro-inflammatoires, selon lequel on incube des cellules mononuclées sanguines avec la protéine Env-SU de MSRV/HERV-W et on dose les cytokines sécrétées par lesdites cellules.

2. Procédé selon la revendication 1, selon lequel les cytokines sont choisies parmi IL-6, IL-10, IL-12p40, IL-1β, IFNγ, IL-12p70 et TNF-α.

3. Procédé selon la revendication 2, selon lequel les cytokines sont choisies parmi IL-6, IL-10, IL-12p40, IL-1β, IFNγ et TNF-α.

4. Procédé selon l'une quelconque des revendications 1 à 3, selon lequel les cellules mononuclées sanguines sont des monocytes ou des cellules dendritiques.

**5.** Procédé selon la revendication 4, selon lequel on détecte et/ou on dose les cytokines IL-6, IL-12p40, IL-12p70 et TNF-α.

**a**

ENV pV14

S291  K316

M1    S30                                                          L449                        S542

Signal
peptide                    Ectodomain                       Trans      Cytoplasmic
                                                            membrane       domain

ENV-

M1    S30                                    K316

Signal
peptid

**b**

MALPYHTFLFTVLLPPFALTAPPPCCCTT SSSPYQEFLWRTRLPGNIDAPSYRSLSKGNSTFTAHTHMPRNCYNSATLCMH
ANTHYWTGKMINPSCPGGLGATVCWTYFTHTSMSDGGGIQGQAREKQVKEAISQLTRGHSTPSPYKGLVLSKLHETLRTHT
RLVSLFNTTLTRLHEVSAQNPTNCWMCLPLHFRPYISIPVPEQWNNFSTEINTTSVLVGPLVSNLEITHTSNLTCVKFSNT
IDTTSSQCIRWVTPPTRIVCLPSGIFFVCGTSAYHCLNGSSESMCFLSFLVPPMTIYTEQDLYNHVVPKPHNK

Fig. 1

**A**

**B**

Fig. 2

**Fig. 3**

**Fig. 4**

**a**

| | |
|---|---|
| CD80 PE | CD86 PE |

— Mock
ENV-SU
— LPS

**b**

TNF-alpha    IL-1beta    IL-6    IL-12p40

ng/ml

Mock control    ENV-SU    LPS

**Fig. 5**

a

b

c

Fig. 6

**Fig. 7**

Fig. 8

**Macrophage
Microgliocyt**

**TLR4**

I

**Anticorps
inhibiteurs   -ENV**

**Production Locale
Cytokinespro-inflammatoire
(IL-6, TNFAlpha, IL-beta.)
Free Radicaux libres (O. et
Metalloprotéase**
........

Schizophrenie          IV    Sclerose en Plaques

*Dysfunction Neuronale*

*Autoimmunité*

*biaisTh1*

-Proximité des cellules
neuronales
-Pas de Lymphocyte       III
-Excitotoxicité
-Relarguage/inhibition
De  Neuromediators

-Presentation aux
Lymphocytes-T
relarguage
d'autoantigènes
-destruction tissulaire

**INFLAMMATION LOCALE**   II

**Fig. 9**

47

Fig. 10

**Fig. 11**

**Fig. 12**

Fig. 13

**a**

SCZ     DS

**b**

SCZ     DS

**c**

SCZ     DS

**Fig. 14**

**Fig. 15A-B**

C

**Fig. 15C**

**Fig. 15D**

**Fig. 16**

**Fig. 17**

**Fig. 18**

**Fig. 19**

**Fig. 20**

.

**Fig. 21**

**Fig. 22**

**Fig. 23**

**Env**

**3C1D5**

**Fig. 24**

**Fig. 25a**

**Fig. 25b**

Kyte/Doolittle hydrophilicity:   Window = 7

Goldman/Engelman/Steitz hydrophilicity:   Window = 7

Protein flexibility:   Window = 7

Surface Probability:   Window = 7

**Fig. 25c**

Surface Probability:   Window = 7

Europäisches
Patentamt

European
Patent Office

Office européen
des brevets

**RAPPORT DE RECHERCHE EUROPEENNE**

Numéro de la demande

EP 16 18 4011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D,P | ROLLAND A ET AL: "Correlation between disease severity and in vitro cytokine production mediated by MSRV (Multiple Sclerosis associated RetroViral element) envelope protein in patients with multiple sclerosis", JOURNAL OF NEUROIMMUNOLOGY, ELSEVIER SCIENCE PUBLISHERS BV, vol. 160, no. 1-2, mars 2005 (2005-03), pages 195-203, XP004745503, ISSN: 0165-5728 * Available online on 08.12.2004.; page 201; figure 4 * | 1-5 | INV. C07K16/28 |
| A | WO 01/31021 A (UNIV GENEVE ; CONRAD BERNARD (CH); MACH BERNARD (CH)) 3 mai 2001 (2001-05-03) * page 26 - page 28 * | 1-5 | |
| A | WO 99/67395 A (INST NAT SANTE RECH MED ; PERIN JEAN PIERRE (FR); RIEGER FRANCOIS (FR)) 29 décembre 1999 (1999-12-29) * page 32 - page 33; tableau 1 * | 1-5 | |
| A | WO 03/035110 A (CEDARS SINAI MEDICAL CENTER) 1 mai 2003 (2003-05-01) * page 8, ligne 1 - ligne 4 * | 1-5 | DOMAINES TECHNIQUES RECHERCHES (IPC)<br>C07K |
| A | BLOND J-L ET AL: "MOLECULAR CHARACTERIZATION AND PLACENTAL EXPRESSION OF HERV-W, A NEW HUMAN ENDOGENOUS RETROVIRUS FAMILY", JOURNAL OF GENERAL VIROLOGY, SOCIETY FOR GENERAL MICROBIOLOGY, READING, GB, vol. 73, no. 2, février 1999 (1999-02), pages 1175-1185, XP000828006, ISSN: 0022-1317 * abrégé * | 1-5 | |

-/--

Le présent rapport a été établi pour toutes les revendications

| Lieu de la recherche | Date d'achèvement de la recherche | Examinateur |
|---|---|---|
| Munich | 12 janvier 2017 | Wagner, René |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**Europäisches Patentamt**
**European Patent Office**
**Office européen des brevets**

# RAPPORT DE RECHERCHE EUROPEENNE

Numéro de la demande

EP 16 18 4011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | JIANG Q ET AL: "Cutting edge: Lipopolysaccharide induces physical proximity between CD14 and Toll-like receptor 4 (TLR4) prior to nuclear translocation of NF-[kappa]B", JOURNAL OF IMMUNOLOGY 01 OCT 2000 UNITED STATES, vol. 165, no. 7, 1 octobre 2000 (2000-10-01), pages 3541-3544, XP002296558, ISSN: 0022-1767 * abrégé * | 1-5 | |
| A | LEHNARDT SEIJA ET AL: "The toll-like receptor TLR4 is necessary for lipopolysaccharide-induced oligodendrocyte injury in the CNS", JOURNAL OF NEUROSCIENCE, vol. 22, no. 7, 1 avril 2002 (2002-04-01), pages 2478-2486, XP002296559, ISSN: 0270-6474 * le document en entier * | 1-5 | |
| A | PERRON H ET AL: "Multiple sclerosis retrovirus particles and recombinant envelope trigger an abnormal immune response in vitro, by inducing polyclonal Vbeta16 T-lymphocyte activation", VIROLOGY, vol. 287, no. 2, 1 septembre 2001 (2001-09-01), pages 321-332, XP002296560, ISSN: 0042-6822 * le document en entier * | 1-5 | DOMAINES TECHNIQUES RECHERCHES (IPC) |

-/--

Le présent rapport a été établi pour toutes les revendications

| Lieu de la recherche | Date d'achèvement de la recherche | Examinateur |
|---|---|---|
| Munich | 12 janvier 2017 | Wagner, René |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches
Patentamt

European
Patent Office

Office européen
des brevets

**RAPPORT DE RECHERCHE EUROPEENNE**

Numéro de la demande

EP 16 18 4011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 95/21256 A (BIO MERIEUX ; BEDIN FREDERIC (FR); BESEME FREDERIC (FR); MALLET FRANCO) 10 août 1995 (1995-08-10) * le document en entier * | 1-5 | |
| A | WO 01/16171 A (INST NAT SANTE RECH MED ; BIO MERIEUX (FR); BLOND JEAN LUC (FR); BOUTO) 8 mars 2001 (2001-03-08) * le document en entier * | 1-5 | |
| A | PERRON H ET AL: "Molecular identification of a novel retrovirus repeatedly isolated from patients with multiple sclerosis", PROCEEDINGS OF THE NATIONAL ACADEMY OF SCIENCES OF USA, NATIONAL ACADEMY OF SCIENCE. WASHINGTON, US, vol. 14, no. 94, 8 juillet 1997 (1997-07-08), pages 7538-7588, XP002059256, ISSN: 0027-8424 * le document en entier * | 1-5 | |
| A | SERRA CATERINA ET AL: "In vitro modulation of the multiple sclerosis (MS)-associated retrovirus by cytokines: Implications for MS pathogenesis.", JOURNAL OF NEUROVIROLOGY, vol. 9, no. 6, décembre 2003 (2003-12), pages 637-643, XP009048660, ISSN: 1355-0284 * le document en entier * | 1-5 | DOMAINES TECHNIQUES RECHERCHES (IPC) |

-/--

Le présent rapport a été établi pour toutes les revendications

| Lieu de la recherche | Date d'achèvement de la recherche | Examinateur |
|---|---|---|
| Munich | 12 janvier 2017 | Wagner, René |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................
& : membre de la même famille, document correspondant

EPO FORM 1503 03.82 (P04C02)

Europäisches
Patentamt

European
Patent Office

Office européen
des brevets

# RAPPORT DE RECHERCHE EUROPEENNE

Numéro de la demande

EP 16 18 4011

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | FIROUZI R ET AL: "Multiple sclerosis-associated retrovirus particles cause T lymphocyte-dependent death with brain hemorrhage in humanized SCID mice model.", JOURNAL OF NEUROVIROLOGY, vol. 9, no. 1, février 2003 (2003-02), pages 79-93, XP009048638, ISSN: 1355-0284 * le document en entier * ----- | 1-5 | |
| A,D | PERRON HERVE ET AL: "Particle-associated retroviral RNA and tandem RGH/HERV-W copies on human chromosome 7q: Possible components of a 'chain-reaction' triggered by infectious agents in multiple sclerosis?", JOURNAL OF NEUROVIROLOGY, vol. 6, no. Supplement 2, mai 2000 (2000-05), pages S67-S75, XP009048659, ISSN: 1355-0284 * le document en entier * ----- | 1-5 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| Munich | 12 janvier 2017 | Wagner, René |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................
& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 16 18 4011

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-01-2017

| Document brevet cité au rapport de recherche | | Date de publication | Membre(s) de la famille de brevet(s) | | Date de publication |
|---|---|---|---|---|---|
| WO 0131021 | A | 03-05-2001 | EP | 1224291 A1 | 24-07-2002 |
| | | | JP | 2003512844 A | 08-04-2003 |
| | | | US | 2004054133 A1 | 18-03-2004 |
| | | | WO | 0131021 A1 | 03-05-2001 |
| WO 9967395 | A | 29-12-1999 | AU | 4375199 A | 10-01-2000 |
| | | | CA | 2331923 A1 | 29-12-1999 |
| | | | EP | 1090122 A1 | 11-04-2001 |
| | | | EP | 1997895 A1 | 03-12-2008 |
| | | | FR | 2780069 A1 | 24-12-1999 |
| | | | JP | 2002518051 A | 25-06-2002 |
| | | | US | 6919438 B1 | 19-07-2005 |
| | | | US | 2005118573 A1 | 02-06-2005 |
| | | | US | 2009263783 A1 | 22-10-2009 |
| | | | WO | 9967395 A1 | 29-12-1999 |
| WO 03035110 | A | 01-05-2003 | EP | 1438070 A1 | 21-07-2004 |
| | | | JP | 2005507920 A | 24-03-2005 |
| | | | US | 2003077279 A1 | 24-04-2003 |
| | | | WO | 03035110 A1 | 01-05-2003 |
| WO 9521256 | A | 10-08-1995 | AT | 285475 T | 15-01-2005 |
| | | | AU | 704440 B2 | 22-04-1999 |
| | | | CA | 2141907 A1 | 05-08-1995 |
| | | | DE | 674004 T1 | 19-09-1996 |
| | | | DE | 69533863 D1 | 27-01-2005 |
| | | | DE | 69533863 T2 | 16-02-2006 |
| | | | EP | 0674004 A1 | 27-09-1995 |
| | | | ES | 2233936 T3 | 16-06-2005 |
| | | | FI | 954699 A | 03-10-1995 |
| | | | JP | 3611856 B2 | 19-01-2005 |
| | | | JP | H08511170 A | 26-11-1996 |
| | | | NO | 953925 A | 04-12-1995 |
| | | | NZ | 279855 A | 27-05-1998 |
| | | | US | 5800980 A | 01-09-1998 |
| | | | US | 5871745 A | 16-02-1999 |
| | | | US | 5871996 A | 16-02-1999 |
| | | | US | 5962217 A | 05-10-1999 |
| | | | US | 6184025 B1 | 06-02-2001 |
| | | | US | 6342383 B1 | 29-01-2002 |
| | | | WO | 9521256 A1 | 10-08-1995 |
| WO 0116171 | A | 08-03-2001 | AT | 531726 T | 15-11-2011 |
| | | | AU | 7297100 A | 26-03-2001 |
| | | | CA | 2383877 A1 | 08-03-2001 |
| | | | EP | 1212359 A1 | 12-06-2002 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE
RELATIF A LA DEMANDE DE BREVET EUROPEEN NO.**

EP 16 18 4011

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-01-2017

| Document brevet cité au rapport de recherche | Date de publication | Membre(s) de la famille de brevet(s) | Date de publication |
|---|---|---|---|
| | | EP 2385058 A1 | 09-11-2011 |
| | | ES 2376726 T3 | 16-03-2012 |
| | | ES 2444649 T3 | 26-02-2014 |
| | | FR 2797889 A1 | 02-03-2001 |
| | | JP 4283475 B2 | 24-06-2009 |
| | | JP 4824731 B2 | 30-11-2011 |
| | | JP 2003510032 A | 18-03-2003 |
| | | JP 2009072194 A | 09-04-2009 |
| | | US 7442550 B1 | 28-10-2008 |
| | | WO 0116171 A1 | 08-03-2001 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **BLAZAR et al.** *Journal of Immunology,* 1997, vol. 159, 5821-5833
- **BIRD et al.** *Science,* 1988, vol. 242, 423-426
- **ARAKAWA et al.** *J. Biochem,* 1996, vol. 120, 657-662
- **CHAUDRAY et al.** *Nature,* 1989, vol. 339, 394-397
- **KÖHLER G. ; MILSTEIN C.** Continuous culture of fused cells secreting antibody of predefined specificity. *Nature,* 1975, vol. 256, 495-497
- **GALFRE G. et al.** *Nature,* 1977, vol. 266, 522-550
- **FIROUZI et al.** *Journal of Neurovirology,* 2003
- **CONRAD, B. et al.** A human endogenous retroviral superantigen as candidate autoimmune gene in type I diabetes. *Cell,* 1997, vol. 90 (2), 303-13
- **PERRON, H. et al.** Molecular identification of a novel retrovirus repeatedly isolated from patients with multiple sclerosis. The Collaborative Research Group on Multiple Sclerosis. *Proc Natl Acad Sci USA,* 1997, vol. 94 (14), 7583-8
- **DEB-RINKER, P. et al.** Molecular characterization of a MSRV-like sequence identified by RDA from monozygotic twin pairs discordant for schizophrenia. *Genomics,* 1999, vol. 61 (2), 133-44
- **PERRON, H. et al.** Isolation of retrovirus from patients with multiple sclerosis [letter]. *Lancet,* 1991, vol. 337 (8745), 862-3
- **BLOND, J.L. et al.** Molecular characterization and placental expression of HERV-W, a new human endogenous retrovirus family. *J Virol,* 1999, vol. 73 (2), 1175-85
- **PERRON, H. et al.** Particle-associated retroviral RNA and tandem RGH/HERV-W copies on human chromosome 7q: possible components of a 'chain-reaction' triggered by infectious agents in multiple sclerosis?. *J Neurovirol,* 2000, vol. 6 (2), 67-75
- **DOLEI, A. et al.** Multiple sclerosis-associated retrovirus (MSRV) in Sardinian MS patients. *Neurology,* 2002, vol. 58 (3), 471-3
- **GARSON, J.A. et al.** Detection of virion-associated MSRV-RNA in serum of patients with multiple sclerosis [letter] [see comments]. *Lancet,* 1998, vol. 351 (9095), 33
- **OLSSON, P. et al.** Retroviral RNA related to ERV9/MSRV in a human serum: a new sequence variant. *AIDS Res Hum Retroviruses,* 1999, vol. 15 (6), 591-3

- **SOTGIU, S. et al.** Multiple sclerosis-associated retrovirus and MS prognosis: an observational study. *Neurology,* 2002, vol. 59 (7), 1071-3
- **PERRON, H. et al.** Multiple sclerosis retrovirus particles and recombinant envelope trigger an abnormal immune response in vitro, by inducing polyclonal Vbeta16 T-lymphocyte activation. *Virology,* 2001, vol. 287 (2), 321-32
- **FIROUZI, R. et al.** Multiple Sclerosis Associated Retrovirus Particles Cause T-Lymphocyte Dependent Death with Brain Hemorrhage, in Humanized SCID Mice Model. *Journal of Neurovirology,* 2003, vol. 9, 79-93
- **LIN, A. et al.** The inflammatory response system in treatment-resistant schizophrenia: increased serum interleukin-6. *Schizophr Res,* 1998, vol. 32 (1), 9-15
- **STEVENS JR.** Neuropathology of schizophren...[PMID: 7125843]Related Articles. *Links*
- **KARLSSON, H. et al.** Retroviral RNA identified in the cerebrospinal fluids and brains of individuals with schizophrenia. *Proc Natl Acad Sci U S A,* 2001, vol. 98 (8), 4634-9
- **PERRON, H.** Microbial Agents triggering Endogenous Retroviruses within Genetic Susceptibility Loci, Resulting in Expression of Superantigen and Gliotoxic Molecules: a Plausible '' Immunovirogenetic '' Cascade causing Multiple Sclerosis ?. *Modern Aspects of Immunobiology,* 2001, vol. 1 (5), 198-203
- **LIU, Y. et al.** Dextromethorphan protects dopaminergic neurons against inflammation-mediated degeneration through inhibition of microglial activation. *J Pharmacol Exp Ther,* 2003, vol. 305 (1), 212-8
- **GAO HM et al.** Synergistic dopaminergic neur...[PMID: 12598611]Related Articles. *Links*
- **MORIMOTO, K. ; T. MURASUGI ; T. ODA.** Acute neuroinflammation exacerbates excitotoxicity in rat hippocampus in vivo. *Exp Neurol,* 2002, vol. 177 (1), 95-104
- **STOLL G.** Inflammatory cytokines in the...[PMID: 12407295]Related Articles. *Links*
- **GUILLEMIN, G.J. ; B.J. BREW.** Implications of the kynurenine pathway and quinolinic acid in Alzheimer's disease. *Redox Rep,* 2002, vol. 7 (4), 199-206

- **KIM EJ et al.** Neuroprotective effects of pr...[PMID: 12237868]Related Articles. *Links*
- **KIM, W.G. et al.** Regional difference in susceptibility to lipopolysaccharide-induced neurotoxicity in the rat brain: role of microglia. *J Neurosci,* 2000, vol. 20 (16), 6309-16
- **CZLONKOWSKA A et al.** Inflammatory changes in the s...[PMID: 10894230]Related Articles. *Links*
- **LICINIO, J. ; M.L. WONG.** The role of inflammatory mediators in the biology of major depression: central nervous system cytokines modulate the biological substrate of depressive symptoms, regulate stress-responsive systems, and contribute to neurotoxicity and neuroprotection. *Mol Psychiatry,* 1999, vol. 4 (4), 317-27
- **COTTER RL et al.** Insights into the neurodegene...[PMID: 10204569]Related Articles. *Links*
- **COTTER, R.L. et al.** Insights into the neurodegenerative process of Alzheimer's disease: a role for mononuclear phagocyte-associated inflammation and neurotoxicity. *J Leukoc Biol,* 1999, vol. 65 (4), 416-27
- **HEESE K et al.** Inflammatory signals induce n...[PMID: 9453564]Related Articles. *Links*
- **HEESE, K. ; C. HOCK ; U. OTTEN.** Inflammatory sig-nals induce neurotrophin expression in human microglial cells. *J Neurochem,* 1998, vol. 70 (2), 699-707
- **SASSER LB et al.** Subchronic toxicity evaluatio...[PMID: 8600286]Related Articles. *Links*
- **CHAO, C.C. et al.** Interleukin-1 and tumor necrosis factor-alpha synergistically mediate neurotoxicity: involvement of nitric oxide and of N-methyl-D-aspartate receptors. *Brain Behav Immun,* 1995, vol. 9 (4), 355-65
- **CHAO CC et al.** Tumor necrosis factor-alpha p...[PMID: 7705222]Related Articles. *Links*
- **CHAO, C.C. ; S. HU.** Tumor necrosis factor-alpha potentiates glutamate neurotoxicity in human fetal brain cell cultures. *Dev Neurosci,* 1994, vol. 16 (3-4), 172-9
- **BAL-PRICE, A. ; G.C. BROWN.** Inflammatory neu-rodegeneration mediated by nitric oxide from activated glia-inhibiting neuronal respiration, causing glutamate release and excitotoxicity. *J Neurosci,* 2001, vol. 21 (17), 6480-91
- **OBRENOVITCH TP.** Quinolinic acid accumulation ...[PMID: 11462760]Related Articles. *Links*
- **OBRENOVITCH, T.P.** Quinolinic acid accumulation during neuroinflammation. Does it imply excitotoxicity?. *Ann N Y Acad Sci,* 2001, vol. 939 (1-10
- **LAW A et al.** Say NO to Alzheimer's disease...[PMID: 11245887]Related Articles. *Links*
- **WERNER, P. ; D. PITT ; C.S. RAINE.** Glutamate ex-citotoxicity--a mechanism for axonal damage and oligodendrocyte death in Multiple Sclerosis?. *J Neural Transm Suppl,* 2000, vol. 60, 375-85
- **PITT D et al.** Glutamate excitotoxicity in a...[PMID: 10613826]Related Articles. *Links*
- **PITT, D. ; P. WERNER ; C.S. RAINE.** Glutamate ex-citotoxicity in a model of multiple sclerosis. *Nat Med,* 2000, vol. 6 (1), 67-70
- **CARLSON NG et al.** Inflammatory cytokines IL-1 a...[PMID: 10490998]Related Articles. *Links*
- **CARLSON, N.G. et al.** Inflammatory cytokines IL-1 alpha, IL-1 beta, IL-6, and TNF-alpha impart neuroprotection to an excitotoxin through distinct path-ways. *J Immunol,* 1999, vol. 163 (7), 3963-8
- **WANG YS et al.** The bacterial endotoxin lipop...[PMID: 9930737]Related Articles. *Links*
- **WANG, Y.S. ; T.D. WHITE.** The bacterial endotoxin lipopolysaccharide causes rapid inappropriate excitation in rat cortex. *J Neurochem,* 1999, vol. 72 (2), 652-60
- **YOLKEN, R.H. et al.** Endogenous retroviruses and schizophrenia. *Brain Res Brain Res Rev,* 2000, vol. 31 (2-3), 193-9
- **KLEINE, T.O. et al.** Approach to discriminate subgroups in multiple sclerosis with cerebrospinal fluid (CSF) basic inflammation indices and TNF-alpha, IL-Ibeta, IL-6, IL-8. *Brain Res Bull,* 2003, vol. 61 (3), 327-46
- **AARLI JA.** Role of cytokines in neurolog...[PMID: 12871095]Related Articles. *Links*
- **AARLI, J.A.** Role of cytokines in neurological disorders. *Curr Med Chem,* 2003, vol. 10 (19), 1931-7
- **VLADIC A et al.** Cerebrospinal fluid and serum...[PMID: 12445803]Related Articles. *Links*
- **MILJKOVIC, D. et al.** Nitric oxide metabolites and interleukin-6 in cerebrospinal fluid from multiple sclerosis patients. *Eur J Neurol,* 2002, vol. 9 (4), 413-8
- **CLERICI M et al.** Single-cell analysis of cytok...[PMID: 11730945]Related Articles. *Links*
- **FEDETZ, M. et al.** The -174/-597 promoter polymorphisms in the interleukin-6 gene are not associated with susceptibility to multiple sclerosis. *J Neurol Sci,* 2001, vol. 190 (1-2), 69-72
- **STELMASIAK Z et al.** IL-6 and sIL-6R concentration...[PMID: 11535934]Related Articles. *Links*
- **VANDENBROECK, K. et al.** High-resolution analysis of IL-6 minisatellite polymorphism in Sardinian multiple sclerosis: effect on course and onset of disease. *Genes Immun,* 2000, vol. 1 (7), 460-3
- **STELMASIAK Z et al.** Interleukin-6 concentration i...[PMID: 11208463]Related Articles. *Links*

- **STELMASIAK, Z. et al.** Interleukin-6 concentration in serum and cerebrospinal fluid in multiple sclerosis patients. *Med Sci Monit,* 2000, vol. 6 (6), 1104-8
- **SCHONROCK LM et al.** Interleukin-6 expression in h...[PMID: 11044583]Related Articles. *Links*
- **CORNFORD, E.M. ; M.E. CORNFORD.** New systems for delivery of drugs to the brain in neurological disease. *Lancet Neurol,* 2002, vol. 1 (5), 306-15
- **SCHMIDT J et al.** Drug targeting by long-circul...[PMID: 12805101]Related Articles. *Links*
- **SCHMIDT, J. et al.** Drug targeting by long-circulating liposomal glucocorticosteroids increases therapeutic efficacy in a model of multiple sclerosis. *Brain,* 2003, vol. 126, 1895-904
- **FOURNIER E et al.** Therapeutic effectiveness of ...[PMID: 12767096]Related Articles. *Links*
- **PARDRIDGE, W.M.** Blood-brain barrier drug targeting enables neuroprotection in brain ischemia following delayed intravenous administration of neurotrophins. *Adv Exp Med Biol,* 2002, vol. 513, 397-430
- **WATANABE S et al.** Chemotherapeutic targeting of...[PMID: 12575735]Related Articles. *Links*
- **WATANABE, S. et al.** Chemotherapeutic targeting of etoposide to regions of the brain on the basis of polyamine level. *J Drug Target,* 2002, vol. 10 (6), 457-61
- **LAHIRI DK et al.** A critical analysis of new mo...[PMID: 12558063]Related Articles. *Links*
- **SCHERRMANN, J.M.** Drug delivery to brain via the blood-brain barrier. *Vascul Pharmacol,* 2002, vol. 38 (6), 349-54
- **WANG JX et al.** Enhanced brain targeting by s...[PMID: 12445558]Related Articles. *Links*
- **WANG, J.X. ; X. SUN ; Z.R. ZHANG.** Enhanced brain targeting by synthesis of 3',5'-dioctanoyl-5-fluoro-2'-deoxyuridine and incorporation into solid lipid nanoparticles. *Eur J Pharm Biopharm,* 2002, vol. 54 (3), 285-90
- **MAHAR DOAN KM et al.** Passive permeability and P-gl...[PMID: 12438524]Related Articles. *Links*
- **HOSOYA, K. ; S. OHTSUKI ; T. TERASAKI.** Recent advances in the brain-to-blood efflux transport across the blood-brain barrier. *Int J Pharm,* 2002, vol. 248 (1-2), 15-29
- **MORA M et al.** Design and characterization o...[PMID: 12425459]Related Articles. *Links*
- **MORA, M. et al.** Design and characterization of liposomes containing long-chain N-acylPEs for brain delivery: penetration of liposomes incorporating GM1 into the rat brain. *Pharm Res,* 2002, vol. 19 (10), 1430-8
- **PERRON, H. et al.** Herpes simplex virus ICP0 and ICP4 immediate early proteins strongly enhance expression of a retrovirus harboured by a leptomeningeal cell line from a patient with multiple sclerosis. *J Gen Virol,* 1993, vol. 74, 65-72
- **SOLDAN, S. et al.** Association of human herpes virus 6 (HHV-6) with multiple sclerosis: increased IgM response to HHV-6 early antigen and detection of serum HHV-6 DNA. *Nat Med,* 1997, vol. 3, 1394-1397
- **HAAHR, S. et al.** Is multiple sclerosis caused by a dual infection with retrovirus and Epstein-Barr virus?. *Neuroepidemiology,* 1992, vol. 11 (4-6), 299-303
- **BERGSTRÖM, T. ; O. ANDERSEN ; A. VAHLNE.** Isolation of herpes simplex virus type 1 during first attack of multiple sclerosis. *Ann Neurol,* 1989, vol. 26, 283-285
- **MARX, C.E. et al.** Cytokine effects on cortical neuron MAP-2 immunoreactivity: implications for schizophrenia. *Biol Psychiatry,* 2001, vol. 50 (10), 743-9
- **MAES M et al.** Effects of atypical antipsych...[PMID: 10706993]Related Articles. *Links*
- **MINAGAR, A. et al.** The role of macrophage/microglia and astrocytes in the pathogenesis of three neurologic disorders: HIV-associated dementia, Alzheimer disease, and multiple sclerosis. *J Neurol Sci,* 2002, vol. 202 (1-2), 13-23
- **JEOHN GH et al.** Go6976 protects mesencephalic...[PMID: 12076986]Related Articles. *Links*
- **GASER, C. et al.** Ventricular enlargement in schizophrenia related to volume reduction of the thalamus, striatum, and superior temporal cortex. *Am J Psychiatry.,* 2004, vol. 161, 154-156
- **KURTZKE, J.** Disability rating scales in multiple sclerosis. *Ann N Y Acad Sci.,* 1984, vol. 436, 347-60
- **KARLSSON, H. et al.** HERV-W-related RNA detected in plasma from individuals with recent-onset schizophrenia or schizoaffective disorder. *Mol Psychiatry,* 2004, vol. 9, 12-13
- **QIU, Z. ; D.L. GRUOL.** Interleukin-6, beta-amyloid peptide and NMDA interactions in rat cortical neurons. *J Neuroimmunol,* 2003, vol. 139 (1-2), 51-7
- **JENNER P.** Oxidative stress in Parkinson...[PMID: 12666096]Related Articles. *Links*
- **LAFON, M. et al.** Human Viral superantigens: to be or not to be transactivated ?. *Trends in Immunology,* 2002, vol. 23 (5), 238-239
- **PRANZATELLI, M.** Innovations in drug delivery to the central nervous system. *Drugs Today (Barc),* 1999, vol. 35, 435-448
- **MERLO, A. ; J. MUELLER-BRAND ; H. MAECKE.** Comparing monoclonal antibodies and small peptidic hormones for local targeting of malignant gliomas. *Acta Neurochir.,* 2003, vol. 88, 83-91

- **ANTONY, J. et al.** Human endogenous retrovirus glycoprotein-mediated induction of redox reactants causes oligodendrocyte death and demyelination. *Nat Neurosci.,* 2004, vol. 7 (10), 1088-95
- **NG, P. ; Y. OSAWA.** Preparation and characterization of the F (ab)2 fragments of an aromatase activity-suppressing monoclonal antibody. *Steroids.,* 1997, vol. 62, 776-81
- **PERRON, H. et al.** In vitro transmission and antigenicity of a retrovirus isolated from a multiple sclerosis patient. *Res Virol,* 1992, vol. 143 (5), 337-50
- **SERRA, C. et al.** Multiple sclerosis and multiple sclerosis-associated retrovirus in Sardinia. *Neurol Sci,* 2001, vol. 22 (2), 171-3
- **ZAWADA M FAU - LIWIEN, I. et al.** MSRV pol sequence copy number as a potential marker of multiple sclerosis. *Pol J Pharmacol,* 2003, vol. 55 (5), 869-75
- **PERRON H FAU - GARSON, J.A. et al.** Molecular identification of a novel retrovirus repeatedly isolated from. *Proc Natl Acad Sci U S A,* 1997, vol. 94 (14), 7583-8
- **ROLLAND, A. et al.** Correlation between disease severity and in vitro cytokine production mediated by MSRV (Multiple Sclerosis associated RetroViral element) envelope protein in patients with multiple sclerosis. *J Neuroimmunol,* 2004
- **KOMURIAN-PRADEL, F. et al.** Molecular cloning and characterization of MSRV-related sequences associated with retrovirus-like particles. *Virology,* 1999, vol. 260 (1), 1-9
- **PERRON, H. et al.** Human Endogenous Retrovirus (HERV-W Env And Gag Proteins: Physiological Expression In Human Brain And Pathophysiological Modulation In Multiple Sclerosis Lesions. *J. Neurovirology,* 2005